# EUROPEAN PATENT SPECIFICATION

(11) **EP 3 768 143 B1**
(45) Date of publication and mention of the grant of the patent: **26.02.2025**
(21) Application number: 19701911.0
(22) Date of filing: 11.01.2019
(51) Int. Cl.: A61B 1/05, A61B 1/01, A61B 1/00, A61B 90/50, A61B 90/35, A61B 90/25, A61B 90/00, G02B 23/24, A61B 34/30, H04N 23/50

(54) **MEDICAL IMAGING APPARATUS AND MEDICAL OBSERVATION SYSTEM**
VORRICHTUNG ZUR MEDIZINISCHEN BILDGEBUNG UND SYSTEM ZUR MEDIZINISCHEN BEOBACHTUNG
APPAREIL D'IMAGERIE MÉDICALE ET SYSTÈME D'OBSERVATION MÉDICALE

(30) Priority: 19.03.2018 JP 2018051127
(43) Date of publication of application: 27.01.2021
(73) Proprietor: Sony Group Corporation, Tokyo 108-0075 (JP)
(72) Inventor: KURODA, Yohei, Tokyo 108-0075 (JP); TOMATSU, Kei, Tokyo 108-0075 (JP); FUKUSHIMA, Tetsuharu, Tokyo 108-0075 (JP); ARAI, Jun, Tokyo 108-0075 (JP)
(74) Representative: D Young & Co LLP
(86) International application number: PCT/JP2019/000662
(87) International publication number: WO 2019/181149

(56) References cited:
- WO-A1-2017/169118
- JP-A- H06 237 881
- US-A1- 2004 147 809
- US-A1- 2007 213 586
- US-A1- 2017 135 563

## Description

### [Technical Field]

The present disclosure relates to a medical imaging apparatus and a medical observation system.

### [Background Art]

As surgical techniques and surgical instruments have developed in recent years, surgery in which various treatments are performed while an affected area is observed using a medical observation apparatus such as a surgical microscope or endoscope (so-called microsurgery) has been frequently performed. In addition, among such medical observation apparatuses, an apparatus that causes a display apparatus such as a monitor to display an image of an affected area captured by an imaging apparatus (a camera) or the like as an electronic image (i.e., a medical imaging apparatus) has been proposed, without being limited to an apparatus that allows an affected area to be optically observed. PTL 1, for example, discloses an example of a diagnostic observation system including a medical imaging apparatus such as a surgical microscope or endoscope.

### [Citation List]

### [Patent Literature]

JP 2000-325361A
JPH06237881 considers a video display device of an endoscope.
US 2007/0213586 relates to an endoscope system and adapter applied to this endoscope system.
US 2004/0147809 describes an endoscopic system with a solid-state light source.
WO2017/169118 relates to a jig-holding device and medical observation device.

### [Summary]

The present invention is defined by the appended claims.

### [Technical Problem]

There are many cases in which the above-described medical imaging apparatus requires complex operations to set a target affected area to be observable in a desired direction. In a case of an endoscope, for example, a situation requiring an operation of rotating a lens barrel in a twisting direction using the extension direction of the lens barrel as an axis is conceivable. In addition, with respect to the above-described medical imaging apparatus, a situation in which an operation for maintaining hand-eye coordination (cooperation between the sense of a hand and the sense of the eyes (vision)) of an operator is complicated can also be conceivable. Specifically, it is desirable to provide a single support device that may be attached to either a surgical microscope or an endoscope and may control the microscope or endoscope by rotation. In particular, it is desirable to provide a single support device that can be used with a surgical microscope and an endoscope (such as an oblique-viewing endoscope where the viewing direction is changed by rotating the endoscope). This allows a surgeon to become familiar with a single support device. However, in this case, light is required by both the surgical microscope and the endoscope. This is problematic as the surgical device is controlled by rotation of the support device which tangles and otherwise makes operating such a device complicated. For example, the light guide cable would get tangled when the oblique-viewing endoscope is rotated. It is an aim of the present disclosure to address this issue.

The present disclosure proposes a technology for further improving operability in observation of an affected area.

### [Solution to Problem]

According to the present invention, a support unit as defined in claim 1 is provided. Advantageous features are defined by the dependent claims.

### [Advantageous Effects of Invention]

According to an embodiment of the present disclosure described above, a technology that can further improve operability in observation of an affected area is provided.

Note that the effects described above are not necessarily limitative. With or in the place of the above effects, there may be achieved any one of the effects described in this specification or other effects that may be grasped from this specification.

### [Brief Description of Drawings]

FIG. 1 is a diagram depicting an example of a schematic configuration of an endoscopic surgery system to which the technology according to the present disclosure can be applied.
FIG. 2 is a block diagram depicting an example of a functional configuration of a camera head and a camera control unit (CCU) depicted in FIG. 1.
FIG. 3 is a perspective diagram illustrating a configuration example of a medical supporting arm apparatus of the present disclosure.
FIG. 4 is a schematic diagram illustrating a configuration of an oblique-viewing endoscope of the present disclosure.
FIG. 5 is a schematic diagram illustrating a comparison of the oblique-viewing endoscope and a straight-viewing endoscope.
FIG. 6 is an explanatory diagram for describing an example of a configuration of a medical imaging apparatus according to Comparative Example 1.
FIG. 7 is an explanatory diagram for describing an example of a configuration of a medical imaging apparatus according to Comparative Example 2.
FIG. 8 is an explanatory diagram for describing another aspect of the medical imaging apparatus according to Comparative Example 2.
FIG. 9 is an explanatory diagram for describing an example of a schematic configuration of a medical imaging apparatus of the present disclosure.
FIG. 10 is an explanatory diagram for describing an example of a schematic configuration of the medical imaging apparatus according to the present disclosure.
FIG. 11 is an explanatory diagram for describing an example of a schematic configuration of the medical imaging apparatus according to the present disclosure.
FIG. 12 is an explanatory diagram for describing an example of a structure of a medical imaging apparatus according to a first practical example.
FIG. 13 is an explanatory diagram for describing an example of a structure of a medical imaging apparatus according to a first practical example.
FIG. 14 is an explanatory diagram for describing an example of an internal structure of the medical imaging apparatus according to the first practical example.
[fig.15] FIG. 15 is an explanatory diagram for describing an example of an internal structure of the medical imaging apparatus according to the first practical example.
[fig.16] FIG. 16 is an explanatory diagram for describing an example of a structure of a camera head of the medical imaging apparatus according to the first practical example.
[fig.17] FIG. 17 is an explanatory diagram for describing an example of a structure of a camera head of the medical imaging apparatus according to the first practical example.
[fig.18] FIG. 18 is an explanatory diagram for describing an example of a structure of a camera head of the medical imaging apparatus according to the first practical example.
[fig.19]FIG. 19 is an explanatory diagram for describing an example of an internal structure of the medical imaging apparatus according to the first practical example.
[fig.20]FIG. 20 is an explanatory diagram for describing an example of an internal structure of the medical imaging apparatus according to the first practical example.
[fig.21]FIG. 21 is an explanatory diagram for describing an example of a structure of a rigid endoscope mounted in the camera head according to the present disclosure.
[fig.22]FIG. 22 is an explanatory diagram for describing an example of a structure of a rigid endoscope mounted in the camera head according to the present disclosure.
[fig.23]FIG. 23 is an explanatory diagram for describing an example of a structure of a rigid endoscope mounted in the camera head according to the present disclosure.
[fig.24]FIG. 24 is an explanatory diagram for describing an example of a structure of a rigid endoscope mounted in the camera head according to the present disclosure.
[fig.25]FIG. 25 is an explanatory diagram for describing an example of an internal structure of the rigid endoscope mounted in the camera head according to the present disclosure.
[fig.26]FIG. 26 is an explanatory diagram for describing an example of an internal structure of the rigid endoscope mounted in the camera head according to the present disclosure.
[fig.27]FIG. 27 is an explanatory diagram for describing an example of a schematic configuration of a medical imaging apparatus according to a second practical example.
[fig.28]FIG. 28 is an explanatory diagram for describing an example of a structure of the medical imaging apparatus according to the second practical example.
[fig.29]FIG. 29 is an explanatory diagram for describing an example of a structure of the medical imaging apparatus according to the second practical example.
[fig.30]FIG. 30 is an explanatory diagram for describing an example of an internal structure of the medical imaging apparatus according to the second practical example.
[fig.31]FIG. 31 is an explanatory diagram for describing an example of an internal structure of the medical imaging apparatus according to the second practical example.
[fig.32]FIG. 32 is an explanatory diagram for describing an example of a structure of an endoscope mounted in a camera head according to the present disclosure.
[fig.33]FIG. 33 is an explanatory diagram for describing an example of a structure of an endoscope mounted in a camera head according to the present disclosure.
[fig.34]FIG. 34 is an explanatory diagram for describing an example of a structure of an endoscope mounted in a camera head according to the present disclosure.
[fig.35]FIG. 35 is an explanatory diagram for describing an example of an internal structure of the endoscope mounted in the camera head according to the present disclosure.
[fig.36]FIG. 36 is an explanatory diagram for describing an example of an internal structure of the endoscope mounted in the camera head according to the present disclosure.
[fig.37]FIG. 37 is an explanatory diagram for describing an overview of an example of a configuration of a camera head of a medical imaging apparatus according to a modified example.
[fig.38]FIG. 38 is an explanatory diagram for describing an overview of an example of an internal configuration of the camera head of the medical imaging apparatus according to the modified example.
[fig.39]FIG. 39 is an explanatory diagram for describing an example of a structure of a support unit of the camera head according to the modified example.
[fig.40]FIG. 40 is an explanatory diagram for describing an example of a structure of a support unit of the camera head according to the modified example.
[fig.41]FIG. 41 is an explanatory diagram for describing an example of an internal structure of the support unit of the camera head according to the modified example.
[fig.42]FIG. 42 is an explanatory diagram for describing an example of an internal structure of the support unit of the camera head according to the modified example.
[fig.43]FIG. 43 is an explanatory diagram for describing an example of a structure of a base unit of the camera head according to the modified example.
[fig.44]FIG. 44 is an explanatory diagram for describing an example of a structure of a base unit of the camera head according to the modified example.
[fig.45]FIG. 45 is an explanatory diagram for describing an example of an internal structure of the support unit of the camera head according to the modified example.
[fig.46]FIG. 46 is an explanatory diagram for describing an example of an internal structure of the support unit of the camera head according to the modified example.
[fig.47]FIG. 47 is an explanatory diagram for describing an aspect of a first application example of the medical imaging apparatus according to the present disclosure.
[fig.48]FIG. 48 is an explanatory diagram for describing an aspect of the first application example of the medical imaging apparatus according to the present disclosure.
[fig.49]FIG. 49 is an explanatory diagram for describing an aspect of the first application example of the medical imaging apparatus according to the present disclosure.
[fig.50]FIG. 50 is an explanatory diagram for describing an aspect of a second application example of the medical imaging apparatus according to the present disclosure.
[fig.51]FIG. 51 is an explanatory diagram for describing an aspect of a second application example of the medical imaging apparatus according to the present disclosure.
[fig.52]FIG. 52 is an explanatory diagram for describing an aspect of a second application example of the medical imaging apparatus according to the present disclosure.
[fig.53]FIG. 53 is an explanatory diagram for describing an aspect of a second application example of the medical imaging apparatus according to the present disclosure.
[fig.54]FIG. 54 is an explanatory diagram for describing an aspect of a second application example of the medical imaging apparatus according to the present disclosure.
[fig.55]FIG. 55 is an explanatory diagram for describing an aspect of a second application example of the medical imaging apparatus according to the present disclosure.

### Description of Embodiments

Hereinafter, (a) examples of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that description will be provided in the following order.
1. Configuration example of endoscope system
2. Configuration example of supporting arm apparatus
3. Basic configuration of oblique-viewing endoscope
4. Technical problems
5. Technical features
   5.1. Schematic configuration
   5.2. First practical example
   5.3. Second practical example
   5.4. Modified example
6. Application examples
   6.1. Application Example 1: Example of control linked with arm
   6.2. Application Example 2: Control example of arm that supports oblique-viewing endoscope
7. Conclusion

### <<1. Configuration example of endoscope system>>

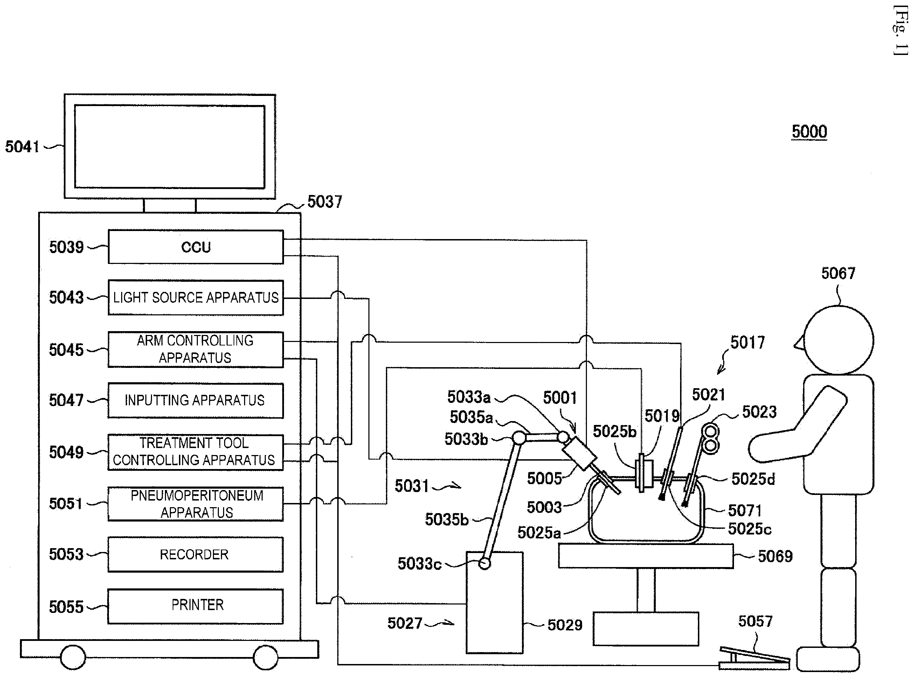

FIG. 1 is a view depicting an example of a schematic configuration of an endoscopic surgery system 5000 to which the technology according to the present disclosure can be applied. In FIG. 1, a state is illustrated in which a surgeon (medical doctor) 5067 is using the endoscopic surgery system 5000 to perform surgery for a patient 5071 on a patient bed 5069. As depicted, the endoscopic surgery system 5000 includes an endoscope 5001, other surgical tools 5017, a supporting arm apparatus 5027 which supports the endoscope 5001 thereon, and a cart 5037 on which various apparatus for endoscopic surgery are mounted.

In endoscopic surgery, in place of incision of the abdominal wall to perform laparotomy, a plurality of tubular aperture devices called trocars 5025a to 5025d are used to puncture the abdominal wall. Then, a lens barrel 5003 of the endoscope 5001 and the other surgical tools 5017 are inserted into body cavities of the patient 5071 through the trocars 5025a to 5025d. In the example depicted, as the other surgical tools 5017, a pneumoperitoneum tube 5019, an energy treatment tool 5021 and forceps 5023 are inserted into body cavities of the patient 5071. Further, the energy treatment tool 5021 is a treatment tool for performing incision and peeling of a tissue, sealing of a blood vessel or the like by high frequency current or ultrasonic vibration. However, the surgical tools 5017 depicted are mere examples at all, and as the surgical tools 5017, various surgical tools which are generally used in endoscopic surgery such as, for example, a pair of tweezers or a retractor may be used.

An image of a surgical region in a body cavity of the patient 5071 imaged by the endoscope 5001 is displayed on a display apparatus 5041. The surgeon 5067 would use the energy treatment tool 5021 or the forceps 5023 while watching the image of the surgical region displayed on the display apparatus 5041 on the real time basis to perform such treatment as, for example, resection of an affected area. Note that, though not depicted, the pneumoperitoneum tube 5019, the energy treatment tool 5021 and the forceps 5023 are supported by the surgeon 5067, an assistant or the like during surgery.

### (Supporting arm apparatus)

The supporting arm apparatus 5027 includes an arm unit 5031 extending from a base unit 5029. In the example depicted, the arm unit 5031 includes joint portions 5033a, 5033b and 5033c and links 5035a and 5035b and is driven under the control of an arm controlling apparatus 5045. The endoscope 5001 is supported by the arm unit 5031 such that the position and the posture of the endoscope 5001 are controlled. Consequently, stable fixation in position of the endoscope 5001 can be implemented.

### (Endoscope)

The endoscope 5001 includes the lens barrel 5003 which has a region of a predetermined length from a distal end thereof to be inserted into a body cavity of the patient 5071, and a camera head 5005 connected to a proximal end of the lens barrel 5003. In the example depicted, the endoscope 5001 is depicted which includes as a rigid endoscope having the lens barrel 5003 of the hard type. However, the endoscope 5001 may otherwise be configured as a flexible endoscope having the lens barrel 5003 of the soft type.

The lens barrel 5003 has, at a distal end thereof, an opening in which an objective lens is fitted. A light source apparatus 5043 is connected to the endoscope 5001 such that light generated by the light source apparatus 5043 is introduced to a distal end of the lens barrel by a light guide extending in the inside of the lens barrel 5003 and is irradiated toward an observation target in a body cavity of the patient 5071 through the objective lens. Note that the endoscope 5001 may be a straight-viewing endoscope or may be an oblique-viewing endoscope or a side-viewing endoscope.

An optical system and an image sensor are provided in the inside of the camera head 5005 such that reflected light (observation light) from an observation target is condensed on the image sensor by the optical system. The observation light is photoelectrically converted by the image sensor to generate an electric signal corresponding to the observation light, namely, an image signal corresponding to an observation image. The image signal is transmitted as RAW data to a CCU 5039. Noted that the camera head 5005 has a function incorporated therein for suitably driving the optical system of the camera head 5005 to adjust the magnification and the focal distance.

Noted that, in order to establish compatibility with, for example, a stereoscopic vision (three dimensional (3D) display), a plurality of image sensors may be provided on the camera head 5005. In this case, a plurality of relay optical systems are provided in the inside of the lens barrel 5003 in order to guide observation light to each of the plurality of image sensors.

### (Various apparatus incorporated in cart)

The CCU 5039 includes a central processing unit (CPU), a graphics processing unit (GPU) or the like and integrally controls operation of the endoscope 5001 and the display apparatus 5041. In particular, the CCU 5039 performs, for an image signal received from the camera head 5005, various image processes for displaying an image based on the image signal such as, for example, a development process (demosaic process) . The CCU 5039 provides the image signal for which the image processes have been performed to the display apparatus 5041. Further, the CCU 5039 transmits a control signal to the camera head 5005 to control driving of the camera head 5005. The control signal may include information relating to an image pickup condition such as a magnification or a focal distance.

The display apparatus 5041 displays an image based on an image signal for which the image processes have been performed by the CCU 5039 under the control of the CCU 5039. If the endoscope 5001 is ready for imaging of a high resolution such as 4K (horizontal pixel number 3840 × vertical pixel number 2160), 8K (horizontal pixel number 7680 × vertical pixel number 4320) or the like and/or ready for 3D display, then a display apparatus by which corresponding display of the high resolution and/or 3D display are possible may be used as the display apparatus 5041. Where the apparatus is ready for imaging of a high resolution such as 4K or 8K, if the display apparatus used as the display apparatus 5041 has a size of equal to or not less than 55 inches, then a more immersive experience can be obtained. Further, a plurality of display apparatus 5041 having different resolutions and/or different sizes may be provided in accordance with purposes.

The light source apparatus 5043 includes a light source such as, for example, a light emitting diode (LED) and supplies irradiation light for imaging of a surgical region to the endoscope 5001.

The arm controlling apparatus 5045 includes a processor such as, for example, a CPU and operates in accordance with a predetermined program to control driving of the arm unit 5031 of the supporting arm apparatus 5027 in accordance with a predetermined controlling method.

An inputting apparatus 5047 is an input interface for the endoscopic surgery system 5000. A user can perform inputting of various kinds of information or instruction inputting to the endoscopic surgery system 5000 through the inputting apparatus 5047. For example, the user would input various kinds of information relating to surgery such as physical information of a patient, information regarding a procedure of the surgery and so forth through the inputting apparatus 5047. Further, the user would input, for example, an instruction to drive the arm unit 5031, an instruction to change an image pickup condition (type of irradiation light, magnification, focal distance or the like) by the endoscope 5001, an instruction to drive the energy treatment tool 5021 or the like through the inputting apparatus 5047.

The type of the inputting apparatus 5047 is not limited and may be that of any one of various known inputting apparatus. As the inputting apparatus 5047, for example, a mouse, a keyboard, a touch panel, a switch, a foot switch 5057 and/or a lever or the like may be applied. Where a touch panel is used as the inputting apparatus 5047, it may be provided on the display face of the display apparatus 5041.

Otherwise, the inputting apparatus 5047 is a device to be mounted on a user such as, for example, a glasses type wearable device or a head mounted display (HMD), and various kinds of inputting are performed in response to a gesture or a line of sight of the user detected by any of the devices mentioned. Further, the inputting apparatus 5047 includes a camera which can detect a motion of a user, and various kinds of inputting are performed in response to a gesture or a line of sight of a user detected from a video imaged by the camera. Further, the inputting apparatus 5047 includes a microphone which can collect the voice of a user, and various kinds of inputting are performed by voice collected by the microphone. By configuring the inputting apparatus 5047 such that various kinds of information can be inputted in a contactless fashion in this manner, especially a user who belongs to a clean area (e.g., the surgeon 5067) can operate an apparatus belonging to an unclean area in a contactless fashion. Further, since the user can operate an apparatus without releasing a possessed surgical tool from its hand, the convenience to the user is improved.

A treatment tool controlling apparatus 5049 controls driving of the energy treatment tool 5021 for cautery or incision of a tissue, sealing of a blood vessel or the like. A pneumoperitoneum apparatus 5051 feeds gas into a body cavity of the patient 5071 through the pneumoperitoneum tube 5019 to inflate the body cavity in order to secure the field of view of the endoscope 5001 and secure the working space for the surgeon. A recorder 5053 is an apparatus capable of recording various kinds of information relating to surgery. A printer 5055 is an apparatus capable of printing various kinds of information relating to surgery in various forms such as a text, an image or a graph.

In the following, especially a characteristic configuration of the endoscopic surgery system 5000 is described in more detail.

### (Supporting arm apparatus)

The supporting arm apparatus 5027 includes the base unit 5029 serving as a base, and the arm unit 5031 extending from the base unit 5029. In the example depicted, the arm unit 5031 includes the plurality of joint portions 5033a, 5033b and 5033c and the plurality of links 5035a and 5035b connected to each other by the joint portion 5033b. In FIG. 1, for simplified illustration, the configuration of the arm unit 5031 is depicted in a simplified form. Actually, the shape, number and arrangement of the joint portions 5033a to 5033c and the links 5035a and 5035b and the direction and so forth of axes of rotation of the joint portions 5033a to 5033c can be set suitably such that the arm unit 5031 has a desired degree of freedom. For example, the arm unit 5031 may preferably be configured such that it has a degree of freedom equal to or not less than 6 degrees of freedom. This makes it possible to move the endoscope 5001 freely within the movable range of the arm unit 5031. Consequently, it becomes possible to insert the lens barrel 5003 of the endoscope 5001 from a desired direction into a body cavity of the patient 5071.

An actuator is provided in each of the joint portions 5033a to 5033c, and the joint portions 5033a to 5033c are configured such that they are rotatable about predetermined axes of rotation thereof by driving of the respective actuators. The driving of the actuators is controlled by the arm controlling apparatus 5045 to control the rotational angle of each of the joint portions 5033a to 5033c thereby to control driving of the arm unit 5031. Consequently, control of the position and the posture of the endoscope 5001 can be implemented. Thereupon, the arm controlling apparatus 5045 can control driving of the arm unit 5031 by various known controlling methods such as force control or position control.

For example, if the surgeon 5067 suitably performs operation inputting through the inputting apparatus 5047 (including the foot switch 5057), then driving of the arm unit 5031 may be controlled suitably by the arm controlling apparatus 5045 in response to the operation input to control the position and the posture of the endoscope 5001. After the endoscope 5001 at the distal end of the arm unit 5031 is moved from an arbitrary position to a different arbitrary position by the control just described, the endoscope 5001 can be supported fixedly at the position after the movement. Note that the arm unit 5031 may be operated in a master-slave fashion. In this case, the arm unit 5031 may be remotely controlled by the user through the inputting apparatus 5047 which is placed at a place remote from the surgery room.

Further, where force control is applied, the arm controlling apparatus 5045 may perform power-assisted control to drive the actuators of the joint portions 5033a to 5033c such that the arm unit 5031 may receive external force by the user and move smoothly following the external force. This makes it possible to move, when the user directly touches with and moves the arm unit 5031, the arm unit 5031 with comparatively weak force. Accordingly, it becomes possible for the user to move the endoscope 5001 more intuitively by a simpler and easier operation, and the convenience to the user can be improved.

Here, generally in endoscopic surgery, the endoscope 5001 is supported by a medical doctor called scopist. In contrast, where the supporting arm apparatus 5027 is used, the position of the endoscope 5001 can be fixed more certainly without hands, and therefore, an image of a surgical region can be obtained stably and surgery can be performed smoothly.

Note that the arm controlling apparatus 5045 may not necessarily be provided on the cart 5037. Further, the arm controlling apparatus 5045 may not necessarily be a single apparatus. For example, the arm controlling apparatus 5045 may be provided in each of the joint portions 5033a to 5033c of the arm unit 5031 of the supporting arm apparatus 5027 such that the plurality of arm controlling apparatus 5045 cooperate with each other to implement driving control of the arm unit 5031.

### (Light source apparatus)

The light source apparatus 5043 supplies irradiation light upon imaging of a surgical region to the endoscope 5001. The light source apparatus 5043 includes a white light source which includes, for example, an LED, a laser light source or a combination of them. In this case, where a white light source includes a combination of red, green, and blue (RGB) laser light sources, since the output intensity and the output timing can be controlled with a high degree of accuracy for each color (each wavelength), adjustment of the white balance of a picked up image can be performed by the light source apparatus 5043. Further, in this case, if laser beams from the respective RGB laser light sources are irradiated time-divisionally on an observation target and driving of the image sensors of the camera head 5005 is controlled in synchronism with the irradiation timings, then images individually corresponding to the R, G and B colors can be picked up time-divisionally. According to the method just described, a color image can be obtained even if a color filter is not provided for the image sensor.

Further, driving of the light source apparatus 5043 may be controlled such that the intensity of light to be outputted is changed for each predetermined time. By controlling driving of the image sensor of the camera head 5005 in synchronism with the timing of the change of the intensity of light to acquire images time-divisionally and synthesizing the images, an image of a high dynamic range free from underexposed blocked up shadows and overexposed highlights can be created.

Further, the light source apparatus 5043 may be configured to supply light of a predetermined wavelength band ready for special light observation. In special light observation, for example, by utilizing the wavelength dependency of absorption of light in a body tissue to irradiate light of a narrower band in comparison with irradiation light upon ordinary observation (namely, white light), narrow band light observation (narrow band imaging) of imaging a predetermined tissue such as a blood vessel of a superficial portion of the mucous membrane or the like in a high contrast is performed. Alternatively, in special light observation, fluorescent observation for obtaining an image from fluorescent light generated by irradiation of excitation light may be performed. In fluorescent observation, it is possible to perform observation of fluorescent light from a body tissue by irradiating excitation light on the body tissue (autofluorescence observation) or to obtain a fluorescent light image by locally injecting a reagent such as indocyanine green (ICG) into a body tissue and irradiating excitation light corresponding to a fluorescent light wavelength of the reagent upon the body tissue. The light source apparatus 5043 can be configured to supply such narrowband light and/or excitation light suitable for special light observation as described above.

### (Camera head and CCU)

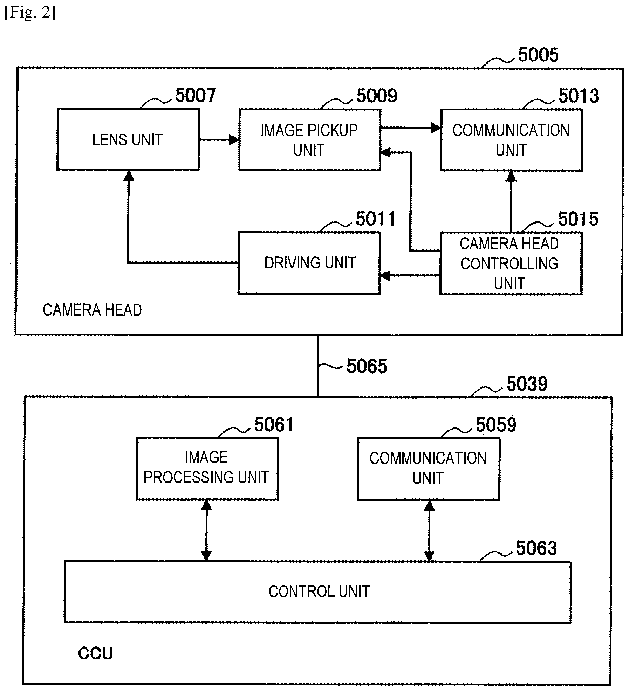

Functions of the camera head 5005 of the endoscope 5001 and the CCU 5039 are described in more detail with reference to FIG. 2. FIG. 2 is a block diagram depicting an example of a functional configuration of the camera head 5005 and the CCU 5039 depicted in FIG. 1.

Referring to FIG. 2, the camera head 5005 has, as functions thereof, a lens unit 5007, an, a driving unit 5011, a communication unit 5013 and a camera head controlling unit 5015. Further, the CCU 5039 has, as functions thereof, a communication unit 5059, an image processing unit 5061 and a control unit 5063. The camera head 5005 and the CCU 5039 are connected to be bidirectionally communicable to each other by a transmission cable 5065.

First, a functional configuration of the camera head 5005 is described. The lens unit 5007 is an optical system provided at a connecting location of the camera head 5005 to the lens barrel 5003. Observation light taken in from a distal end of the lens barrel 5003 is introduced into the camera head 5005 and enters the lens unit 5007. The lens unit 5007 includes a combination of a plurality of lenses including a zoom lens and a focusing lens. The lens unit 5007 has optical properties adjusted such that the observation light is condensed on a light receiving face of the image sensor of the image pickup unit 5009. Further, the zoom lens and the focusing lens are configured such that the positions thereof on their optical axis are movable for adjustment of the magnification and the focal point of a picked up image.

The image pickup unit 5009 includes an image sensor and disposed at a succeeding stage to the lens unit 5007. Observation light having passed through the lens unit 5007 is condensed on the light receiving face of the image sensor, and an image signal corresponding to the observation image is generated by photoelectric conversion of the image sensor. The image signal generated by the image pickup unit 5009 is provided to the communication unit 5013.

As the image sensor which is included by the image pickup unit 5009, an image sensor, for example, of the complementary metal oxide semiconductor (CMOS) type is used which has a Bayer array and is capable of picking up an image in color. Note that, as the image sensor, an image sensor may be used which is ready, for example, for imaging of an image of a high resolution equal to or not less than 4K. If an image of a surgical region is obtained in a high resolution, then the surgeon 5067 can comprehend a state of the surgical region in enhanced details and can proceed with the surgery more smoothly.

Further, the image sensor which is included by the image pickup unit 5009 includes such that it has a pair of image sensors for acquiring image signals for the right eye and the left eye compatible with 3D display. Where 3D display is applied, the surgeon 5067 can comprehend the depth of a living body tissue in the surgical region more accurately. Note that, if the image pickup unit 5009 is configured as that of the multiplate type, then a plurality of systems of lens units 5007 are provided corresponding to the individual image sensors of the image pickup unit 5009.

The image pickup unit 5009 may not necessarily be provided on the camera head 5005. For example, the image pickup unit 5009 may be provided just behind the objective lens in the inside of the lens barrel 5003.

The driving unit 5011 includes an actuator and moves the zoom lens and the focusing lens of the lens unit 5007 by a predetermined distance along the optical axis under the control of the camera head controlling unit 5015. Consequently, the magnification and the focal point of a picked up image by the image pickup unit 5009 can be adjusted suitably.

The communication unit 5013 includes a communication apparatus for transmitting and receiving various kinds of information to and from the CCU 5039. The communication unit 5013 transmits an image signal acquired from the image pickup unit 5009 as RAW data to the CCU 5039 through the transmission cable 5065. Thereupon, in order to display a picked up image of a surgical region in low latency, preferably the image signal is transmitted by optical communication. This is because, upon surgery, the surgeon 5067 performs surgery while observing the state of an affected area through a picked up image, it is demanded for a moving image of the surgical region to be displayed on the real time basis as far as possible in order to achieve surgery with a higher degree of safety and certainty. Where optical communication is applied, a photoelectric conversion module for converting an electric signal into an optical signal is provided in the communication unit 5013. After the image signal is converted into an optical signal by the photoelectric conversion module, it is transmitted to the CCU 5039 through the transmission cable 5065.

Further, the communication unit 5013 receives a control signal for controlling driving of the camera head 5005 from the CCU 5039. The control signal includes information relating to image pickup conditions such as, for example, information that a frame rate of a picked up image is designated, information that an exposure value upon image picking up is designated and/or information that a magnification and a focal point of a picked up image are designated. The communication unit 5013 provides the received control signal to the camera head controlling unit 5015. Note that also the control signal from the CCU 5039 may be transmitted by optical communication. In this case, a photoelectric conversion module for converting an optical signal into an electric signal is provided in the communication unit 5013. After the control signal is converted into an electric signal by the photoelectric conversion module, it is provided to the camera head controlling unit 5015.

Note that the image pickup conditions such as the frame rate, exposure value, magnification or focal point are set automatically by the control unit 5063 of the CCU 5039 on the basis of an acquired image signal. In other words, an auto exposure (AE) function, an auto focus (AF) function and an auto white balance (AWB) function are incorporated in the endoscope 5001.

The camera head controlling unit 5015 controls driving of the camera head 5005 on the basis of a control signal from the CCU 5039 received through the communication unit 5013. For example, the camera head controlling unit 5015 controls driving of the image sensor of the image pickup unit 5009 on the basis of information that a frame rate of a picked up image is designated and/or information that an exposure value upon image picking up is designated. Further, for example, the camera head controlling unit 5015 controls the driving unit 5011 to suitably move the zoom lens and the focus lens of the lens unit 5007 on the basis of information that a magnification and a focal point of a picked up image are designated. The camera head controlling unit 5015 may further include a function for storing information for identifying the lens barrel 5003 and/or the camera head 5005.

Note that, by disposing the components such as the lens unit 5007 and the image pickup unit 5009 in a sealed structure having high airtightness and waterproof, the camera head 5005 can be provided with resistance to an autoclave sterilization process.

Now, a functional configuration of the CCU 5039 is described. The communication unit 5059 includes a communication apparatus for transmitting and receiving various kinds of information to and from the camera head 5005. The communication unit 5059 receives an image signal transmitted thereto from the camera head 5005 through the transmission cable 5065. Thereupon, the image signal may be transmitted preferably by optical communication as described above. In this case, for the compatibility with optical communication, the communication unit 5059 includes a photoelectric conversion module for converting an optical signal into an electric signal. The communication unit 5059 provides the image signal after conversion into an electric signal to the image processing unit 5061.

Further, the communication unit 5059 transmits, to the camera head 5005, a control signal for controlling driving of the camera head 5005. The control signal may also be transmitted by optical communication.

The image processing unit 5061 performs various image processes for an image signal in the form of RAW data transmitted thereto from the camera head 5005. The image processes include various known signal processes such as, for example, a development process, an image quality improving process (a bandwidth enhancement process, a super-resolution process, a noise reduction (NR) process and/or an image stabilization process) and/or an enlargement process (electronic zooming process) . Further, the image processing unit 5061 performs a detection process for an image signal in order to perform AE, AF and AWB.

The image processing unit 5061 includes a processor such as a CPU or a GPU, and when the processor operates in accordance with a predetermined program, the image processes and the detection process described above can be performed. Note that, where the image processing unit 5061 includes a plurality of GPUs, the image processing unit 5061 suitably divides information relating to an image signal such that image processes are performed in parallel by the plurality of GPUs.

The control unit 5063 performs various kinds of control relating to image picking up of a surgical region by the endoscope 5001 and display of the picked up image. For example, the control unit 5063 generates a control signal for controlling driving of the camera head 5005. Thereupon, if image pickup conditions are inputted by the user, then the control unit 5063 generates a control signal on the basis of the input by the user. Alternatively, where the endoscope 5001 has an AE function, an AF function and an AWB function incorporated therein, the control unit 5063 suitably calculates an optimum exposure value, focal distance and white balance in response to a result of a detection process by the image processing unit 5061 and generates a control signal.

Further, the control unit 5063 controls the display apparatus 5041 to display an image of a surgical region on the basis of an image signal for which image processes have been performed by the image processing unit 5061. Thereupon, the control unit 5063 recognizes various objects in the surgical region image using various image recognition technologies. For example, the control unit 5063 can recognize a surgical tool such as forceps, a particular living body region, bleeding, mist when the energy treatment tool 5021 is used and so forth by detecting the shape, color and so forth of edges of the objects included in the surgical region image. The control unit 5063 causes, when it controls the display unit 5041 to display a surgical region image, various kinds of surgery supporting information to be displayed in an overlapping manner with an image of the surgical region using a result of the recognition. Where surgery supporting information is displayed in an overlapping manner and presented to the surgeon 5067, the surgeon 5067 can proceed with the surgery more safety and certainty.

The transmission cable 5065 which connects the camera head 5005 and the CCU 5039 to each other is an electric signal cable ready for communication of an electric signal, an optical fiber ready for optical communication or a composite cable ready for both of electrical and optical communication.

Here, while, in the example depicted, communication is performed by wired communication using the transmission cable 5065, the communication between the camera head 5005 and the CCU 5039 may be performed otherwise by wireless communication. Where the communication between the camera head 5005 and the CCU 5039 is performed by wireless communication, there is no necessity to lay the transmission cable 5065 in the surgery room. Therefore, such a situation that movement of medical staff in the surgery room is disturbed by the transmission cable 5065 can be eliminated.

An example of the endoscopic surgery system 5000 to which the technology according to the present disclosure can be applied has been described above. It is to be noted here that, although the endoscopic surgery system 5000 has been described as an example, the system to which the technology according to the present disclosure can be applied is not limited to the example. For example, the technology according to the present disclosure may be applied to a flexible endoscopic system for inspection or a microscopic surgery system.

### <<2. Configuration example of supporting arm apparatus>>

Next, an example of a configuration of a supporting arm apparatus to which the technology according to an embodiment of the present disclosure can be applied will be described below. Although the supporting arm apparatus that will be described below is an example of a supporting arm apparatus configured to support an endoscope at the distal end of an arm unit, the present embodiment is not limited thereto. In addition, in a case in which a supporting arm apparatus according to an embodiment of the present disclosure is applied to the medical field, the supporting arm apparatus can function as a medical supporting arm apparatus.

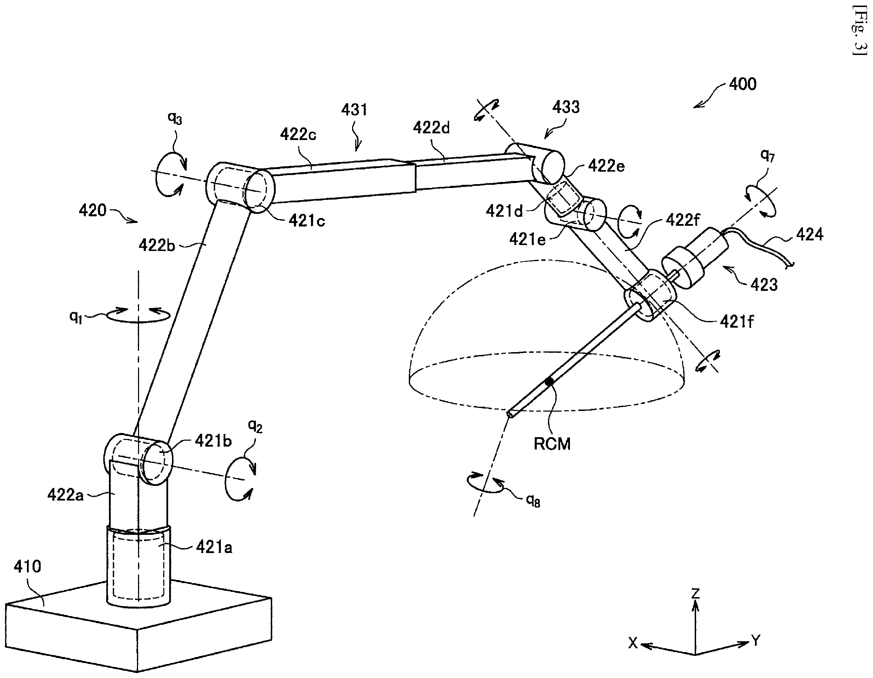

FIG. 3 is a perspective diagram illustrating an appearance of a supporting arm apparatus 400 according to the present embodiment. As illustrated in FIG. 3, the supporting arm apparatus 400 according to the present embodiment includes a base unit 410 and an arm unit 420. The base unit 410 is a base of the supporting arm apparatus 400, and the arm unit 420 extends from the base unit 410. Although not illustrated in FIG. 3, a control unit configured to integrally control the supporting arm apparatus 400 may be provided in the base unit 410, and driving of the arm unit 420 may be controlled by the control unit. The control unit is constituted by various signal processing circuits such as a central processing unit (CPU) or a digital signal processor (DSP).

The arm unit 420 has a plurality of active joint portions 421a to 421f, a plurality of links 422a to 422f, and an endoscope apparatus 423 as a distal end unit provided at a distal end of the arm unit 420.

The links 422a to 422f are substantially bar-like members. One end of the link 422a is connected to the base unit 410 through the active joint portion 421a, the other end of the link 422a is connected to one end of the link 422b through the active joint portion 421b, and the other end of the link 422b is connected to one end of the link 422c through the active joint portion 421c. The other end of the link 422c is connected to the link 422d through a passive sliding mechanism 431, and the other end of the link 422d is connected to one end of the link 422e through a passive joint portion 200. The other end of the link 422e is connected to one end of the link 422f through the active joint portions 421d and 421e. The endoscope apparatus 423 is connected to the distal end of the arm unit 420, that is, the other end of the link 422f, through the active joint portion 421f. By the ends of the plurality of links 422a to 422f being connected to each other by the active joint portions 421a to 421f, the passive sliding mechanism 431, and the passive joint portion 433 with the base unit 410 as a fulcrum as described above, a shape of an arm extending from the base unit 410 is configured.

A position and attitude of the endoscope apparatus 423 are controlled by actuators, which are respectively provided at the active joint portions 421a to 421f of the arm unit 420, being drive-controlled. The distal end of the endoscope apparatus 423 enters a body cavity of a patient, which is a treatment site, and images a partial region of the treatment site. However, the distal end unit provided at the distal end of the arm unit 420 is not limited to the endoscope apparatus 423, and various other medical mechanisms may be connected to the distal end of the arm unit 420 as the distal end unit. As described above, the supporting arm apparatus 400 is configured as a medical supporting arm apparatus including a medical mechanism.

Here, in the following description, the supporting arm apparatus 400 will be described by defining coordinate axes as illustrated in FIG. 3. Also, a vertical direction, a longitudinal direction, and a horizontal direction are defined in accordance with the coordinate aces. That is, a vertical direction with respect to the base unit 410 provided at a floor surface is defined as the z-axis direction and the vertical direction. Also, a direction in which the arm unit 420 extends from the base unit 410 (that is, a direction in which the endoscope apparatus 423 is positioned with respect to the base unit 410), which is a direction orthogonal to the z-axis, is defined as the y-axis direction and the longitudinal direction. Further, a direction orthogonal to the y-axis and the z-axis is defined as the x-axis direction and the horizontal direction.

The active joint portions 421a to 421f connect the links to each other such that the links are rotatable. The active joint portions 421a to 421f have an actuator and a rotation mechanism that is rotation-driven with respect to a predetermined rotation axis by driving of the actuator. By separately controlling rotation-driving of each of the active joint portions 421a to 421f, it is possible to control driving of the arm unit 420, for example, expanding or contracting (folding) the arm unit 420. Here, driving of the active joint portions 421a to 421f may be controlled by known body cooperative control and ideal joint control. Since the active joint portions 421a to 421f have the rotation mechanism as described above, in the following description, driving control of the active joint portions 421a to 421f specifically refers to control of a rotational angle and/or a generated torque (torque caused to be generated by the active joint portions 421a to 421f) of the active joint portions 421a to 421f.

The passive sliding mechanism 431 is a mode of a passive form changing mechanism, and connects the link 422c and the link 422d such that the link 422c and the link 422d are able to reciprocate relative to each other in a predetermined direction. For example, the passive sliding mechanism 431 may connect the link 422c and the link 422d such that the link 422c and the link 422d are able to linearly move relative to each other. However, the reciprocating motion of the link 422c and the link 422d is not limited to the linear motion and may also be a reciprocating motion in a direction forming an arc shape. For example, a reciprocating operation of the passive sliding mechanism 431 is performed by a user, and a distance between the active joint portion 421c at one end side of the link 422c and the passive joint portion 433 is set to vary. Consequently, an overall form of the arm unit 420 can be changed.

The passive joint portion 433 is a mode of a passive form changing mechanism, and connects the link 422d and the link 422e such that the link 422d and the link 422e are able to rotate relative to each other. For example, a rotating operation of the passive joint portion 433 is performed by the user, and an angle formed between the link 422d and the link 422e is set to vary. Consequently, an overall form of the arm unit 420 can be changed.

In the present specification, "attitude of an arm unit" refers to a state of an arm unit that can be changed by driving control of the actuator provided at the active joint portions 421a to 421f by a control unit in a state in which a distance between neighboring active joint portions with one or a plurality of links sandwiched therebetween is constant. Also, "form of an arm unit" refers to a state of an arm unit that can be changed due to a change in a distance between neighboring active joint portions with links sandwiched therebetween or a change in an angle formed between the links connecting the neighboring active joint portions in accordance with the passive form changing mechanism being operated.

The supporting arm apparatus 400 has six active joint portions 421a to 421f, and six degrees of freedom is realized therein with respect to driving of the arm unit 420. That is, while driving control of the supporting arm apparatus 400 is realized by driving control of the six active joint portions 421a to 421f by the control unit, the passive sliding mechanism 431 and the passive joint portion 433 are not subject to driving control by the control unit.

Specifically, as illustrated in FIG. 3, the active joint portions 421a, 421d, and 421f are provided such that long-axis directions of the links 422a and 422e connected to the active joint portions 421a and 421d, respectively, and an imaging direction of the endoscope apparatus 423 connected to the 421f are set to be rotation axis directions of the active joint portions 421a, 421d, and 421f. The active joint portions 421b, 421c, and 421e are provided such that an x-axis direction, which is a direction in which connection angles of each of the links 422a to 422c, 422e, 422f, and the endoscope apparatus 423 connected to the active joint portions 421b, 421c, and 421e are changed in a y-z plane (the plane defined by the y-axis and the z-axis), is set to be a rotation axis direction. As described above, the active joint portions 421a, 421d, and 421f have a function of performing so-called yawing, and the active joint portions 421b, 421c, and 421e have a function of performing so-called pitching.

By having such a configuration of the arm unit 420, since six degrees of freedom is realized with respect to driving of the arm unit 420 in the supporting arm apparatus 400 according to the present embodiment, it is possible to cause the endoscope apparatus 423 to freely move within a movable range of the arm unit 420. In FIG. 3, a hemisphere is illustrated as an example of a movable range of the endoscope apparatus 423. If a central point RCM (remote center of motion) of the hemisphere is an imaging center of a treatment site imaged by the endoscope apparatus 423, by causing the endoscope apparatus 423 on a spherical surface of the hemisphere in a state in which the imaging center of the endoscope apparatus 423 is fixed to the central point of the hemisphere, it is possible to image the treatment site from various angles.

The example of the configuration of the supporting arm apparatus to which the technology according to the present disclosure can be applied has been described above.

### <<3. Basic configuration of oblique-viewing endoscope>>

Next, a basic configuration of an oblique-viewing endoscope as an example of an endoscope will be described.

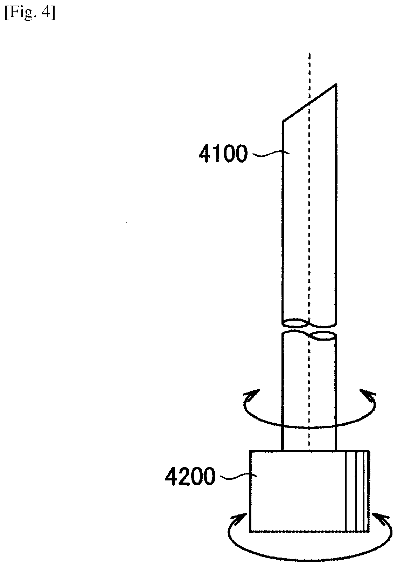

FIG. 4 is a schematic diagram illustrating a configuration of an oblique-viewing endoscope 4100 according to the present disclosure. As illustrated in FIG. 4, the oblique-viewing endoscope 4100 is mounted at the distal end of a camera head 4200. The oblique-viewing endoscope 4100 corresponds to the lens barrel 5003 described in FIGS. 1 and 2, and the camera head 4200 corresponds to the camera head 5005 described in FIGS. 1 and 2. The oblique-viewing endoscope 4100 and the camera head 4200 can rotate independently of each other. An actuator is provided between the oblique-viewing endoscope 4100 and the camera head 4200, as in each of joint portions 5033a, 5033b, and 5033c, and the oblique-viewing endoscope 4100 is rotated with respect to the camera head 4200 by driving of the actuator. Accordingly, a rotation angle θ_{z} which will be described below is controlled.

The oblique-viewing endoscope 4100 is supported by the supporting arm apparatus 5027. The supporting arm apparatus 5027 holds the oblique-viewing endoscope 4100, instead of a scopist, and has a function of moving the oblique-viewing endoscope 4100 such that a desired site can be observed through an operation of an operator or an assistant.

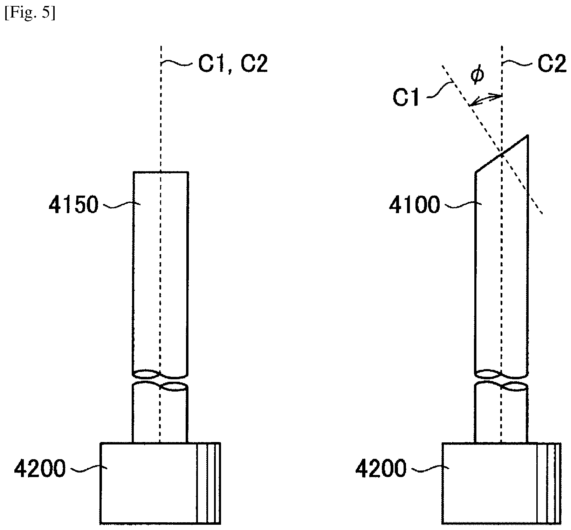

FIG. 5 is a schematic diagram illustrating a comparison of the oblique-viewing endoscope 4100 and a straight-viewing endoscope 4150. In the straight-viewing endoscope 4150, an orientation of an objective lens toward a subject (C1) coincides with the longitudinal direction of the straight-viewing endoscope 4150 (C2). On the other hand, in the oblique-viewing endoscope 4100, the orientation of an objective lens toward a subject (C1) has a predetermined angle ϕ with respect to the longitudinal direction of the oblique-viewing endoscope 4100 (C2) .

The basic configuration of the oblique-viewing endoscope has been described above as an example of an endoscope.

### <<4. Technical problems>>

Next, technical problems of a medical imaging apparatus such as an endoscope or a microscope will be described below.

There are many cases with respect to a medical optical device such as endoscopes and microscopes in which camera heads and optical systems are designed to correspond to each other one to one or to be optimized in accordance with their types. In a case in which such devices are used, for example, it is necessary to change a device to be used in accordance with a procedure such that a camera head for a rigid endoscope is used in a case in which a rigid endoscope is used as an optical system, a camera head for a microscope is used in a case in which a microscope is used as an optical system, or the like. In such a situation in which a device to be used is changed in accordance with a procedure, there are cases in which medical staff need to individually learn an operation method of each device and a time required for learning the operation method of each device increases. In addition, since individual devices vary in accordance with procedures, labor, storage places, purchase costs, and the like relating to maintenance and preparation of the devices increase in accordance with types thereof, which can be a burden to medical staff.

In addition, there are cases in which a medical optical device has a varying method of use depending on its applications. As a specific example, a case in which an oblique-viewing endoscope is used as a rigid endoscope may be exemplified. In this case, for example, since a visual field is expanded by turning the oblique-viewing endoscope, an operation of rotating the rigid endoscope (the oblique-viewing endoscope) with respect to a camera head is necessary such that a direction in which the rigid endoscope is attached to the camera head is changed. Of course, although the example discusses an oblique-viewing endoscope, the disclosure is not so limited and may be applicable to any endoscope which may be rotated relative to the camera head.

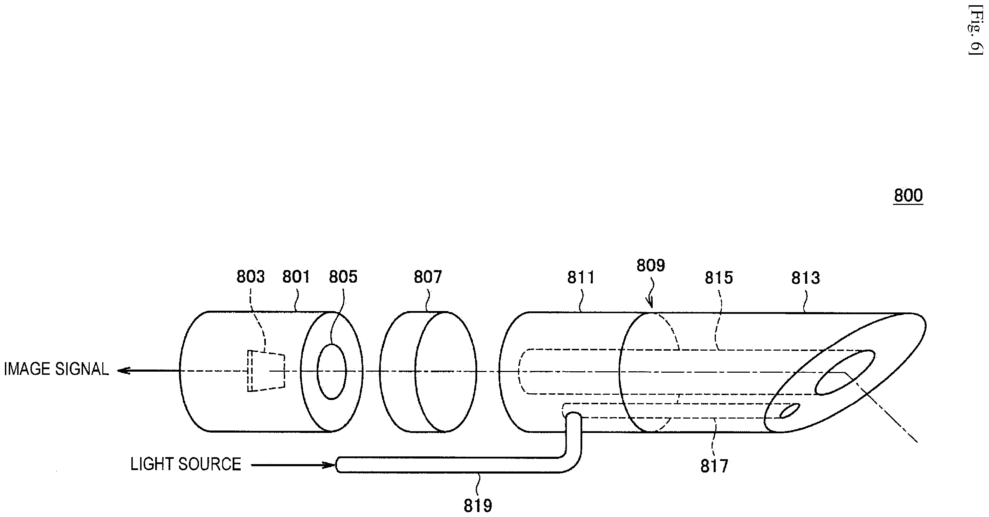

Here, as an example of a method of use of a medical optical device in accordance with an application, an example of a configuration of a medical imaging apparatus will be described in detail, focusing on a case in which an oblique-viewing endoscope is used as a rigid endoscope. For example, FIG. 6 is an explanatory diagram for describing an example of a configuration of a medical imaging apparatus according to Comparative Example 1, illustrating an example of a schematic configuration of a case in which an oblique-viewing endoscope is used as a rigid endoscope. Note that shapes and sizes of the camera head and the endoscope (oblique-viewing endoscope) are schematically illustrated in the example of FIG. 6 to make description easier to understand.

A medical imaging apparatus 800 according to Comparative Example 1 includes a camera head 801, a rigid endoscope 809, and an adaptor 807 as illustrated in FIG. 6.

The camera head 801 includes an image sensor 803, an opening is provided in the imaging direction of the image sensor 803, and light incident from the opening into the inside of the camera head 801 forms an image on the image sensor 803. In addition, the camera head 801 may have an imaging optical system 805 (e.g., an image forming optical system, etc.) in the front stage of the image sensor 803. In this case, light incident from the opening into the inside of the camera head 801 forms an image on the image sensor 803 via the imaging optical system 805.

The rigid endoscope 809 includes a lens barrel 813 and a base unit 811 supporting the lens barrel 813. The lens barrel 813 has an imaging optical system 815 and a light source supply system 817 such that the systems extend in the direction in which the lens barrel 813 extends (i.e., the axial direction of the cylindrical part). In addition, the rigid endoscope 809 is detachable from the camera head 801 via the adaptor 807. Specifically, the rigid endoscope 809 is attached to the camera head 801 via the adaptor 807 such that the end of the base unit 811 on the side opposite to the side thereof supporting the lens barrel 813 (which will also be referred to as a "rear end" below) is positioned in the front stage of the image sensor 803 of the camera head 801. In addition, at this time, in the case in which the rigid endoscope 809 is attached to the camera head 801, the rigid endoscope is supported to be rotatable with respect to the camera head 801 using the direction in which the lens barrel 813 extends as a rotation axis.

The imaging optical system 815 corresponds to one or more optical systems for acquiring an image of an affected area to be observed, and is provided to penetrate the lens barrel 813 and the base unit 811 in the direction in which the lens barrel 813 extends. Note that, although the imaging optical system 815 is illustrated in a tubular shape in the example illustrated in FIG. 6, this is merely a schematic illustration of the imaging optical system 815, and does not necessarily limit a configuration of the imaging optical system 815. As a specific example, the imaging optical system 815 may be configured by providing an opening penetrating the lens barrel 813 and the base unit 811 in the direction in which the lens barrel 813 extends and one or more optical systems (e.g., an objective lens, and the like) in the opening. In such a configuration, light incident from a distal end of the lens barrel 813 into the imaging optical system 815 is emitted from the rear end of the base unit 811 via the imaging optical system 815. That is, by attaching the rigid endoscope 809 to the camera head 801, light guided through the imaging optical system 815 is incident into the camera head 801 via the adaptor 807 and forms an image on the image sensor 803 via the imaging optical system 805.

The light source supply system 817 corresponds to a so-called light guide and guides light from a light source apparatus (e.g., the light source apparatus 5043 illustrated in FIG. 1) to the affected area. The light source supply system 817 can include various optical systems, for example, optical fibers, one or more lenses, and the like. Note that, although the light source supply system 817 is illustrated in a tubular shape in the example of FIG. 6, this is merely a schematic illustration of the light source supply system 817 and does not limit a configuration of the light source supply system 817. That is, at least a part of the light source supply system 817 may be constituted by optical fibers, or at least a part thereof may be constituted by one or more lenses and the like.

Note that no light source supply system is provided on the camera head 801 side in the medical imaging apparatus 800. For this reason, a light source supply system 819 such as a light guide cable is attached to the rigid endoscope 809, independently of the camera head 801. That is, the light source supply system 819 is connected to the base unit 811 at a position at which the end thereof opposite to the end connected to the light source apparatus is different from the rear end of the base unit 811 to which the camera head 801 is attached. For example, a connection part is provided such that the connection part protrudes from the base unit 811 in a radial direction in a case in which the extension direction of the lens barrel 813 is used as an axis in the example illustrated in FIG. 6, and the light source supply system 819 is connected to the connection part.

With the above-described configuration, the image sensor 803, the imaging optical system 805, and the imaging optical system 815 are disposed such that the optical axis thereof substantially coincides with the rotation axis of the rigid endoscope 809 in the medical imaging apparatus 800. Accordingly, even in a case in which the rigid endoscope 809 is rotated with respect to the camera head 801, a state in which an image of the affected area acquired by the imaging optical system 815 can be formed on the image sensor 803 is maintained in the medical imaging apparatus 800.

Meanwhile, since the light source supply system 819 is separately connected to the rigid endoscope 809 without going through the camera head 801 in the medical imaging apparatus 800 illustrated in FIG. 6, the connection part of the light source supply system 819 protrudes from the rigid endoscope 809 in the radial direction with respect to the rotation axis. Thus, in the case in which the rigid endoscope 809 is rotated with respect to the camera head 801, there are cases in which an operator may be hindered from handling the medical imaging apparatus 800 (e.g., an operation of rotating the rigid endoscope 809) due to the connection part and the light source supply system 819 connected to further extend from the connection part.

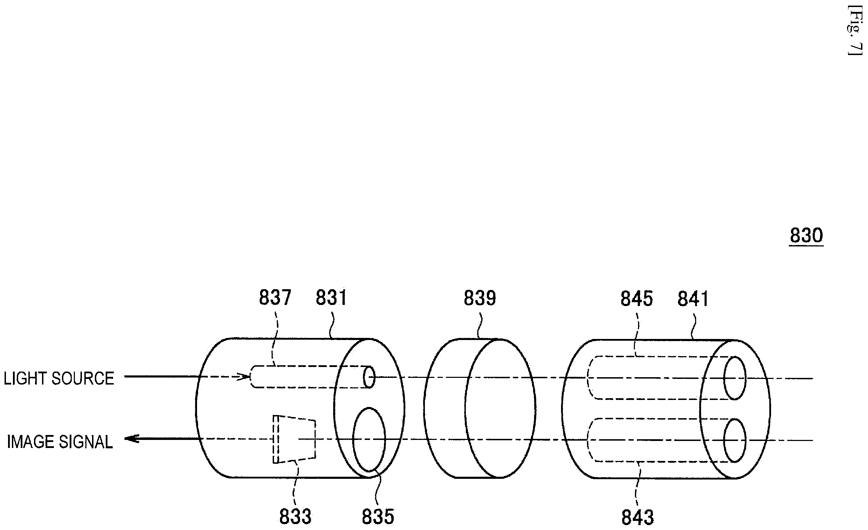

As a method for solving the problem, for example, a method of connecting the light source supply system as well as an imaging optical system to the rigid endoscope from the camera head side via an adaptor, is conceivable. For example, FIG. 7 is an explanatory diagram for describing an example of a configuration of a medical imaging apparatus according to Comparative Example 2, illustrating an example of a schematic configuration of the case in which the light source supply system as well as an imaging optical system is connected to the rigid endoscope from the camera head side via an adaptor. Note that, in the example illustrated in FIG. 7, shapes and sizes of a camera head and an endoscope are schematically illustrated to make the description easier to understand.

A medical imaging apparatus 830 according to Comparative Example 2 includes a camera head 831, a rigid endoscope 841, and an adaptor 839 as illustrated in FIG. 7. Note that the camera head 831, the rigid endoscope 841, and the adaptor 839 correspond to each of the camera head 801, the rigid endoscope 809, and the adaptor 807 of the medical imaging apparatus 800 illustrated in FIG. 6. Meanwhile, the medical imaging apparatus 830 illustrated in FIG. 7 has an imaging optical system and a light source supply system each provided in the camera head 831 and the rigid endoscope 841 with different configurations from the medical imaging apparatus 800 described with reference to FIG. 6. Thus, the configuration of the medical imaging apparatus 830 according to Comparative Example 2 will be described below particularly focusing on differences from the medical imaging apparatus 800 illustrated in FIG. 6, and detailed description of parts substantially similar to the medical imaging apparatus 800 will be omitted.

The camera head 831 includes a light source supply system 837, in addition to an image sensor 833, an imaging optical system 835 provided in the front stage of the image sensor 833 as illustrated in FIG. 7. The light source supply system 837 is provided to penetrate the camera head 831 in an extension direction of the rigid endoscope 841 (i.e., along a rotation axis of the rigid endoscope 841) attached to the camera head 831 via the adaptor 839. Note that, in order to secure an installation space for the light source supply system 837 in the camera head 831, installation positions of the image sensor 833 and the imaging optical system 835 are different from those in the camera head 801 illustrated in FIG. 6. Specifically, the image sensor 833 and the imaging optical system 835 are installed in the camera head 831 illustrated in FIG. 7 such that the optical axis of each of the image sensor 833 and the imaging optical system 835 is separated from the rotation axis of the rigid endoscope 841 in a radial direction from the rotation axis. Likewise, the light source supply system 837 is installed such that an optical axis of the light source supply system 837 is separated from the rotation axis of the rigid endoscope 841 in the radial direction from the rotation axis.

An imaging optical system 843 and a light source supply system 845 are provided in the rigid endoscope 841 on the basis of the above-described configuration of the camera head 831. That is, the imaging optical system 843 is provided in the rigid endoscope 841 such that the imaging optical system 843 is positioned in the front stage of the image sensor 833 in a case in which the rigid endoscope 841 is attached to the camera head 831 in the medical imaging apparatus 830 illustrated in FIG. 7. Likewise, the light source supply system 845 is provided in the rigid endoscope 841 such that the light source supply system 845 is positioned in the front stage of the light source supply system 837 in the case in which the rigid endoscope 841 is attached to the camera head 831. With this configuration, it is not necessary for the medical imaging apparatus 830 to have a connection part for connecting a light source supply system to the rigid endoscope 841, independently of the camera head 831.

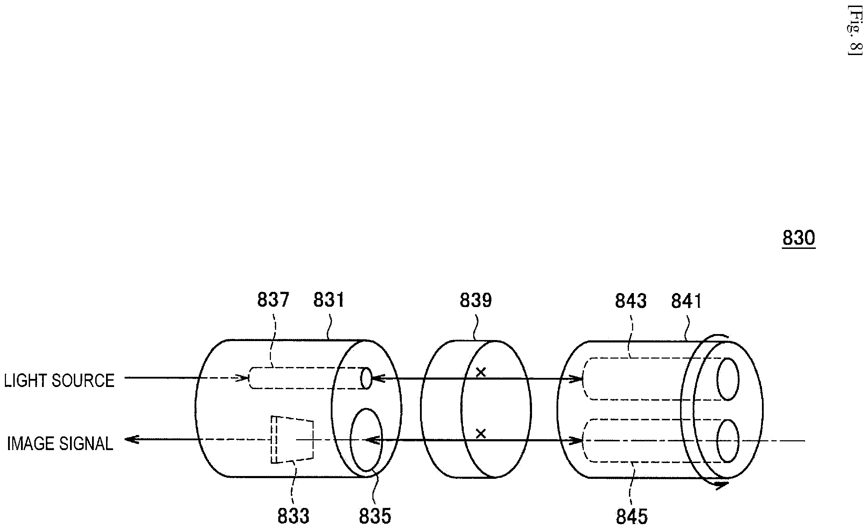

However, in the medical imaging apparatus 830 illustrated in FIG. 7, there is a difficulty in making the rigid endoscope 841 rotatable with respect to the camera head 831. For example, FIG. 8 is an explanatory diagram for describing another aspect of the medical imaging apparatus 830 according to Comparative Example 2, illustrating an example of a schematic configuration of a case in which the rigid endoscope 841 is rotated with respect to the camera head 831.

As described above, in the medical imaging apparatus 830 according to Comparative Example 2, each of optical axes of the image sensor 833 and light source supply system 837 does not coincide with the rotation axis of the rigid endoscope 841. For this reason, in a case in which the rigid endoscope 841 is rotated with respect to the camera head 831, it may be difficult to maintain the state in which the imaging optical system 843 is positioned in the front stage of the image sensor 833 as illustrated in FIG. 8. That is, in this case, it may be difficult to form an image of an affected area acquired by the imaging optical system 843 on the image sensor 833. Likewise, in the case in which the rigid endoscope 841 is rotated with respect to the camera head 831, it may be difficult to maintain the state in which the light source supply system 845 is positioned in the latter stage of the light source supply system 837. That is, it may be difficult to guide light guided from a light source apparatus by light source supply system 837 to the affected area via the light source supply system 845. Note that one of the image sensor 833 and the light source supply system 837 can also be disposed such that the optical axis thereof coincides with the rotation axis. Also in such a case, however, since the optical axis of the other one does not coincide with the rotation axis, it is still difficult to make the rigid endoscope 841 rotatable with respect to the camera head 831.

That is, it is necessary in the medical imaging apparatus 830 illustrated in FIG. 7 to rotate the rigid endoscope 841 along with the camera head 831 in an integrated manner to rotate the rigid endoscope 841 using the direction in which the lens barrel of the rigid endoscope 841 extends as an axis. For this reason, the medical imaging apparatus 830 is likely to have worse operability than in the case in which the rigid endoscope 809 is rotated with respect to the camera head 801, like the medical imaging apparatus 800 illustrated in FIG. 6, due to a different reason from that in the case of the medical imaging apparatus 800. In addition, there may be a case with respect to the medical imaging apparatus 830 in which it is difficult to maintain hand-eye coordination (i.e., cooperation between the sense of a hand and the sense of the eyes (vision)) of an operator since, when the camera head 831 is rotated, the image sensor 833 also rotates along therewith.

Taking the above-described circumstances into consideration, the present disclosure proposes an example of a technology for a medical imaging apparatus that enables an affected area to be observed using a medical optical apparatus such as an endoscope or a microscope, the technology being for further improving operability for observation of the affected area. In addition, the present disclosure proposes an example of a technology that can further reduce a burden on medical staff when acquiring an operation method, labor, storage places, purchase costs, and the like relating to maintenance and preparation of an apparatus in a situation in which an apparatus (e.g., a medical optical apparatus) to be used is changed in accordance with a procedure by sharing at least a part of a configuration (e.g., a camera head).

### <<5. Technical features>>

Technical features of a medical imaging apparatus according to the present disclosure will be described below.

### <5.1. Schematic configuration>

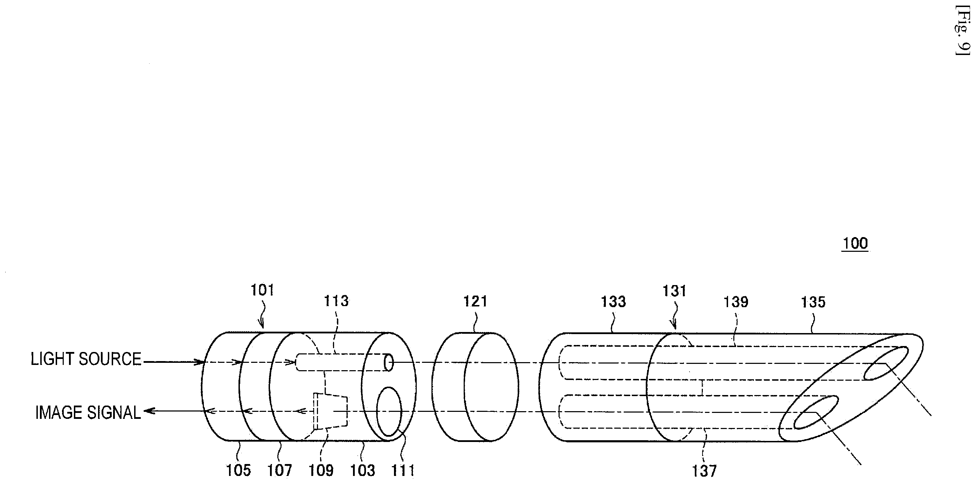

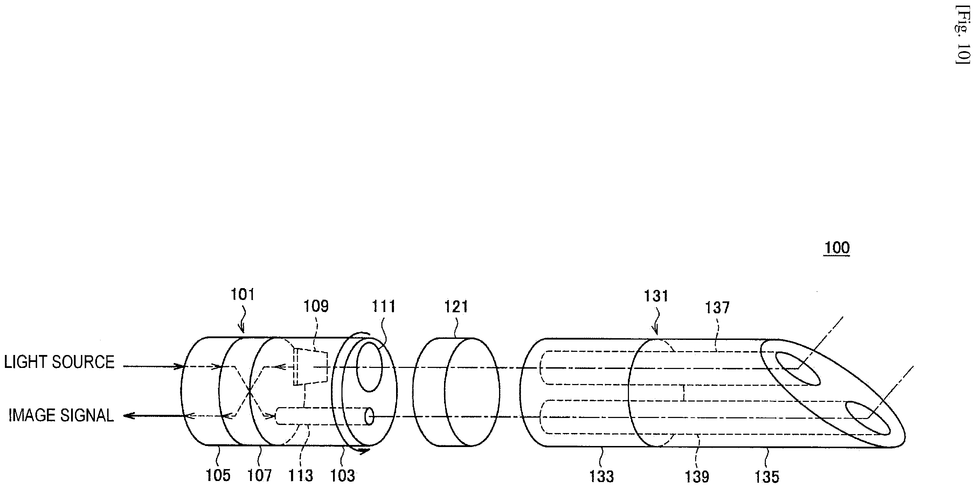

First, an example of a schematic configuration of a medical imaging apparatus according to the present disclosure will be described. For example, FIGS. 9 and 10 are explanatory diagrams for describing examples of schematic configurations of the medical imaging apparatus according to the present disclosure. Note that an example of a case in which a rigid endoscope (endoscope) is applied as a medical optical apparatus is introduced in the examples of FIGS. 9 and 10. In addition, FIGS. 9 and 10 schematically illustrate shapes and sizes of the camera head and the endoscope (oblique-viewing endoscope) to make features of the medical imaging apparatus according to the present disclosure easier to understand.

First, an example of a schematic configuration of a medical imaging apparatus 100 will be described with reference to FIG. 9. The medical imaging apparatus 100 includes the camera head 101, the rigid endoscope 131, and an adaptor 121 as illustrated in FIG. 9.

The camera head 101 includes a support unit 103, a base unit 105, and a drive unit 107.

The support unit 103 supports an image sensor 109 and a light source supply system 113. As a more specific example, the image sensor 109 and the light source supply system 113 are built into a housing of the support unit 103 in the example illustrated in FIG. 9. Note that, for the sake of convenience, an imaging direction of the image sensor (e.g., the image sensor 109 illustrated in FIG. 9) supported by the camera head (i.e., the right direction in FIG. 9) will also be referred to as a "front side" and the direction opposite to the imaging direction (i.e., the left direction in FIG. 9) will also be referred to as a "rear side" in the following description. In addition, the image sensor 109 corresponds to an example of an "imaging unit."

The support unit 103 has an opening in the front stage of the image sensor 109, and light from the opening incident onto the support unit 103 forms an image on the image sensor 109. In addition, the support unit 103 may support an imaging optical system 111 in the opening such that the imaging optical system 111 is positioned in the front stage (in front) of the image sensor 109. In this case, light from the opening incident onto the support unit 103 forms an image on the image sensor 109 via the imaging optical system 111. In addition, the light source supply system 113 is supported by the support unit 103 such that the light source supply system penetrates the support unit 103 from the rear side to the front side.

In addition, the rigid endoscope 131 is detachable from the support unit 103 via the adaptor 121 so that the rigid endoscope 131 is positioned on the front side of the image sensor 109 and the light source supply system 113.

The light source supply system 113 corresponds to a so-called light guide, and guides light from a light source apparatus (e.g., the light source apparatus 5043 illustrated in FIG. 1) to the rigid endoscope 131 attached to the front side of the support unit 103. The light source supply system 113 can be constituted by, for example, optical fibers, one or more optical systems, and the like.

The base unit 105 corresponds to a portion of the camera head 101 to which an arm unit is connected (i.e., a portion supported by the arm unit) in a case in which the camera head 101 is attached to a supporting arm apparatus. The base unit 105 supports the support unit 103 via the drive unit 107. At this time, a rear end side of the support unit 103 (i.e., the end side opposite to the imaging direction of the image sensor 109) is supported by a front end side of the base unit 105. In addition, the drive unit 107 causes the support unit 103 to rotate with respect to the base unit 105 about an axis C10 extending from the rear side to the front side (i.e., a rotation axis extending in the imaging direction of the image sensor 109). Note that, at this time, the axis C10 may not necessarily coincide with an optical axis of the image sensor 109. That is, the image sensor 109 may be installed such that the optical axis of the image sensor 109 is separated from the axis C10 in a radial direction around the axis C10. Likewise, the axis C10 may not necessarily coincide with an optical axis of the light source supply system 113. Additionally, the drive unit 107 may or may not be required to rotate the support unit 103 about the axis C10. Note that the front end of the base unit 105 (i.e., the end supporting the support unit 103) corresponds to an example of a "first end," and the rear end of the support unit 103 (i.e., the end supported by the base unit 105) corresponds to an example of a "second end."

With the above-described configuration, a signal line for supplying power to the image sensor 109 is supported in the base unit 105 such that the signal line extends from the rear side to the front side of the base unit 105. In addition, the signal line is electrically connected to the image sensor 109 via the rear end of the support unit 103. In addition, the signal line may partly branch in the support unit 103 and be able to supply power to the rigid endoscope 131 attached to the front side of the support unit 103. In addition, similarly to the signal line, a signal line for transmitting an image signal in accordance with an imaging result of the image sensor 109 to a predetermined output destination (e.g., the CCU 5039 illustrated in FIG. 1, etc.) may be provided. In addition, similarly to the signal line, a signal line for transmitting a control signal for controlling an operation of the image sensor 109 from outside the camera head 101 (e.g., the CCU 5039 illustrated in FIG. 1, etc.) to the image sensor 109 may be provided. Note that the signal line for supplying power to the image sensor 109, the signal line for transmitting the image signal, and the signal line for transmitting the control signal may be commonly used. Thus, in the following description, a case in which the signal line for supplying power to the image sensor 109, the signal line for transmitting the image signal, and the signal line for transmitting the control signal are commonly used will be described as an example. Of course, the image sensor 109 may be connected wirelessly to the CCU 5039 rather than using a wired connection. Accordingly, a wired connection is not required.

In addition, the light source supply system that guides light from the light source apparatus is also supported in the base unit 105 such that the light source supply system extends from the rear side to the front side of the base unit 105, similarly to the signal line. Note that the light source supply system supported in the base unit 105 may be integrated with the light source supply system 113 supported by the support unit 103. In addition, as another example, the light source supply system supported in the base unit 105 may be provided separately from the light source supply system 113 supported by the support unit 103. Note that, also in that case, light guided by the light source supply system supported in the base unit 105 is then incident on the light source supply system 113 supported by the support unit 103 and guided to the rigid endoscope 131 via the light source supply system 113.

The rigid endoscope 131 includes a lens barrel 135 and a base unit 133 supporting the lens barrel 135. The lens barrel 135 is supported by the base unit 133 such that the lens barrel extends forward from a front end of the base unit 133. The imaging optical system 137 and the light source supply system 139 are provided in the lens barrel 135 such that the systems extend in the extension direction of the lens barrel 135 (i.e., an axial direction of a cylindrical part). In addition, the rigid endoscope 131 is detachable from the support unit 103 of the camera head 101 via the adaptor 121. Specifically, the rigid endoscope 131 is attached to the support unit 103 via the adaptor 121 such that the end of the base unit 133 on the opposite side to the side on which the lens barrel 135 is supported (i.e., the rear end thereof) is positioned in the front stage of the image sensor 109 supported by the support unit 103.

The imaging optical system 137 corresponds to one or more optical systems for acquiring an image of an affected area to be observed, and is provided to penetrate the lens barrel 135 and the base unit 133 in the direction (i.e., from the front side to the rear side) in which the lens barrel 135 extends. Note that, although the imaging optical system 137 is illustrated in a tubular shape in the example illustrated in FIG. 9, this is merely a schematic illustration of the imaging optical system 137, and does not necessarily limit a configuration of the imaging optical system 137. As a specific example, the imaging optical system 137 may be configured by providing an opening penetrating the lens barrel 135 and the base unit 133 in the direction in which the lens barrel 135 extends and one or more optical systems (e.g., an objective lens, and the like) in the opening. In such a configuration, light incident from a distal end of the lens barrel 135 into the imaging optical system 137 is emitted from the rear end of the base unit 133 via the imaging optical system 137. That is, by attaching the rigid endoscope 131 to the camera head 101, light guided through the imaging optical system 137 is incident into the camera head 101 via the adaptor 121 and forms an image on the image sensor 109 via the imaging optical system 111.

The light source supply system 139 corresponds to a so-called light guide and guides light from the light source apparatus to an affected area. The light source supply system 139 can be constituted by various optical systems, for example, optical fiber or fibers, one or more lenses, and the like. The light source supply system 139 is provided to penetrate the lens barrel 135 and the base unit 133 in the extension direction of the lens barrel 135 (e.g., from the front side to the rear side). That is, when the rigid endoscope 131 is attached to the camera head 101, light from the light source apparatus guided via the light source supply system 113 of the camera head 101 side is incident on the light source supply system 139 via the adaptor 121, then guided via the light source supply system 139, and then emitted from a distal end of the lens barrel 135 toward the affected area.

Next, movements of each unit of the medical imaging apparatus 100 in a case in which the support unit 103 is rotated with respect to the base unit 105 about the axis C10 will be described with reference to FIG. 10. In that case, the image sensor 109 and the light source supply system 113 supported by the support unit 103 rotate about the axis C10 in an integrated manner along rotation of the support unit 103 as illustrated in FIG. 10. In particular, the light source supply system is configured to pass through the axis at the point of rotation. This allows a mounted endoscope to be rotated without the light source supply system being affected. In addition, in a case in which the rigid endoscope 131 is attached to the support unit 103 via the adaptor 121, the rigid endoscope 131 rotates integrally with the support unit 103 about the axis C10 along the rotation of the support unit 103 as illustrated in FIG. 10. That is, even in a case in which the support unit 103 is rotated with respect to the base unit 105, it is possible to maintain a state in which the imaging optical system 137 is positioned in the front stage of the image sensor 109 and the light source supply system 139 is positioned in the latter stage of the light source supply system 113.

Note that, even in the case in which the support unit 103 is rotated with respect to the base unit 105, supply of power to the image sensor 109, transmission of an image signal from the image sensor 109 to a predetermined output destination, and a state in which light from the light source can be guided to the affected area via the lens barrel 135 are maintained. Details of a configuration for realizing the conditions will be separately described below.

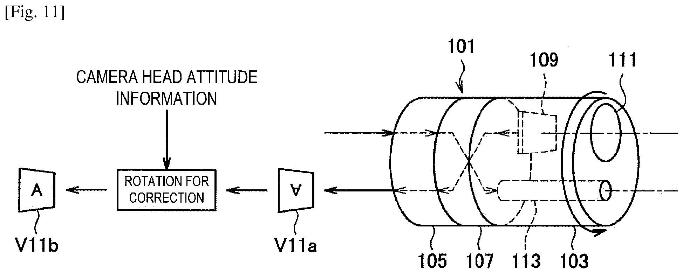

In addition, the medical imaging apparatus 100 may have a mechanism for maintaining hand-eye coordination of an operator. For example, FIG. 11 is an explanatory diagram for describing an example of a schematic configuration of the medical imaging apparatus illustrating an example of a schematic mechanism for maintaining hand-eye coordination of an operator.

When the support unit 103 is rotated with respect to the base unit 105 about the axis C10 as illustrated in FIG. 10, the image sensor 109 supported by the support unit 103 integrally rotates as well, and an attitude of the image sensor 109 changes. As a specific example, in a case in which the support unit 103 is rotated 180 degrees, the top and the bottom of the image sensor 109 are inverted before and after the rotation. Considering this situation, in the medical imaging apparatus 100, an attitude of the camera head 101, particularly, an attitude of the image sensor 109 in accordance with the rotation of the support unit 103, is sensed, and an image V11 in accordance with an imaging result of the image sensor 109 is rotated for correction in accordance with the sensing result. In the example illustrated FIG. 11, for example, an image V11a of which the top and the bottom have been inverted along the rotation of the support unit 103 is rotated for correction in accordance with the rotation amount of the support unit 103, and then an image V11b corrected for the inverted top and bottom is output.

Note that the attitude of the image sensor 109 in accordance with the rotation of the support unit 103 can be sensed by using an encoder and one or more various sensors, for example, an acceleration sensor, a gyro sensor, and the like. In other words, the orientation of the image sensor 109 is sensed by an attitude detection unit as will be explained. In addition, the various sensors for sensing the attitude or a unit for detecting the attitude using sensing results of the various sensors (e.g., a processor, etc.) corresponds to an example of the "attitude detection unit." In addition, a subject that performs the correction is not particularly limited. As a specific example, a control unit (e.g., a CPU, etc.) included in the camera head may have the function of performing the correction. In addition, as another example, an image processing unit of an external apparatus such as the CCU may perform the correction. In the case in which an external apparatus such as the CCU performs the correction, the sensing result of the attitude of the image sensor 109 in accordance with the rotation is transmitted from the camera head side to the external apparatus, and then the external apparatus rotates the image V11 for correction. In addition, as another example, in a case in which an IP converter that performs IP transmission of surgical images has an image processing function, the IP converter may perform rotation for correction. Note that the subject that performs the correction corresponds to an example of a "correction unit."

The example of the schematic configuration of the medical imaging apparatus according to the the present disclosure has been described above with reference to FIGS. 9 to 11.

### <5.2. First practical example>

Next, a first practical example of the medical imaging apparatus according to the the present disclosure will be described. In the first practical example, a case in which the rigid endoscope serving as a medical optical apparatus is attached to the camera head will be focused on as an example of a detailed structure of the medical imaging apparatus and more detailed structures of the camera head and the rigid endoscope will be described.

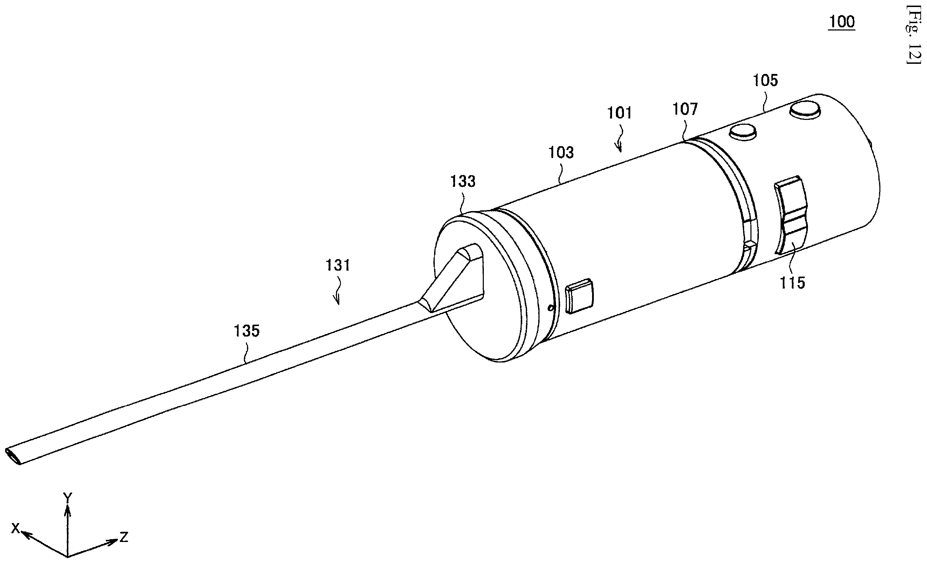

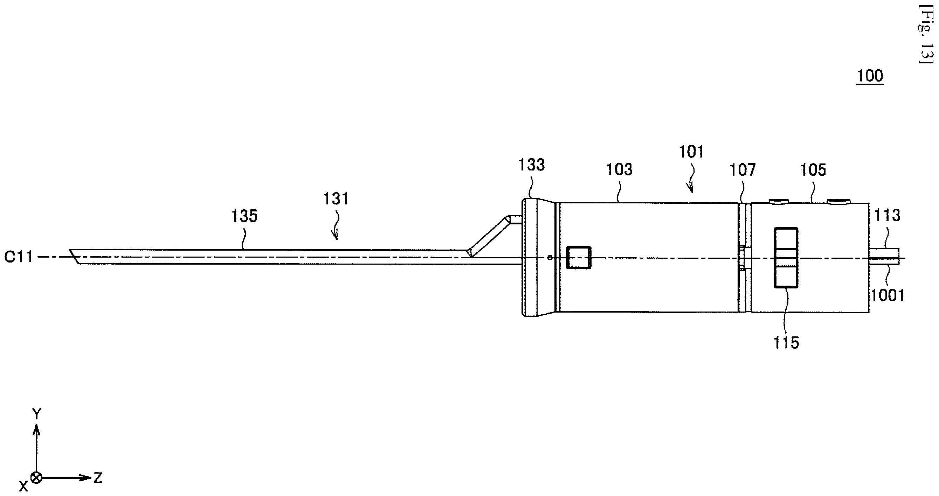

For example, FIGS. 12 and 13 are explanatory diagrams for describing an example of a structure of the medical imaging apparatus according to the first practical example, illustrating an appearance of the medical imaging apparatus in the case in which the rigid endoscope serving as a medical optical apparatus is attached to the camera head. Specifically, FIG. 12 illustrates a perspective view of the medical imaging apparatus according to the first practical example. In addition, FIG. 13 illustrates a side view of the medical imaging apparatus according to the first practical example.

Note that, for the sake of convenience, the front-back direction of the medical imaging apparatus (i.e., the imaging direction of the image sensor provided in the camera head) will be referred to as a z direction and directions orthogonal to the z direction will be referred to as an x direction and a y direction below unless specified otherwise. In other words, in a case in which the rigid endoscope having a lens barrel formed to extend forward on straight line is attached to the camera head, the extension direction of the lens barrel of the rigid endoscope substantially coincides with the z direction.

In FIGS. 12 and 13, constituent elements given with similar reference numerals to those of FIGS. 9 and 10 are similar ones to those in the example of FIGS. 9 and 10. Note that, although the adaptor 121 is not explicitly illustrated in the example illustrated in FIGS. 12 and 13, a medical optical apparatus such as the rigid endoscope 131 may be detachable from the camera head 101 via the adaptor 121, as in the example illustrated in FIGS. 9 and 10. In addition, an axis C11 illustrated in FIG. 13 corresponds to the axis C10 illustrated in FIGS. 9 and 10.

Each of the support unit 103 and the base unit 105 has a housing formed in a substantially cylindrical shape. The cylindrical shape allows the operator to grip and rotate the housing more easily. This reduces the likelihood of erroneous movement. In addition, an end of the support unit 103 on one side in an axial direction (the z direction) of the substantially cylindrical housing (a rear end) is rotatably supported by an end of the base unit 105 on one side in an axial direction (the z direction) of the substantially cylindrical housing (a front end) about the axis (i.e., the central axis C11 illustrated in FIG. 13). In addition, a drive unit 107 is interposed at the portion of the base unit 105 supporting the support unit 103, and the drive unit 107 is driven to cause the support unit 103 to rotate with respect to the base unit 105 about the axis C11. In addition, an operation unit 115 for operating drive of the drive unit 107 is provided on a side surface of the base unit 105. That is, the drive unit 107 is driven when an operator operates the operation unit 115, and the support unit 103 rotates about the axis C11 along with the drive of the drive unit 107.

A rear end of the rigid endoscope 131 of a base unit 133 is attached to a front end of the support unit 103 (i.e., the end on the opposite side to the base unit 105). The lens barrel 135 of the rigid endoscope 131 is formed in a substantially cylindrical shape and is supported on the front end of the base unit 133 (i.e., the end on the opposite side to the rear end) such that the axis of the cylinder extends forward.

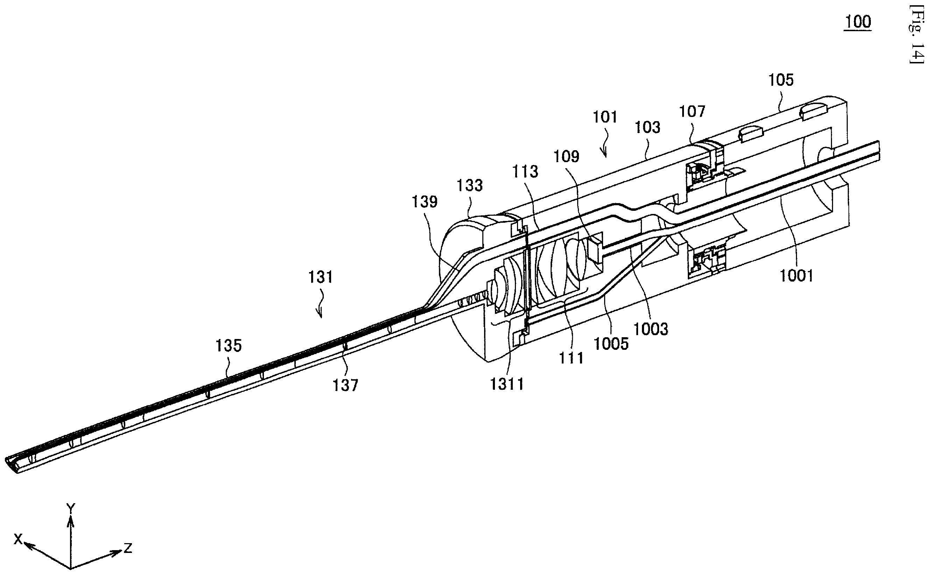

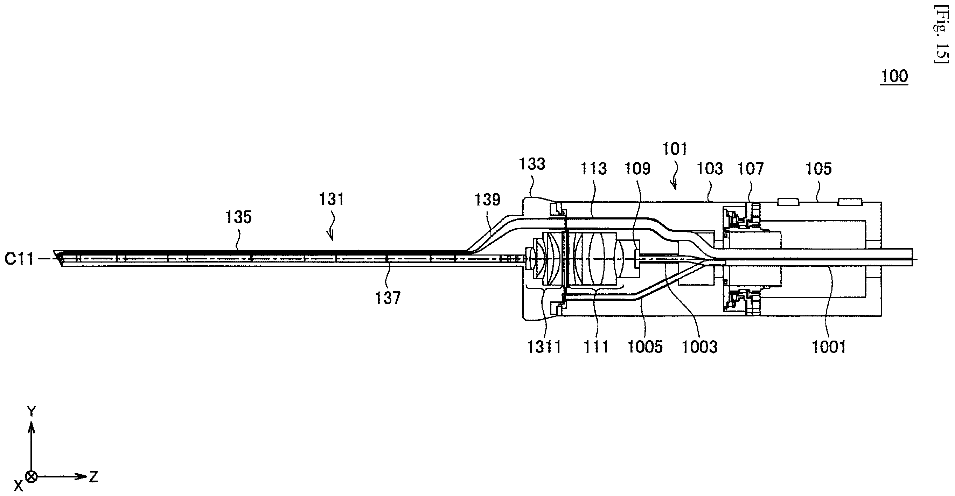

Here, an overview of an internal structure of the medical imaging apparatus 100 according to the first practical example will be described with reference to FIGS. 14 and 15. FIGS. 14 and 15 are explanatory diagram for describing an example of the internal structure of the medical imaging apparatus 100 according to the first practical example, illustrating a cross-section of the medical imaging apparatus 100 illustrated in FIGS. 12 and 13 obtained by cutting the medical imaging apparatus 100 at a y-z plane including the axis C11 illustrated in FIG. 13.

The imaging optical system 137 and the light source supply system 139 are provided in the lens barrel 135 of the rigid endoscope 131 to extend in the extension direction of the lens barrel 135 as described above. Specifically, each of the imaging optical system 137 and the light source supply system 139 is provided to penetrate the lens barrel 135 and the base unit 133 in the extension direction of the lens barrel 135 (i.e., the front-back direction). In addition, an optical system 1311 for focusing on a subject (e.g., an optical system for controlling auto focus (AF)) may be provided as a part of the imaging optical system 137. In the example illustrated in FIG. 15, for example, the optical system 1311 is provided inside the base unit 133.

The light source supply system 113 is supported in the camera head 101 such that the light source supply system extends from the front side to the rear side through a hollow cavity. The provision of the hollow cavity allows the light source supply system 113 to rotate freely without becoming tangled. Specifically, an end of the light source supply system 113 is exposed on the front end of the support unit 103. In addition, the light source supply system 113 extends from the front side to the rear side of the support unit 103 (i.e., in the extension direction of the axis C11) and further extends from the rear end of the support unit 103 to outside of the support unit 103. A portion of the light source supply system 113 extending to outside of the support unit 103 extends to penetrate the base unit 105 from the front side to the rear side (i.e., along the axis C11), and a part thereof is exposed from the rear end of the base unit 105 to outside of the base unit 105 (i.e., outside the camera head 101). Note that the portion of the light source supply system 113 exposed from the rear end of the camera head 101 is connected to, for example, the light source apparatus or another light source supply system connected to the light source apparatus.

In addition, in a case in which the rigid endoscope 131 is attached to the support unit 103, a portion of the light source supply system 113 exposed on the front end of the support unit 103 faces the end of the light source supply system 139 exposed to the rear end side of the base unit 133 of the rigid endoscope 131. That is, the light source supply system 139 is positioned in the latter stage of the light source supply system 113. Accordingly, light emitted from the light source apparatus and then guided via the light source supply system 113 is incident into the light source supply system 139 from the rear end side of the base unit 133, then guided through the light source supply system 139, and then emitted from the distal end of the lens barrel 135 to the affected area.

The support unit 103 of the camera head 101 supports the image sensor 109 therein, and the front side of the image sensor 109 is opened. Note that, in the present practical example, the image sensor 109 is supported such that the center of the imaging plane of the image sensor 109 substantially coincides with the axis C11 on an x-y plane. In addition, the support unit 103 may support the imaging optical system 111 in the opening such that the imaging optical system 111 is positioned in the front stage (front side) of the image sensor 109. In the case in which the rigid endoscope 131 is attached to the support unit 103, the opening faces the end of the imaging optical system 137 exposed to the rear end side of the base unit 133 of the rigid endoscope 131. That is, the imaging optical system 137 is positioned in the front stage of the image sensor 109. Accordingly, light guided by the imaging optical system 137 is incident from the opening into the camera head 101 and forms an image on the image sensor 109 via the imaging optical system 111.

A signal line for supplying power from an external apparatus (power supply apparatus, etc.) is connected to the image sensor 109. Specifically, a signal line 1001 is supported in the base unit 105 to extend from the rear side to the front side of the base unit 105, and a portion passing the rear end of the support unit 103 and extending inside the support unit 103 (which will also be referred to as a "signal line 1003" below) is electrically connected to the image sensor 109.

In addition, the signal line 1001 partly branches separately from the signal line 1003, and power can be supplied to the rigid endoscope 131 attached to the front of the support unit 103 via the brand part (which will also be referred to as a "signal line 1005" below). In addition, the signal line 1001 may be used a s a signal line for transmitting an image signal in accordance with an imaging result of the image sensor 109 to outside of the camera head 101 (e.g., the CCU, etc.). In addition, the signal line 1001 may be used as a signal line for transmitting a control signal from an external apparatus (e.g., the CCU, etc.) for operating the camera head 101 to the camera head 101.

The overview of a configuration (structure) of the medical imaging apparatus 100 according to the first practical example has been described above with reference to FIGS. 12 to 15.

### (Camera head)

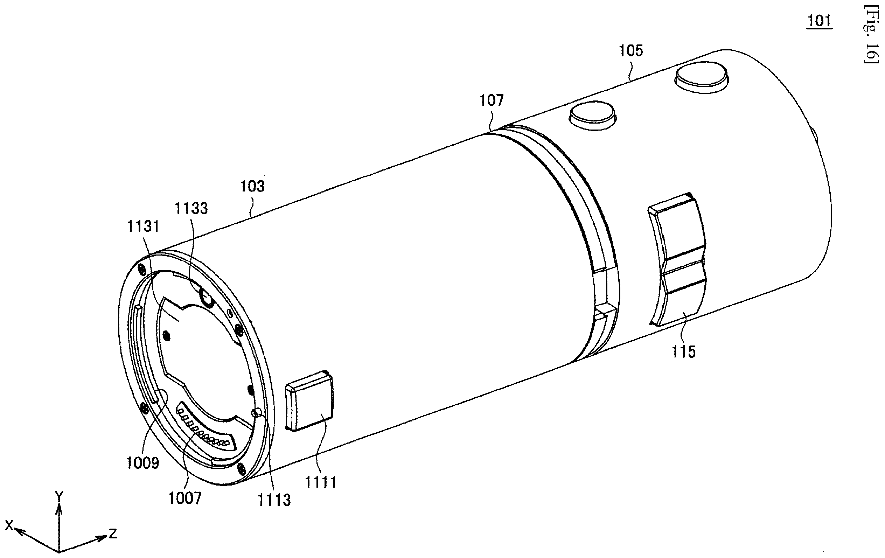

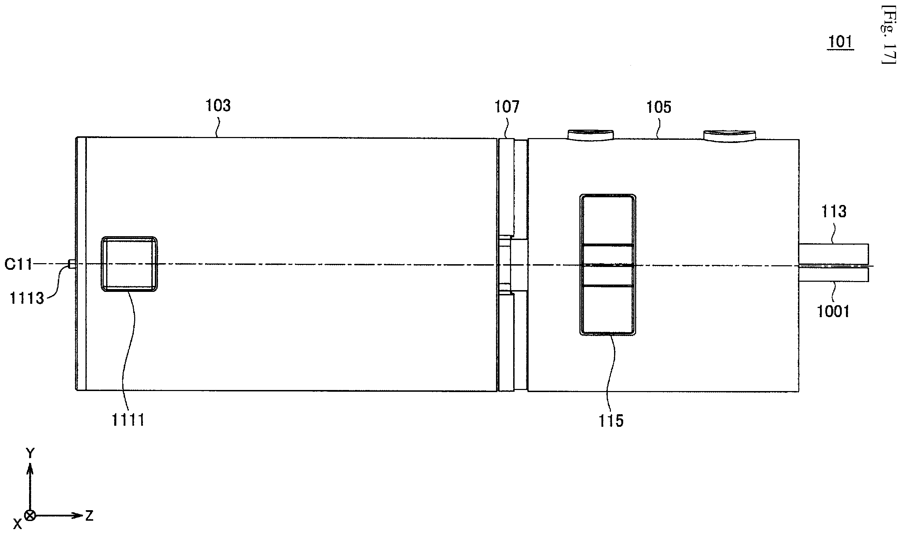

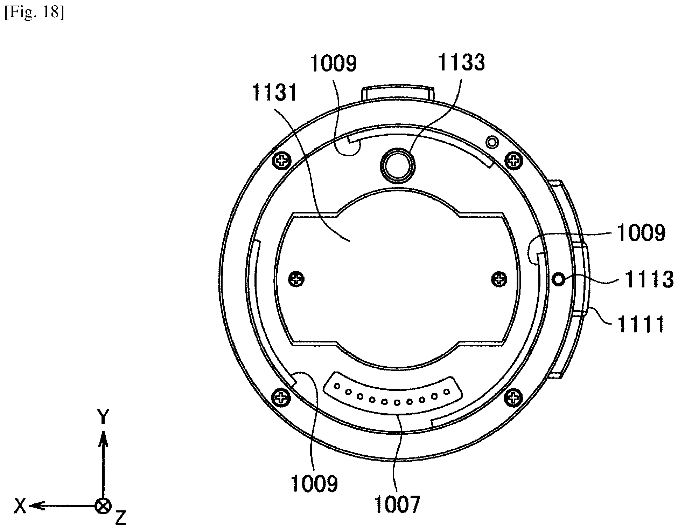

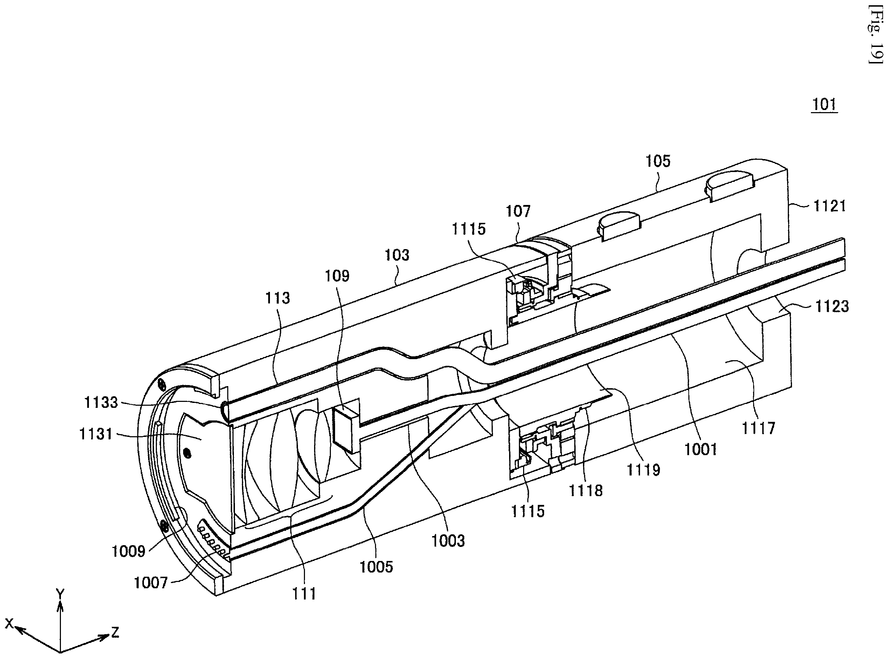

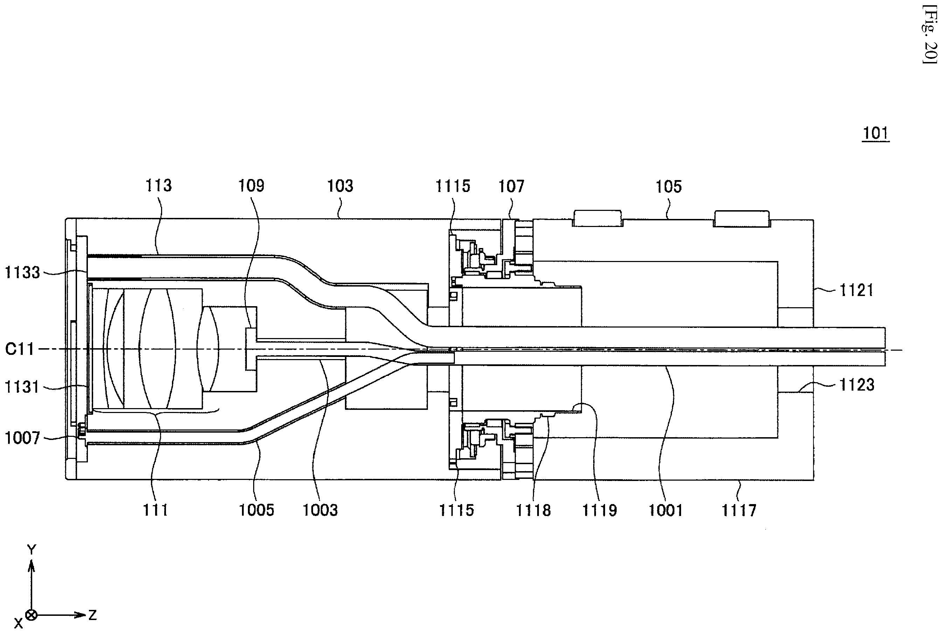

Next, a configuration (structure) of the camera head 101 according to the first practical example will be described in more detail with reference to FIGS. 16 to 20. FIGS. 16 to 18 are explanatory diagrams for describing an example of the structure of the camera head of the medical imaging apparatus according to the first practical example. Specifically, FIG. 16 illustrates a perspective view of the camera head of the medical imaging apparatus according to the first practical example. In addition, FIG. 17 illustrates a side view of the camera head of the medical imaging apparatus according to the first practical example. In addition, FIG. 18 illustrates a rear view of the camera head of the medical imaging apparatus according to the first practical example. In addition, FIGS. 19 and 20 are explanatory diagrams for describing an example of an internal structure of the medical imaging apparatus according to the first practical example, illustrating a cross-section obtained by cutting the camera head 101 illustrated in FIGS. 16 to 18 at the y-z plane including the axis C11 illustrated in FIG. 17. Note that the axis C11 illustrated in FIGS. 17 and 20 corresponds to the axis C11 illustrated in FIG. 15. In addition, since constituent elements given with similar reference numerals to those of FIGS. 12 to 15 in FIGS. 16 to 20 represent similar constituent elements to those in the example illustrated in FIGS. 12 to 15, detailed description thereof will be omitted.

A detachable unit 1009 is provided on the front end of the support unit 103 as illustrated in FIGS. 16 to 18. The detachable unit 1009 is for allowing the base unit 133 of the rigid endoscope 131 to be detached from the end. As a specific example, in the example illustrated in FIGS. 16 to 18, the detachable unit 1009 has a so-called threeclaw bayonet configuration. That is, in this case, the detachable unit 1009 can be constituted by engagement claws for bayonet-coupling the rear end of the base unit 133 of the rigid endoscope 131 with the front end of the support unit 103, grooves into which engagement claws fit, and the like. Note that a configuration of the detachable unit 1009 is not particularly limited as long as the base unit 133 of the rigid endoscope 131 is detachable from the support unit 103.

A lock mechanism 1113 for locking the rigid endoscope 131 attached to the front end of the support unit 103 so as not to come off from the support unit 103 is provided on the end. In other words, the lock mechanism is provided on the adapter and locks the endoscope (or as noted later, the microscope) in position. For example, a lock pin is provided as the lock mechanism 1113 in the example illustrated in FIGS. 16 to 18. The lock pin controls rotation of the rigid endoscope 131 with respect to the support unit 103 about the axis C11 by fitting into a recess provided on the rear end of the base unit 133 of the rigid endoscope 131. That is, since an operation for removing the rigid endoscope 131 from the support unit 103 is hindered, the mechanism can prevent the rigid endoscope 131 of the support unit 103 from coming off therefrom. Note that a configuration of the lock mechanism 1113 may be appropriately changed in accordance with a configuration of the detachable unit 1009 (i.e., a detachment method of the rigid endoscope 131 from the support unit 103).

An operation unit 1111 for releasing the locking state by the lock mechanism 1113 is provided on a side surface of the support unit 103. As a specific example, when an operator operates the operation unit 1111, the locking pin (the lock mechanism 1113) projecting forward from the front end of the support unit 103 slides backward and is housed, and thereby the fitting state of the locking pin into the recess is released. Accordingly, the rigid endoscope 131 can be rotated with respect to the support unit 103 about the axis C11, the rigid endoscope 131 attached to the support unit 103 can be removed from the support unit 103.

Reference numeral 1131 represents an incidence plane of the imaging optical system 111. That is, the opening in which the image sensor 109 and the imaging optical system 111 are housed is provided such that the incidence plane 1131 faces the end of the imaging optical system 137 exposed on the rear end of the base unit 133 of the rigid endoscope 131 in the case in which the rigid endoscope 131 is attached to the support unit 103. Accordingly, light guided through the imaging optical system 137 of the rigid endoscope 131 is incident into the imaging optical system 111 via the incidence plane 1131 (i.e., inside the support unit 103) and forms an image on the image sensor 109 supported in the support unit 103 via the imaging optical system 111.

Reference numeral 1133 represents an emission plane of the light source supply system 113. That is, the light source supply system 113 is arranged such that the emission plane 1133 faces the end of the light source supply system 139 exposed on the rear end side of the base unit 133 of the rigid endoscope 131 in the case in which the rigid endoscope 131 is attached to the support unit 103. Accordingly, light emitted from the light source apparatus and guided through the light source supply system 113 is emitted from the emission plane 1133 to outside of the support unit 103, then is incident into the light source supply system 139 from the rear end of the base unit 133, then is guided through the light source supply system 139, and then is emitted from the distal end of the lens barrel 135 toward the affected area.

Reference numeral 1007 is a terminal for supplying part of power supplied to the camera head 101 to the rigid endoscope 131 attached to the support unit 103 of the camera head 101. The terminal 1007 is electrically connected to another terminal provided on the rear end (which will also be referred to as a "terminal 1121" below) of the base unit 133 of the rigid endoscope 131 in the case in which the rigid endoscope 131 is attached to the support unit 103. Accordingly, part of power supplied to the camera head 101 can be supplied to the rigid endoscope 131 via the terminal 1007.

In addition, the terminal 1007 may include a terminal (interface) for exchanging information with the medical optical apparatus (e.g., the rigid endoscope 131) attached to the camera head 101. With this configuration, for example, when information stored in the medical optical apparatus (e.g., information regarding the medical optical apparatus) attached to the camera head 101 is reported from the medical optical apparatus to the camera head 101, the camera head 101 can also recognize the medical optical apparatus attached thereto and information regarding the medical optical apparatus. For example, the information may include the optical parameters of the medical optical apparatus. This allows for automatic recognition and application of optical information to the camera head 101.

In addition, with the above configuration, for example, by feeding a recognition result of information regarding the medical optical apparatus back to the supporting arm apparatus from the camera head 101, the supporting arm apparatus can change control of the arm unit in accordance with the medical optical apparatus attached to the camera head 101. Particularly, with respect to medical optical apparatuses attachable to the camera head 101, a weight, a position of the center of gravity at the time of attachment, and the like can vary depending on each of the medical optical apparatuses of a wide range such as rigid endoscopes and microscopes. Even in such a situation, the supporting arm apparatus can control drive of joints of the arm such as controlling torque of the joints necessary for gravity compensation in accordance with the medical optical apparatus attached to the camera head 101, using a recognition result of the medical optical apparatus. This allows for automatic application of information relating to the medical optical apparatus.

Note that information reported from the medical optical apparatus to the camera head 101 is not particularly limited. For example, information regarding specifications (various parameters) of the medical optical apparatus may be reported from the medical optical apparatus to the camera head 101. In addition, as another example, identification information for identifying the medical optical apparatus may be reported from the medical optical apparatus to the camera head 101. In this case, for example, data (table) in which identification information of each medical optical apparatus is associated with various types of information regarding the medical optical apparatus may be stored in a predetermined storage area. Accordingly, various types of information regarding a medical optical apparatus can be read from the data on the basis of the identification information acquired from the medical optical apparatus.

Note that a storage area in which the data is held is not particularly limited as long as the data can be readable by an apparatus that uses the information regarding the medical optical apparatus (e.g., the camera head 101 or the supporting arm apparatus). As a specific example, the data may be held in a storage area of the camera head 101 or the supporting arm apparatus. In addition, as another example, the data may be held in an external apparatus such as a server connected to an apparatus that uses the information regarding the medical optical apparatus via a network.

Next an example of a structure for allowing the support unit 103 to rotate with respect to the base unit 105 about the axis C11 will be described in more detail.

The base unit 105 is formed in a hollow shape of which the housing has an opening 1117 along the axis C11 as illustrated in FIGS. 19 and 20. In addition, a substantially cylindrical protrusion 1118 having an opening 1119 along the axis C11 is formed on the rear end side of the support unit 103 such that the central axis thereof substantially coincides with the axis C11. Note that each of the openings 1117 and 1119 is formed to be substantially cylindrical having a central axis substantially coinciding with the axis C11. In other words, a cylindrical hollow portion of which the central axis substantially coincides with the axis C11 is formed in the housing of the base unit 105. In addition, the protrusion 1118 is formed such that a width thereof in the radial direction with respect to the axis C11 shorter than a width of the opening 1117 in the radial direction. With this configuration, the protrusion 1118 in which the opening 1119 is formed on the support unit 103 side fits into the opening 1117 on the base unit 105 side, and thus the support unit 103 is supported to be rotatable with respect to the base unit 105 about the axis C11. In addition, at this time, the opening 1119 of the protrusion 1118 is connected to the opening 1117 of the base unit 105 along the axis C11.

In addition, the drive unit 107 is provided on the base unit 105 side, and the drive unit 107 applies torque to the protrusion 1118 of the support unit 103 in the circumferential direction around the axis C11. Accordingly, the support unit 103 rotates with respect to the base unit 105 about the axis C11. As a configuration for realizing this operation, for example, the drive unit 107 includes a ring-shaped ultrasonic motor 1115 in the example illustrated in FIGS. 19 and 20. Of course, a configuration of the drive unit 107 is not necessarily limited as long as the operation can be realized. Indeed, the drive unit 107 may have no motor and may be rotated by a user.

With the above-described configuration, each of the light source supply system 113 and the signal line 1001 is supported such that it is positioned within the openings 1117 and 1119 partially connected to each other. In addition, an opening 1123 is provided on an end 1121 of the base unit 105, a portion of each of the light source supply system 113 and the signal line 1001 extending rearward is exposed from the opening 1123 to outside of the base unit 105, and further extends to the rear side of the camera head 101. For example, the opening 1123 is formed to be substantially cylindrical having a central axis substantially coinciding with the axis C11 in the example illustrated in FIGS. 19 and 20. That is, each of the light source supply system 113 and the signal line 1001 is supported such that it penetrates the hollow portion of the base unit 105 (i.e., the opening 1119) along the axis C11.

Note that the opening 1123 may be formed to have a width to the extent that the light source supply system 113 and the signal line 1001 may not be fixed to the base unit 105 on the end 1121 of the base unit 105. With this configuration, even if the support unit 103 rotates with respect to the base unit 105, it is possible to prevent occurrence of a tangle in the bundle of the light source supply system 113 and the signal line 1001 in the limited space for the hollow portion of the base unit 105 and further a situation in which the light source supply system 113 or the signal line 1001 is disconnected. As noted above, the signal line 1001 may also not be provided. For example, the signal line 1001 may be replaced with a wireless connection.

The configuration (structure) of the camera head 101 according to the first practical example has been described above in more detail with reference to FIGS. 16 to 20.

### (Rigid endoscope)

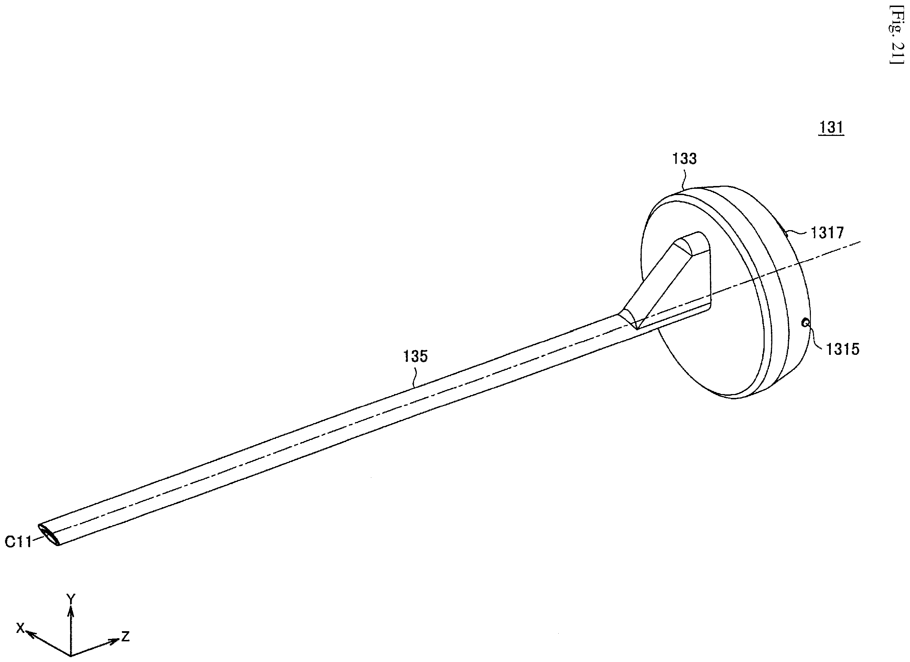

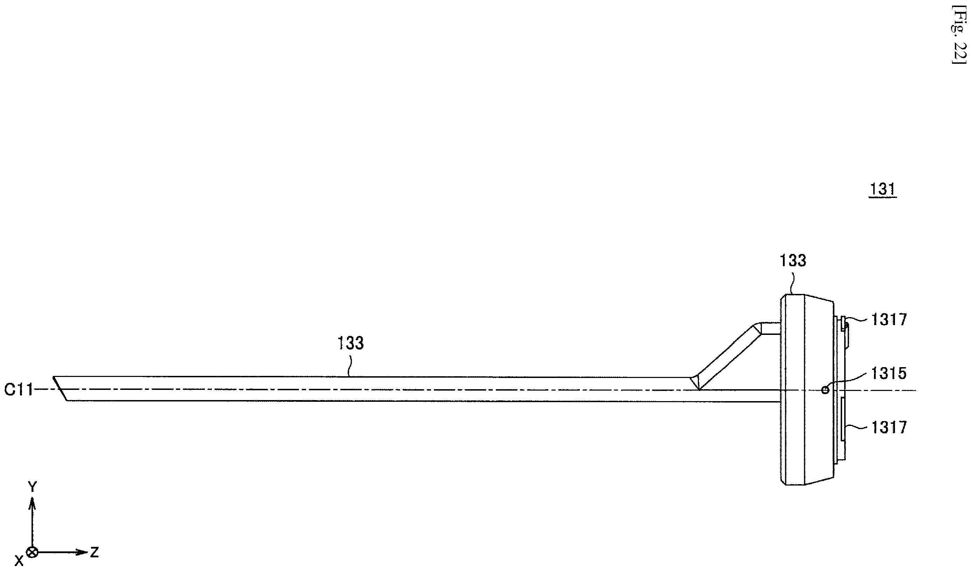

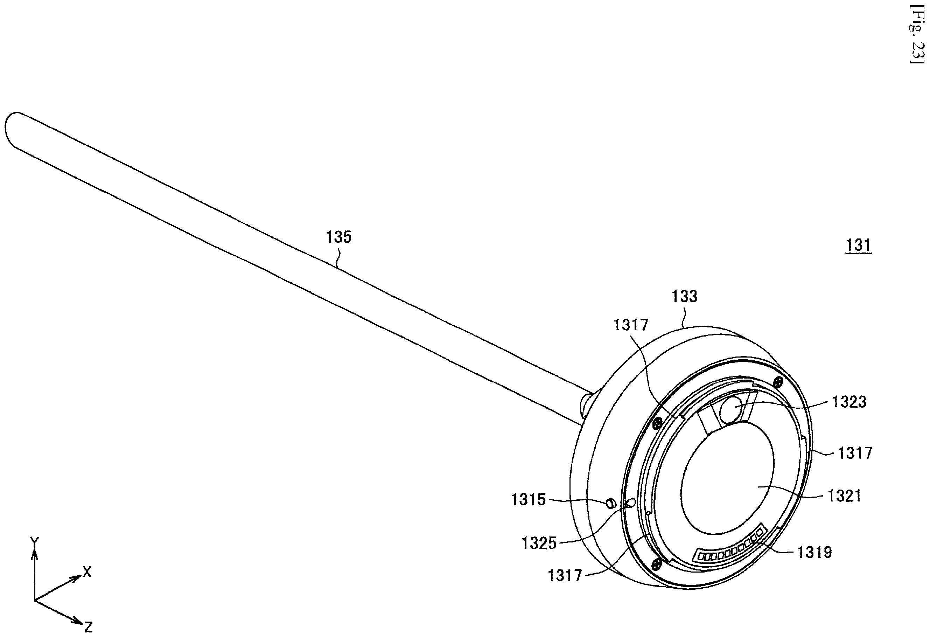

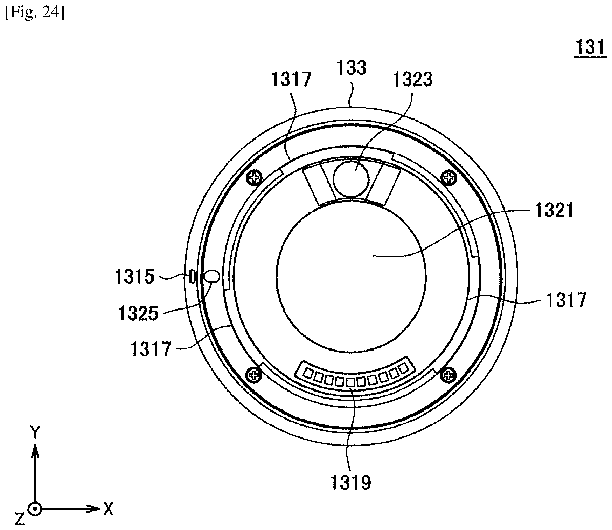

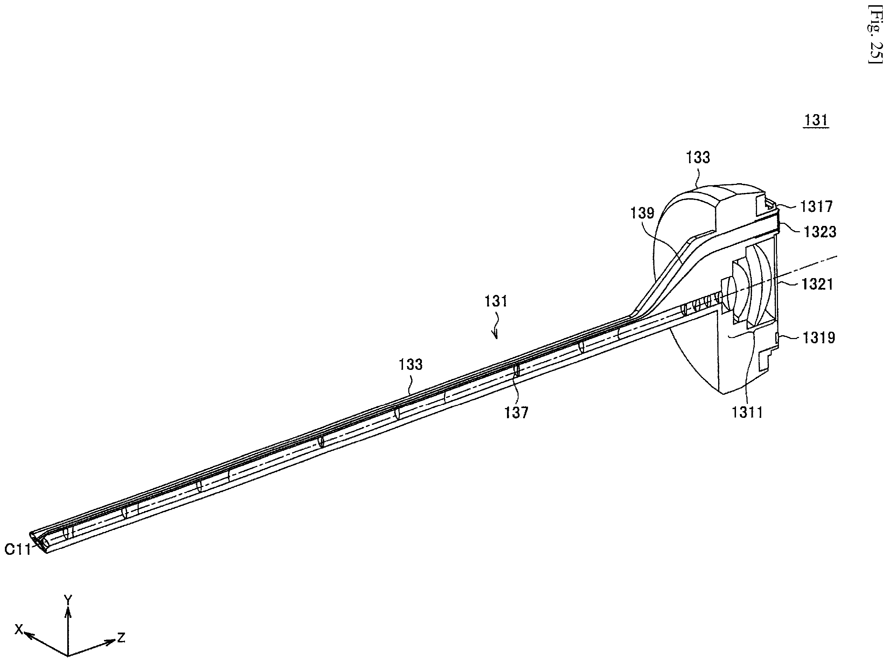

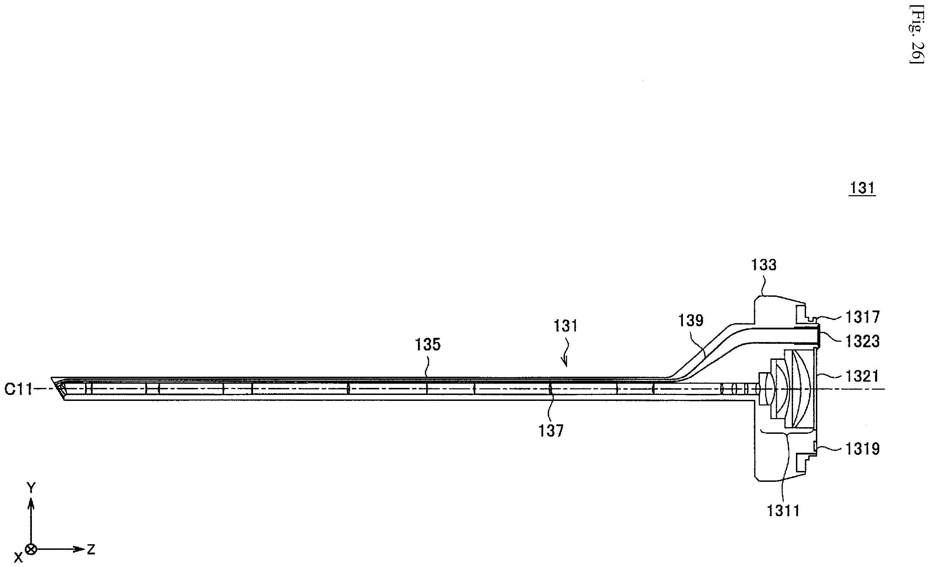

Next, as an example of the medical optical apparatus to be attached to the camera head (e.g., the camera head 101 according to the above-described first practical example) according to the present disclosure, a structure of a the rigid endoscope 131 will be described in more detail with reference to FIGS. 21 to 26. FIGS. 21 to 24 are explanatory diagram for describing an example of the structure of the rigid endoscope to be attached to the camera head according to the present disclosure. Specifically, FIG. 21 shows a perspective view of a case in which the rigid endoscope to be attached to the camera head is obliquely viewed from a front. In addition, FIG. 22 shows a side view of the rigid endoscope to be attached to the camera head. In addition, FIG. 23 shows a perspective view of a case in which the rigid endoscope to be attached to the camera head is obliquely viewed from behind. In addition, FIG. 24 shows a rear view of the rigid endoscope to be attached to the camera head. In addition, FIGS. 25 and 26 are explanatory diagram for describing an example of an internal structure of the rigid endoscope to be attached to the camera head, illustrating a cross-section obtained by cutting the rigid endoscope 131 illustrated in FIGS. 21 to 24 at the y-z plane including the axis C11 illustrated in FIG. 22. Note that the axis C11 illustrated in FIGS. 21, 22, 25, and FIG. 26 corresponds to the axis C11 illustrated in FIGS. 13 and 15. In addition, since constituent elements in FIGS. 21 to 26 given with similar reference numerals to those in FIGS. 12 to 15 represent similar constituent elements to those in the example illustrated in FIGS. 12 to 15, detailed description thereof will be omitted.

The rigid endoscope 131 includes the lens barrel 135 and the base unit 133 supporting the lens barrel 135 as described above. The lens barrel 135 is supported by the base unit 133 such that the lens barrel extends forward from the front end of the base unit 133. At this time, the lens barrel 135 is supported by the base unit 133 such that the axis of the lens barrel 135 (i.e., the axis of the cylindrical portion) substantially coincides with the axis C11 as illustrated in FIGS. 21 and 22. With this configuration, in a case in which the support unit 103 rotates about the axis C11 in a state in which the rigid endoscope 131 is attached to the support unit 103 of the camera head 101, the lens barrel 135 integrally rotates with the support unit 103 without shake of the axis of the lens barrel 135.

A detachable unit 1317 is provided on the rear end side of the base unit 133 as a configuration for allowing the base unit 133 to be detachable from the front end of the support unit 103 of the camera head 101 as illustrated in FIGS. 21 to 24. Note that a configuration of the detachable unit 1317 is determined in accordance with a configuration of the detachable unit 1009 on the camera head 101 side. That is, in a case in which the detachable units 1009 and 1317 are configured such that the detachable units 1009 and 1317 are bayonet-coupled with each other, either of the detachable unit 1009 and 1317 can include engagement claws for the bayonet-coupling, and the other one can include grooves into which the engagement claws fit or the like. Of course, similarly to the detachable unit 1009, a configuration of the detachable unit 1317 is not particularly limited as long as from which the base unit 133 of the rigid endoscope 131 can be detached from the support unit 103.

A lock mechanism 1325 for locking the rigid endoscope 131 so as not to come off from the support unit 103 in a case in which the rigid endoscope 131 is attached to the front end of the support unit 103 of the camera head 101 is provided on the rear end of the base unit 133. The lock mechanism 1325 fits to, for example, the lock mechanism 1113 on the camera head 101 side, and thereby hinders rotation of the rigid endoscope 131 with respect to the support unit 103 about the axis C11. As a more specific example, in a case in which the lock mechanism 1113 on the camera head 101 side is a lock pin, the lock mechanism 1325 on the rigid endoscope 131 side can be a recess to which the lock pin fits. Of course, the lock mechanism 1325 side may have a lock pin. In this case, the lock mechanism 1113 side has a recess to which the lock pin fits. In addition, configurations of the lock mechanisms 1113 and 1325 are not particularly limited as long as the rigid endoscope 131 can be locked in order not to come off from the support unit 103.

In addition, a marker 1315 indicating a position of the lock mechanism 1325 may be provided at a position corresponding to a periphery of the lock mechanism 1325 on a side surface of the base unit 133.

One end of the imaging optical system 137 provided in the lens barrel 135 of the rigid endoscope 131 is exposed on the rear end of the base unit 133 of the rigid endoscope 131 as described above. Reference numeral 1321 indicated in FIGS. 23 and 24, for example, represents the end (emission plane) of the imaging optical system 137. That is, the imaging optical system 137 is provided such that the emission plane 1321 faces the incidence plane 1131 on the camera head 101 side in the case in which the rigid endoscope 131 is attached to the support unit 103 of the camera head 101. Accordingly, light guided by the imaging optical system 137 of the rigid endoscope 131 is incident onto the imaging optical system 111 (i.e., inside the support unit 103) via the incidence plane 1131 and forms an image on the image sensor 109 supported in the support unit 103 via the imaging optical system 111.

In addition, one end of the light source supply system 139 provided in the lens barrel 135 of the rigid endoscope 131 is exposed on the rear end side of the base unit 133 of the rigid endoscope 131. For example, reference numeral 1323 illustrated in FIGS. 23 and 24 represents the end (incidence plane) of the light source supply system 139. That is, the light source supply system 139 is provided such that the incidence plane 1323 faces the emission plane 1133 on the camera head 101 side in the case in which the rigid endoscope 131 is attached to the support unit 103 of the camera head 101. Accordingly, light guided by the light source supply system 113 is emitted to outside of the support unit 103 from the emission plane 1133, then incident onto the light source supply system 139 from the rear end side of the base unit 133, then guided through the light source supply system 139, and then emitted from the distal end of the lens barrel 135 to the affected area.

Reference numeral 1319 is a terminal for the rigid endoscope 131 to receive supply of power from the camera head 101 side. The terminal 1319 is arranged at a position at which the terminal 1319 can be electrically connected to the terminal 1007 on the camera head 101 side in the case in which the rigid endoscope 131 is attached to the support unit 103 of the camera head 101.

In addition, the optical system 1311 for focusing on a subject may be provided as part of the imaging optical system 137 as described above. For example, the optical system 1311 is provided in the base unit 133 in the example illustrated in FIGS. 25 and 26. In addition, at least a partial optical system of the optical system 1311 may shift its position along the optical axis through so-called AF control. In this case, the AF control may be realized by, for example, by driving a drive unit such as a motor with power supplied from camera head 101 side via the terminal 1319.

The structure of the rigid endoscope 131 has been described in more detail as an example of the medical optical apparatus to be attached to the camera head according to the present disclosure with reference to FIGS. 21 to 26.

In the camera head 101 of the medical imaging apparatus 100 according to the first practical example, the image sensor 109 and the light source supply system 113 are supported by the support unit 103, and the support unit 103 rotates with respect to the base unit 105 about the axis C11 as described above. Accordingly, the image sensor 109 and the light source supply system 113 are integrally rotated. In addition, in the case in which the rigid endoscope 131 is attached to the front side of the support unit 103, the rigid endoscope 131 rotates integrally with the support unit 103. Accordingly, even in the case in which the support unit 103 rotates with respect to the base unit 105, the state in which the imaging optical system 137 of the rigid endoscope 131 side is positioned in the front stage of the image sensor 109 and the light source supply system 139 of the rigid endoscope 131 side is positioned in the latter stage of the light source supply system 113 is maintained. In addition, with the above-described configuration, a light guide that guides light from a light source can be provided such that it extends from the rear side of the camera head 101 toward the light source. Accordingly, for example, it is not necessary to separately provide a connection part for connecting a light source supply system to the medical optical apparatus, unlike the medical imaging apparatus 800 according to Comparative Example 1 described with reference to FIG. 6. Therefore, the medical imaging apparatus 100 is easier to be handled by an operator and has further improved operability than the medical imaging apparatus 800 according to Comparative Example 1.

### <5.3. Second practical example>

Next, a second practical example of a medical imaging apparatus according the present disclosure will be described. A medical optical apparatus to be attached to the camera head is not limited only to a rigid endoscope, various medical optical apparatuses can be selectively attached to the camera head depending on applications. Therefore, in the second practical example, an example of a structure of a medical imaging apparatus (particularly, a structure of a microscope to be attached to the camera head) will be described, focusing on a case in which a microscope is used as another medical optical apparatus, rather than a rigid endoscope.

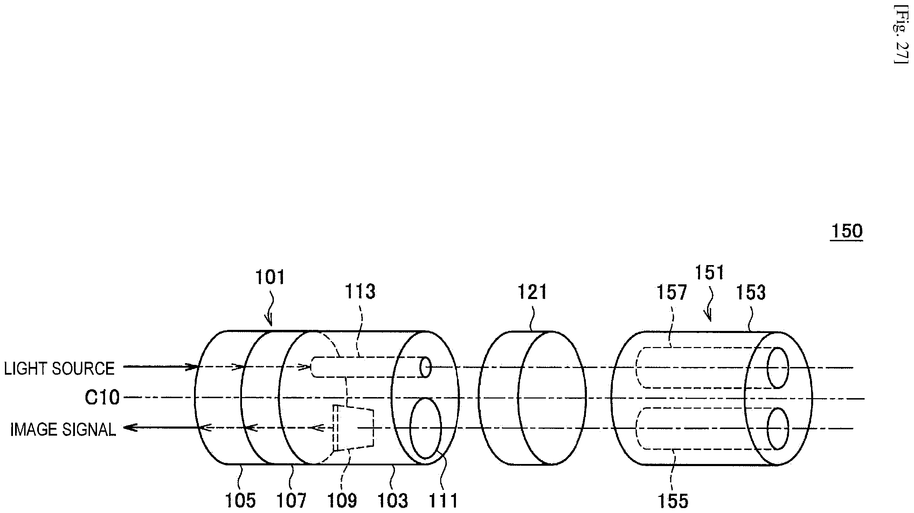

First, an example of a schematic configuration of the medical imaging apparatus in a case in which a microscope is applied as a medical optical apparatus will be described with reference to FIG. 27 as an example of a schematic configuration of the medical imaging apparatus according to the second practical example. FIG. 27 is an explanatory diagram for describing the example of the schematic configuration of the medical imaging apparatus according to the second practical example. Note that, in the following description, the medical imaging apparatus according to the second practical example may be referred to as a "medical imaging apparatus 150" for the sake of convenience in order to distinguish from the medical imaging apparatuses according to the above-described example and other practical example.

The medical imaging apparatus 150 according to the second practical example includes a camera head 101, a microscope 151, and an adaptor 121 as illustrated in FIG. 27. Note that, since configurations of the camera head 101 and the adaptor 121 are similar to those of the medical imaging apparatus 100 described with reference to FIGS. 9 and 10, detailed description thereof will be omitted. In addition, the axis C10 illustrated in FIG. 27 corresponds to the axis C10 illustrated in FIGS. 9 and 10.

The imaging optical system 155 corresponds to one or more optical systems for acquiring an image of an affected area to be observed, and is provided to penetrate the lens barrel 153 in the direction (i.e., from the front side to the rear side) in which the lens barrel 153 extends. Note that, although the imaging optical system 155 is illustrated in a tubular shape in the example illustrated in FIG. 27, this is merely a schematic illustration of the imaging optical system 155, and does not necessarily limit a configuration of the imaging optical system 155. As a specific example, the imaging optical system 155 may be configured by providing an opening penetrating the lens barrel 153 in the direction in which the lens barrel 153 extends and one or more optical systems (e.g., an objective lens, and the like) in the opening. The microscope 151 has a lens barrel 153 extending from the rear side to the front side. An imaging optical system 155 and a light source supply system 157 are provided in the lens barrel 153 such that the systems extend in the extension direction (i.e., the axial direction of the substantially cylindrical part) of the lens barrel 153. Note that the imaging optical system 155 and the light source supply system 157 correspond to the imaging optical system 137 and the light source supply system 139 of the above-described rigid endoscope 131. In addition, the microscope 151 is detachable from a support unit 103 of the camera head 101 via the adaptor 121. Specifically, the microscope 151 is attached to the support unit 103 via the adaptor 121 such that the rear end thereof is positioned in front of the support unit 103 (i.e., in the front stage of an image sensor 109 supported by the support unit 103).

With this configuration, light incident onto the imaging optical system 155 from an end 1517 of the imaging optical system 155 positioned on the front end side of the lens barrel 153 is emitted from the rear end of the lens barrel 153 through the imaging optical system 155. That is, when the microscope 151 is attached to the camera head 101, the light guided by the imaging optical system 155 is incident onto the camera head 101 via the adaptor 121 and forms an image on the image sensor 109 via the imaging optical system 111.

The light source supply system 157 corresponds to a so-called light guide and guides light from the light source apparatus to an affected area. The light source supply system 157 can be constituted by various optical systems, for example, optical fibers, one or more lenses, and the like. The light source supply system 157 is provided to penetrate the lens barrel 153 in the extension direction of the lens barrel 153 (e.g., from the front side to the rear side). That is, when the microscope 151 is attached to the camera head 101, light from the light source apparatus guided through the light source supply system 113 on the camera head 101 side is incident on the light source supply system 157 via the adaptor 121. Then, the light guided through the light source supply system 157 is emitted from an end 1519 of the light source supply system 157 positioned on the front end of the lens barrel 153 toward the affected area.

In addition, in the case in which the support unit 103 is rotated with respect to the base unit 105 about the axis C10, the image sensor 109 and the light source supply system 113 supported by the support unit 103 integrally rotate about the axis C10 along with the rotation of the support unit 103 as described above with reference to FIG. 27. In addition, in a case in which the microscope 151 is attached to the support unit 103 via the adaptor 121, the microscope 151 rotates integrally with the support unit 103 about the axis C10 along the rotation of the support unit 103. That is, even in a case in which the support unit 103 is rotated with respect to the base unit 105, it is possible to maintain a state in which the imaging optical system 155 is positioned in the front stage of the image sensor 109 and the light source supply system 157 is positioned in the latter stage of the light source supply system 113.

The example of the schematic configuration of the medical imaging apparatus in the case in which a microscope is applied as a medical optical apparatus has been described above with reference to FIG. 27 as an example of a schematic configuration of the medical imaging apparatus according to the second practical example.

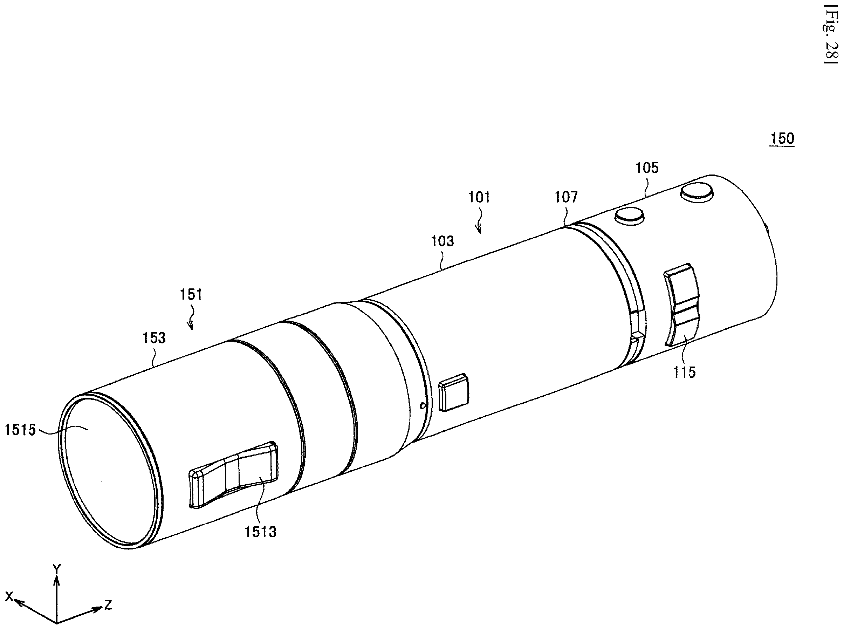

Next, an overview of an example of a structure of the medical imaging apparatus according to the second practical example will be described with reference to FIGS. 28 to 31. Note that detailed description of a structure of the camera head 101 will be omitted since it is similar to that of the medical imaging apparatus 100 according to the first practical example, and a structure of the microscope 151 will be mainly described.

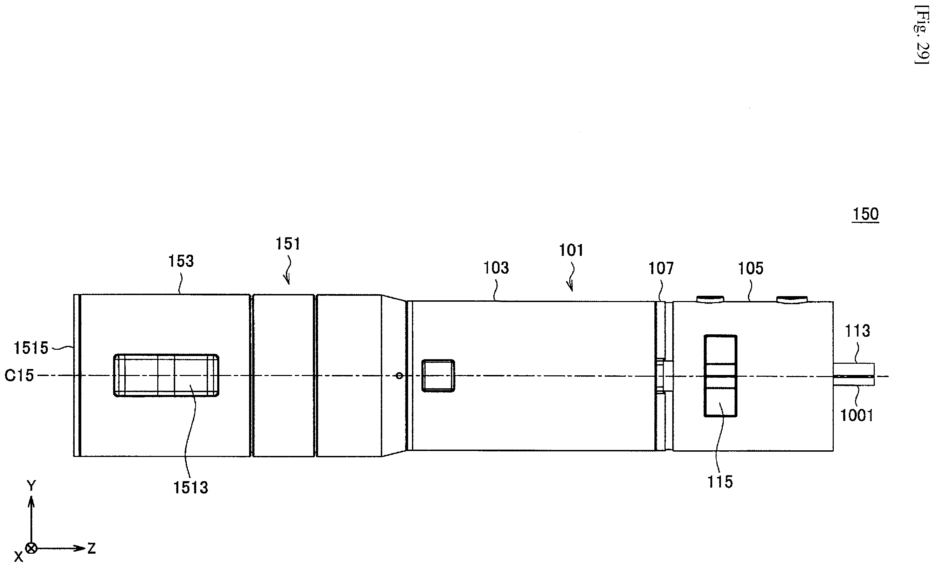

First, an overview of a structure of the medical imaging apparatus according to the second practical example will be described mainly focusing on its appearance with reference to FIGS. 28 and 29. FIGS. 28 and 29 are explanatory diagrams for describing an example of a structure of the medical imaging apparatus according to the second practical example, illustrating an appearance of the medical imaging apparatus in the case in which the microscope serving as a medical optical apparatus is attached to the camera head. Specifically, FIG. 28 illustrates a perspective view of the medical imaging apparatus according to the second practical example. In addition, FIG. 29 illustrates a side view of the medical imaging apparatus according to the second practical example.

In FIGS. 28 and 29, constituent elements given with similar reference numerals to those of FIG. 27 represent similar constituent elements in the example illustrated in FIG. 27. Note that explicit illustration of the adaptor 121 is omitted in the example of FIGS. 28 and 29.

In the microscope 151, the rear end of the lens barrel 153 is attached to the front end of the support unit 103 of the camera head 101 (i.e., the end on the opposite side to the base unit 105) as illustrated in FIGS. 28 and 29. The lens barrel 153 of the microscope 151 is formed in a substantially cylindrical shape, and in a case in which the microscope 151 is attached to the camera head 101, it is supported by the support unit 103 of the camera head 101 such that an axis of the substantially cylindrical portion thereof extends forward. Note that the axis C15 illustrated in FIG. 29 corresponds to the axis C10 illustrated in FIG. 27.

In addition, the microscope 151 may include a desired filter 1515 to be detachable from the front end. Specifically, in a case in which the filter 1515 is attached thereto, the filter 1515 is held in front of each of the front end 1517 of the imaging optical system 155 and the front end 1519 of the light source supply system 157. For this reason, for example, light incident onto the imaging optical system 155 from the front side of the lens barrel 153 is restricted by the filter 1515 held in the front stage of the imaging optical system 155. With this configuration, for example, by attaching a filter that transmits only light having a predetermined wavelength band in accordance with an observation target or the like as the filter 1515, the medical imaging apparatus 150 can also be used in so-called special light observation such as fluorescence observation.

In addition, the microscope 151 may also have a zoom function. In this case, the microscope 151 may have an operation unit 1513 for controlling focal distances (i.e., zoom magnifications). For example, the operation unit 1513 is provided on a side surface of the lens barrel 153 in the example illustrated in FIGS. 28 and 29. Note that, without being limited to the operation unit 1513 for the zoom function, an input interface for realizing a special function of the microscope 151 may be provided on the microscope 151 side. This also applies to other medical optical apparatuses, without being limited to the microscope 151.

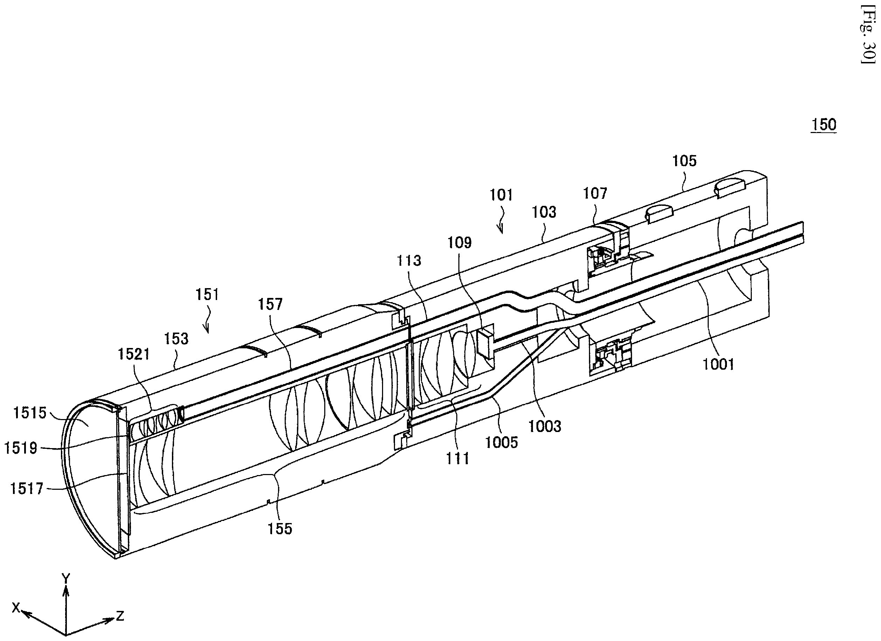

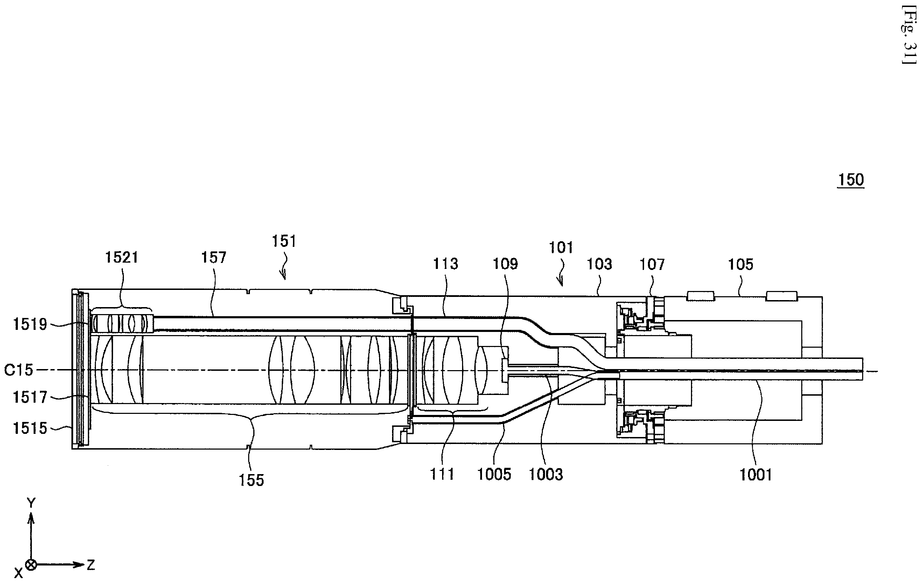

Next, an overview of an internal structure of the medical imaging apparatus 150 according to the second practical example will be described with reference to FIGS. 30 and 31. FIGS. 30 and 31 are explanatory diagram for describing an example of the internal structure of the medical imaging apparatus 150 according to the second practical example, illustrating a cross-section of the medical imaging apparatus 150 illustrated in FIGS. 28 and 29 obtained by cutting the medical imaging apparatus 150 at a y-z plane including the axis C15 illustrated in FIG. 29.

The imaging optical system 155 and the light source supply system 157 are provided in the lens barrel 153 of the microscope 151 such that the systems extend in the extension direction (i.e., the front-rear direction) of the lens barrel 153 as described above. Specifically, each of the imaging optical system 155 and the light source supply system 157 is provided to penetrate the lens barrel 153 in the extension direction of the lens barrel 153 (i.e., the front-rear direction).

The imaging optical system 155 may include an optical system for focusing on subjects (e.g., an optical system for realizing AF control). In addition, in a case in which the microscope 151 has a zoom function, the imaging optical system 155 may include an optical system for controlling focal distances (i.e., zoom magnifications). In addition, in the case in which the microscope 151 has the zoom function, a zoom optical system 1521 for controlling an irradiation range of light guided by the light source supply system 157 and emitted to outside (which may also be referred to simply as "emitted light" below) may be provided as a part of the light source supply system 157. For example, one or more lenses for controlling the irradiation range of the emitted light is provided on the front end side of the light source supply system 157 in FIGS. 30 and 31 as the zoom optical system 1521. Note that the zoom optical system 1521 may be controlled such that a position of at least part of the optical system is shifted along an optical axis so that, for example, the range of the emitted light is controlled in conjunction with control the a focal distance (zoom magnification) of the imaging optical system 155.

The overview of the example of the structure of the medical imaging apparatus according to the second practical example has been described above with reference to FIGS. 28 to 31.

### (Microscope)

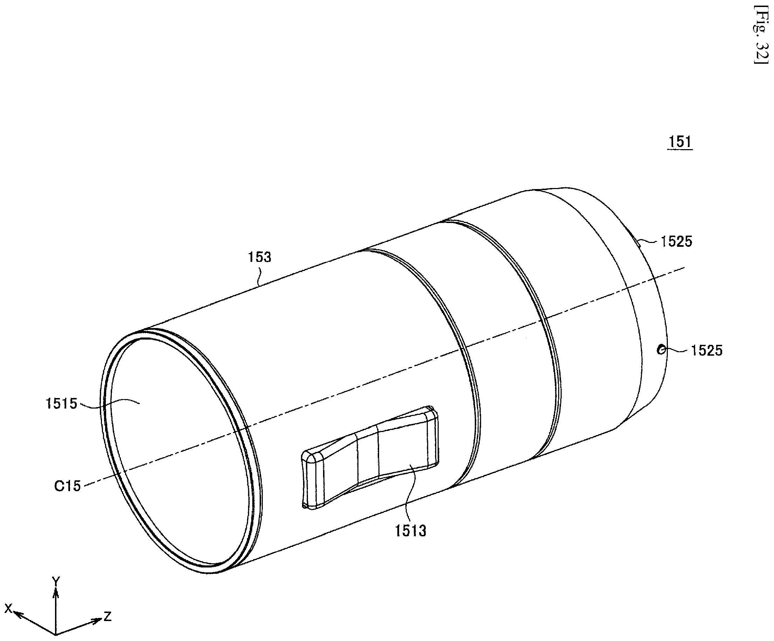

Next, as an example of the medical optical apparatus to be attached to the camera head according to the present disclosure, a structure of the microscope 151 will be described in more detail with reference to FIGS. 32 to 36. FIGS. 32 to 36 are explanatory diagram for describing an example of the structure of the microscope to be attached to the camera head according to the present disclosure.

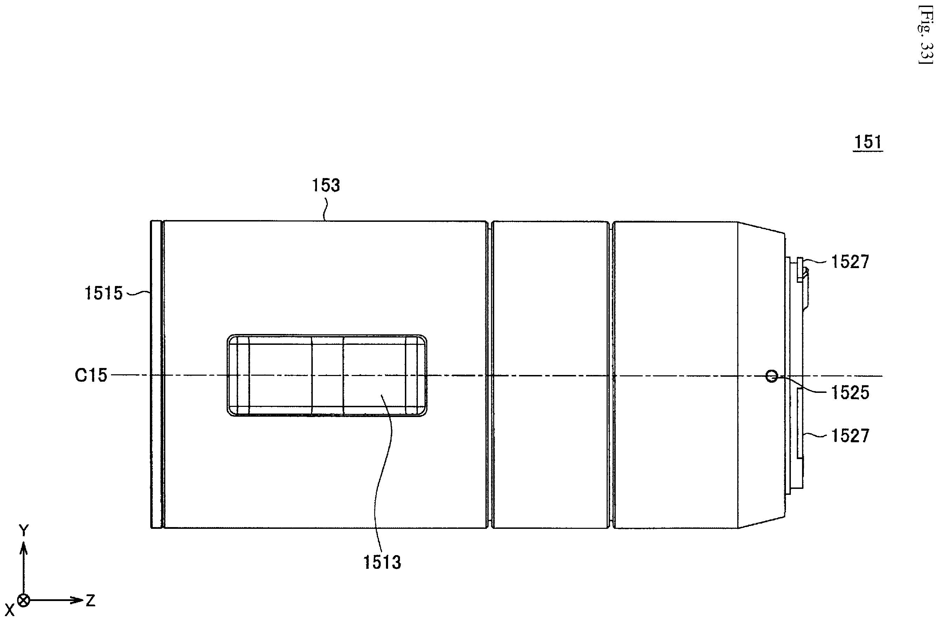

Specifically, FIG. 32 shows a perspective view of a case in which the microscope to be attached to the camera head is obliquely viewed from a front. In addition, FIG. 33 shows a side view of the microscope to be attached to the camera head. In addition, FIG. 33 shows a perspective view of a case in which the microscope to be attached to the camera head is obliquely viewed from behind. In addition,

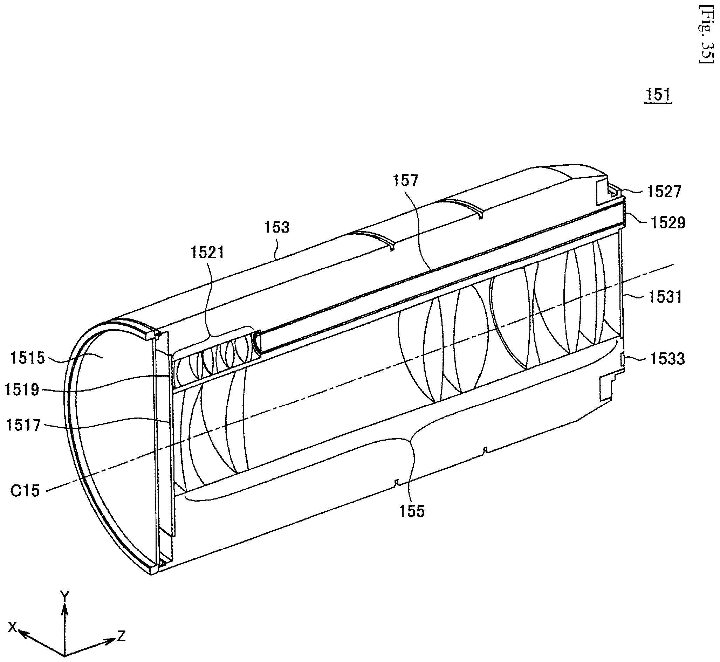

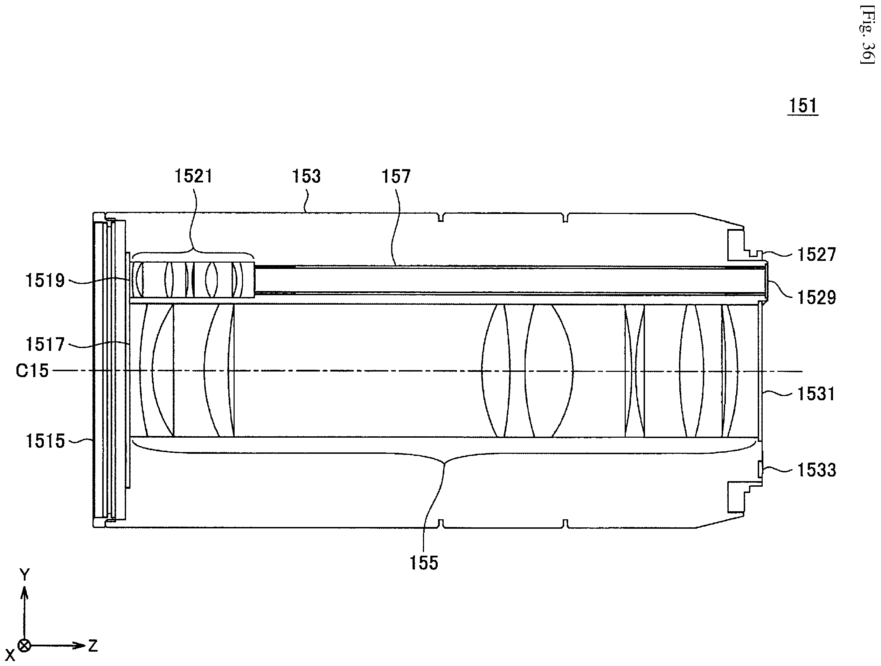

FIGS. 35 and 36 are explanatory diagrams for describing an example of an internal structure of the microscope to be attached to the camera head illustrating a cross-section obtained by cutting the microscope 151 illustrated in FIGS. 32 to 34 at the y-z plane including the axis C15 illustrated in FIG. 33. Note that the axis C15 illustrated in FIGS. 32, 33, 35, and FIG. 36 corresponds to the axis C15 illustrated in FIGS. 13 and 15. In addition, since constituent elements in FIGS. 32 to 36 given with similar reference numerals to those in FIGS. 28 to 31 represent similar constituent elements to those in the example illustrated in FIGS. 28 to 31, detailed description thereof will be omitted.

The camera head 101 can selectively have the above-described microscope 151 or rigid endoscope 131, or the like attached thereto as a medical optical apparatus. Thus, the structure of the rear end of the microscope 151 to be attached to the front end of the camera head 101 is common for the rigid endoscope 131 described above with reference to FIGS. 21 to 26.

Specifically, a detachable unit 1527 is provided on the rear end of the lens barrel 153, as a configuration for allowing the lens barrel 153 to be detachable from the front end of the support unit 103 of the camera head 101 as illustrated in FIGS. 32 to 34. The detachable unit 1527 has a similar structure to the detachable unit 1317 of the above-described rigid endoscope 131.

A lock mechanism 1535 for locking the microscope 151 so as not to come off from the support unit 103 in the case in which the microscope 151 is attached to the front end of the support unit 103 of the camera head 101 is provided on the rear end of the lens barrel 153. The lock mechanism 1535 has a similar structure to the lock mechanism 1325 of the above-described rigid endoscope 131. That is, the lock mechanism 1535 hinders rotation of the microscope 151 with respect to the support unit 103 about the axis C15 by, for example, fitting into the lock mechanism 1113 on the camera head 101 side and locks the microscope 151 in position. In addition, a marker 1525 indicating a position of the lock mechanism 1535 may be provided at a position on a side surface of the lens barrel 153 corresponding to a periphery of the lock mechanism 1535.

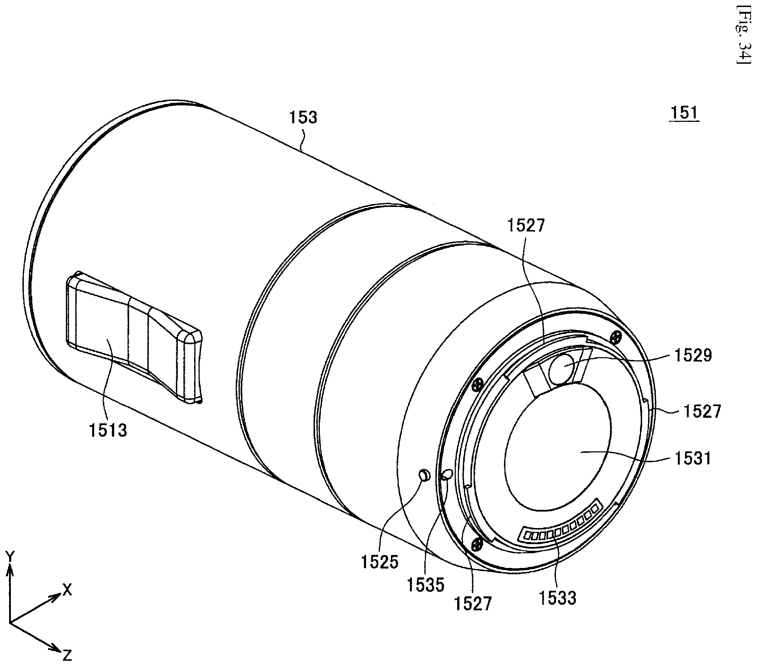

In addition, one end of the imaging optical system 155 provided in the lens barrel 153 of the microscope 151 is exposed on the rear end side of the lens barrel 153 of the microscope 151 as illustrated in FIGS. 34 to 36. For example, reference numeral 1531 represents the end (emission plane) of the imaging optical system 155. That is, the imaging optical system 155 is provided such that the emission plane 1531 faces the incidence plane 1131 of the camera head 101 in the case in which the microscope 151 is attached to the support unit 103 of the camera head 101. Accordingly, light guided by the imaging optical system 155 of the microscope 151 is incident onto the imaging optical system 111 (i.e., inside the support unit 103) via the incidence plane 1131 and forms an image on the image sensor 109 supported in the support unit 103 via the imaging optical system 111.

In addition, an end of the light source supply system 157 provided in the lens barrel 153 of the microscope 151 is exposed on the rear end side of the lens barrel 153 of the microscope 151. For example, reference numeral 1529 represents the end (incidence plane) of the light source supply system 157. That is, the light source supply system 157 is provided such that the incidence plane 1529 faces the emission plane 1133 on the camera head 101 side in the case in which the microscope 151 is attached to the support unit 103 of the camera head 101. Accordingly, light guided by the light source supply system 113 is emitted to outside of the support unit 103 from the emission plane 1133, then incident onto the light source supply system 157 from the rear end side of the lens barrel 153, then guided through the light source supply system 157, and then emitted from the front end of the lens barrel 153 toward the affected area.

In addition, reference numeral 1533 represents a terminal for the microscope 151 to receive supply of power from the camera head 101 side. The terminal 1533 is arranged at a position at which the terminal 1533 can be electrically connected to the terminal 1007 on the camera head 101 side in the case in which the microscope 151 is attached to the support unit 103 of the camera head 101. Note that power for performing AF control and zoom control in the microscope 151 may be supplied from the camera head 101 side via, for example, the terminal 1533. As a specific example, the AF control and the zoom control may be realized by driving a drive unit such as a motor with power supplied from the camera head 101 side via the terminal 1533 and shifting at least a part of the optical system (lenses, etc.) included in the imaging optical system 155 along the optical axis. This applies also to a case in which a focal distance (zoom magnification) of the imaging optical system 155 is controlled or a case in which an irradiation range of light emitted from the light source supply system 157 is controlled.

The structure of the microscope 151 has been described above in more detail with reference to FIGS. 32 to 36 as an example of the medical optical apparatus to be attached to the camera head according to the present disclosure.

### <5.4. Modified examples>

Next, another example of the structure of the camera head will be described as a modified example of the medical imaging apparatus according to the present disclosure.

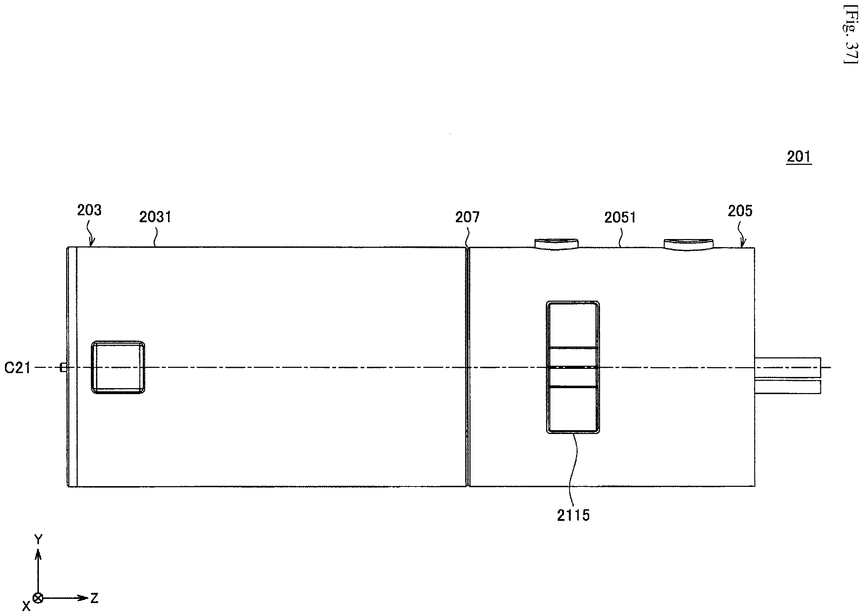

First, an overview of an example of a configuration (structure) of a camera head of a medical imaging apparatus according to a modified example will be described with reference to FIG. 37. FIG. 37 is an explanatory diagram for describing an overview of the example of the configuration of the camera head of the medical imaging apparatus according to the modified example, illustrating a side view of the camera head. Note that the camera head according to the modified example may be referred to as a "camera head 201" in the following description to be distinguished from the camera head 101 according to the above-described example.

The camera head 201 includes a support unit 203, a base unit 205, and a drive unit 207 as illustrated in FIG. 37. The support unit 203, the base unit 205, and the drive unit 207 correspond to the support unit 103, the base unit 105, and the drive unit 207 of the camera head 101 described above with reference to FIGS. 16 to 20. In addition, the axis C21 corresponds to the axis C11 of the above-described camera head 101.

Each of the support unit 203 and the base unit 205 has a housing formed in a substantially cylindrical shape. Note that, in a case in which the housings of the support unit 203 and the base unit 205 are to be distinguished from each other in the following description, the housing on the support unit 203 side will be referred to as a "housing 2031" and housing on the base unit 205 side will be referred to as a "housing 2051" for the sake of convenience. In addition, the base unit 205 is formed in a hollow shape in which the housing 2051 has an opening 2117 along the axis C21. Note that the opening 2117 is formed in a substantially cylindrical shape having the central axis substantially coinciding with the axis C21. In other words, the housing 2051 of the base unit 205 has a cylindrical hollow portion having the central axis substantially coinciding with the axis C21.

An end of the support unit 203 on one side in an axial direction (the z direction) of the substantially cylindrical housing 2051 (a rear end) is rotatably supported by an end of the base unit 205 on one side in an axial direction (the z direction) of the substantially cylindrical housing 2031 (a front end) about the axis (i.e., the central axis C21 illustrated in FIG. 37). In addition, a drive unit 207 is interposed at the portion of the base unit 205 supporting the support unit 203, and the drive unit 207 is driven to cause the support unit 203 to rotate with respect to the base unit 205 about the axis C21. In addition, an operation unit 215 for operating drive of the drive unit 207 is provided on a side surface of the base unit 205. That is, the drive unit 207 is driven when an operator operates the operation unit 215, and the support unit 203 rotates about the axis C21 along with the drive of the drive unit 207.

The support unit 203 has the medical optical apparatus such as the above-described rigid endoscope 131 (see FIGS. 21 to 26) or microscope 151 (see FIGS. 32 to 36) detachable from its front end (i.e., the end on the opposite side to the base unit 205). Note that, since the structure of the medical optical apparatus detachable from the support unit 203 is similar to that of the support unit 103 of the camera head 101, detailed description thereof will be omitted.

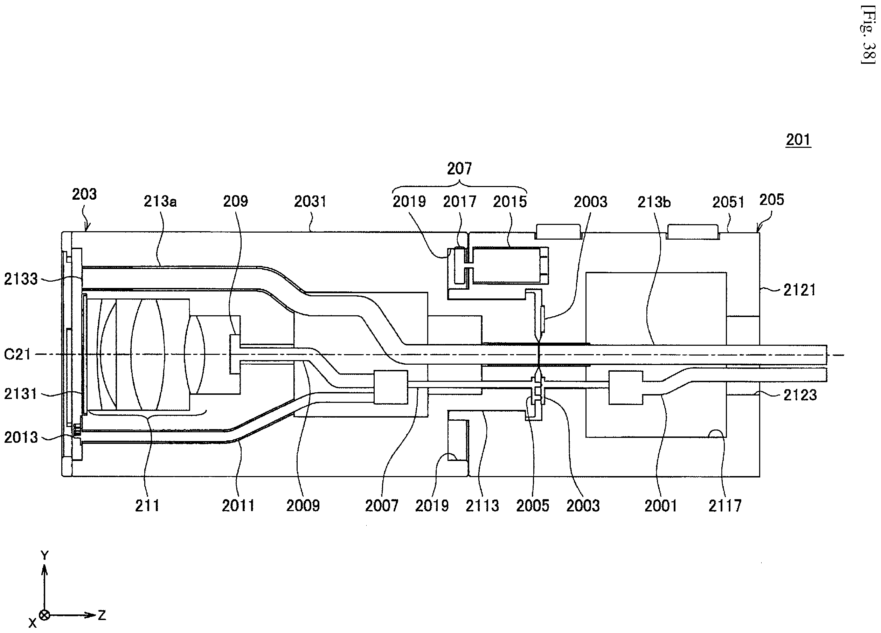

Next, an overview of an example of an internal structure of the camera head of the medical imaging apparatus according to the modified example will be described with reference to FIG. 38. FIG. 38 is an explanatory diagram for describing the overview of the example of the internal configuration of the camera head of the medical imaging apparatus according to the modified example, illustrating a cross-section obtained by cutting the camera head 201 illustrated in FIG. 37 at the y-z plane including the axis C21 illustrated in FIG. 37.

A light source supply system 213 is supported in the camera head 201 such that it extends from the front side to the rear side. Note that in the camera head 201 according to the modified example, the light source supply system 213 is physically separated into a portion supported on the support unit 203 side and a portion supported on the base unit 205 side. Thus, in the following description, the portion of the light source supply system 213 supported on the support unit 203 side will also be referred to as a "light source supply system 213a" and the portion thereof supported on the base unit 205 side will also be referred to as a "light source supply system 213b" for the sake of convenience. In addition, in a case in which the light source supply systems 213a and 213b are not particularly distinguished, they may be referred to simply as "light source supply systems 213". Note that the light source supply system 213a supported on the support unit 203 side corresponds to an example of a "first light source supply system," and the light source supply system 213b supported on the base unit 205 side corresponds to an example of a "second light source supply system."

The light source supply system 213a extends to penetrate the support unit 203 from the front side to the rear side (i.e., the extension direction of the axis C21). In addition, the light source supply system 213b is supported in the hollow portion of the housing 2051 (i.e., inside the opening 2117) to extend rearward (i.e., along the axis C21). Specifically, the light source supply system 213b extends to penetrate the base unit 205 from the front side to the rear side (i.e., the extension direction of the axis C21), and a part thereof is exposed from an opening 2123 provided on a rear end 2121 of the housing 2051 of the base unit 205 to outside of the housing 2051 (i.e., outside the camera head 201) as illustrated in FIG. 38. In the example of FIG. 38, the opening 2123 is formed in a substantially cylindrical shape having the central axis substantially coinciding with the axis C21. Note that the portion of the light source supply system 213b exposed rearward from the opening 2123 provided on the rear end 2121 of the camera head 201 is connected to, for example, a light source apparatus or another light source supply system connected to the light source apparatus. With this configuration, even in a case in which the support unit 203 rotates with respect to the base unit 205, the light source supply systems 213a and 213b are supported such that the state in which the rear end of the light source supply system 213a faces the front end of the light source supply system 213b is maintained.

Specifically, the light source supply system 213a is arranged such that the central axis of the rear end of the light source supply system 213a substantially coincides with the axis C21 (e.g., the rotation axis) on the rear end of the support unit 203. In addition, the light source supply system 213b is arranged such that the central axis of the front end of the light source supply system 213b substantially coincides with the axis C21 (e.g., the rotation axis) on the front end of the base unit 205. With this configuration, even in a case in which the support unit 203 rotates with respect to the base unit 205, a state in which each of the rear end of the light source supply system 213a and the front end of the light source supply system 213b is positioned on the axis C21 (i.e., on the rotation axis) is maintained. That is, even in the case in which the support unit 203 rotates with respect to the base unit 205, the state in which the rear end of the light source supply system 213a faces the front end of the light source supply system 213b is maintained. Accordingly, even in the case in which the support unit 203 rotates with respect to the base unit 205, light from the light source apparatus guided through the light source supply system 213b can be caused to be incident on the light source supply system 213a.

In addition, a front end of the light source supply system 213a is exposed on the front end of the support unit 203. Note that, in the following description, the end will also be referred to as an "emission plane 2133" for the sake of convenience. That is, light emitted from the light source apparatus and guided through the light source supply system 213 is emitted from the emission plane 2133 to outside the camera head 201 (i.e., outside the support unit 103).

The housing 2031 of the support unit 203 supports the image sensor 209 therein, and the front side of the image sensor 209 is opened. Note that, in the present practical example, the image sensor 209 is supported such that the center of the imaging plane of the image sensor 209 substantially coincides with the axis C21 on an x-y plane. In addition, the support unit 203 may support the imaging optical system 211 in the opening such that the imaging optical system 211 is positioned in the front stage (front side) of the image sensor 209. In the case in which the rigid endoscope 131 is attached to the support unit 203, the opening faces the end of the imaging optical system 137 exposed to the rear end side of the base unit 133 of the rigid endoscope 131. That is, the imaging optical system 137 is positioned in the front stage of the image sensor 209. Accordingly, light guided by the imaging optical system 137 is incident from the opening into the camera head 201 and forms an image on the image sensor 209 via the imaging optical system 211. Note that this configuration is not limited only to the rigid endoscope 131 but is applied to a medical optical apparatus that is detachable from the camera head of the medical imaging apparatus, such as the microscope 151.

In addition, a signal line for supplying power from an external apparatus (a power supply apparatus, etc.) is connected to the image sensor 209. Specifically, the support unit 203 has a terminal 2005 exposed on the rear end and a signal line 2007 in the housing 2031 that extends forward from the terminal 2005. The terminal 2005 is electrically connected to a terminal 2003 exposed on the front end of the base unit 205. A signal line 2001 is electrically connected to the terminal 2003 on the opposite side to the front exposed portion of the support unit 203 in the housing 2051 (e.g., in the opening 2117) of the base unit 205 formed in the hollow shape. The signal line 2001 is supported to extend rearward from the portion connected to the terminal 2003 (i.e., along the axis C21) in the hollow portion of the housing 2051 (i.e., inside the opening 2117). In addition, the signal line 2001 is exposed outside the housing 2051 from the opening 2123 provided on the rear end 2121 of the housing 2051 and further extends to the rear side of the camera head 201. Note that the portion of the signal line 2001 further extending to the rear side of the camera head 201 is electrically connected to an external apparatus. In addition, the signal line 2007 branches into signal lines 2009 and 2011, and the signal line 2009 is electrically connected to the image sensor 209. Accordingly, power can be supplied from the external apparatus (the power supply apparatus, etc.) to the image sensor 209 via the signal lines 2001, 2007, and 2009. Note that the signal lines 2007 and 2009 supported on the support unit 203 side correspond to an example a "first signal line," and the signal line 2001 supported on the base unit 205 side corresponds to an example of a "second signal line." In addition, the rear end of the signal line 2007 or the terminal 2005 connected to the end corresponds to an example of a "fifth end." In addition, the front end of the signal line 2001 connected to the terminal 2003 corresponds to an example of a "sixth end."

In addition, the signal line 2011 is electrically connected to a terminal 2013 exposed on the front end of the support unit 203. The terminal 2013 corresponds to the terminal 1007 of the above-described camera head 101. Accordingly, power can be supplied from the external apparatus (power supply apparatus, etc.) to the medical optical apparatus attached to the front end of the support unit 203 via the signal lines 2001, 2007, and 2011.

Note that, even in the case in which the support unit 203 rotates with respect to the base unit 205, the electrical connection relation between the terminal 2005 on the support unit 203 side and the terminal 2003 on the base unit 205 side is maintained. A mechanism for maintaining the electrical connection relation between the terminal 2005 and the terminal 2003 as described above will be separately described in detail.

The drive unit 207 is constituted by a pinion gear 2017 and a motor 2015 on the base unit 205 side and a gear 2019 on the support unit 203 side. The pinion gear 2017 and the motor 2015 for rotating the pinion gear 2017 are supported on the front end of the base unit 205 as illustrated in FIG. 37. At this time, a rotation axis of the pinion gear 2017 is separated from the axis C21 in the radial direction and supported to be in substantially parallel with the axis C21. In addition, the support unit 203 extends rearward such that a partial side surface of the substantially cylindrical housing 2031 has an inner circumferential surface on the rear end, and the gear 2019 (internal gear) is formed along the inner circumferential surface. The pinion gear 2017 fits to the gear 2019, and when the pinion gear 2017 rotates by driving of the motor 2015, the support unit 203 rotates with respect to the base unit 205 about the axis C21. Note that the above-described configuration of the drive unit 207 is merely an example, and a configuration of the drive unit 207 is not particularly limited as long as it causes the support unit 203 to rotate with respect to the base unit 205 about the axis C21. As a specific example, the drive unit 207 may have a ring-shaped ultrasonic motor, like the drive unit 107 of the above-described camera head 101.

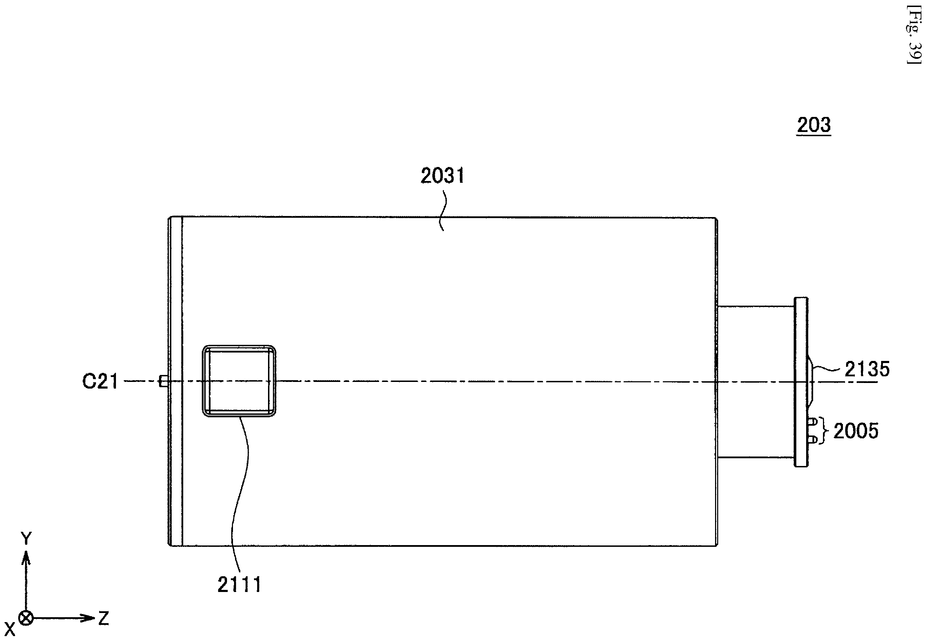

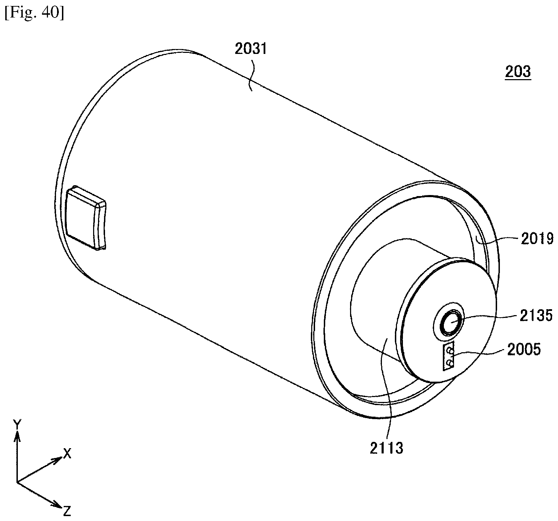

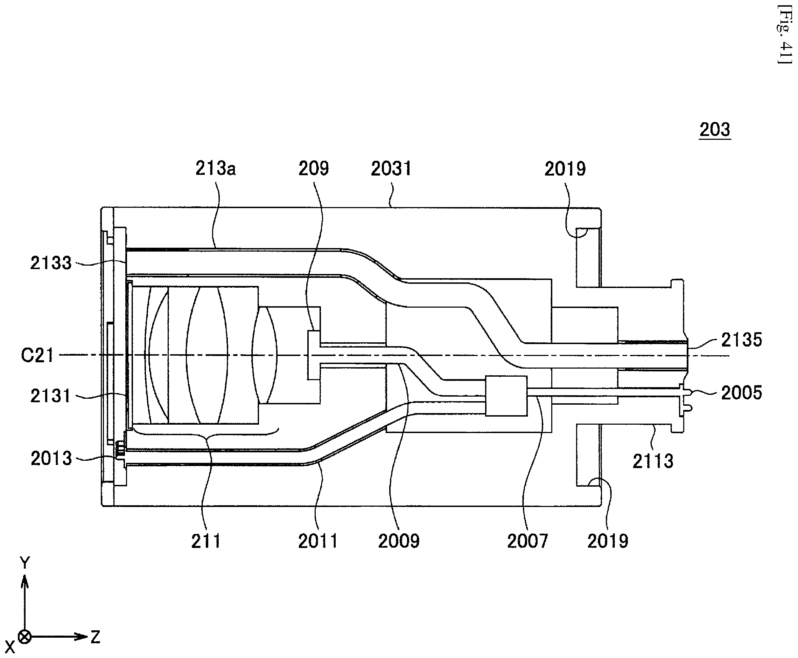

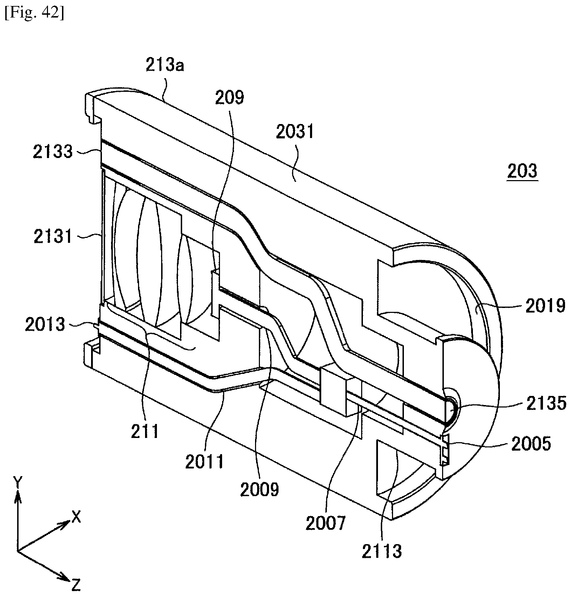

Here, in a structure of the support unit 203, particularly a structure of the rear end thereof will be described with reference to FIGS. 39 to 42. FIGS. 39 and 40 are explanatory diagrams for describing an example of a structure of the support unit of the camera head according to the modified example, illustrating an appearance of the support unit. Specifically, FIG. 39 illustrates a side view of the support unit of the camera head according to the modified example. In addition, FIG. 40 illustrates a perspective view obtained by obliquely viewing the support unit of the camera head according to the modified example from a rear side. In addition, FIGS. 41 and 42 are explanatory diagrams for describing an example of an internal structure of the support unit of the camera head according to the modified example, illustrating a cross-section obtained by cutting the support unit 203 illustrated in FIGS. 39 and 40 at the y-z plane including the axis C21 illustrated in FIG. 39. Note that the axis C21 illustrated in FIGS. 39 and 41 corresponds to the axis C21 illustrated in FIGS. 37 and 38. In addition, since constituent elements in FIGS. 39 to 42 given with similar reference numerals to those in FIGS. 37 and 38 represent similar constituent elements in FIGS. 37 and 38, detailed description thereof will be omitted.

A substantially cylindrical protrusion 2113 extending along the axis C21 is formed on the rear end of the support unit 203 to have the central axis substantially coinciding with the axis C21 as illustrated in FIGS. 39 and 40. Note that the protrusion 2113 is formed to have a width in the radial direction around the axis C21 shorter than the width of the housing 2031 in the radial direction. In addition, the support unit 203 extends rearward such that a partial side surface of the substantially cylindrical housing 2031 has the inner circumferential surface on the rear end, and the gear 2019 is formed along the inner circumferential surface as described above.

In addition, the rear end (which will also be referred to as an "incidence plane 2135" below) of the light source supply system 213a and a part of the terminal 2005 are exposed on the rear end of the protrusion 2113 as illustrated in FIGS. 39 to 42. Note that the light source supply system 213a is arranged such that the central axis of the rear end of the light source supply system 213a substantially coincides with the axis C21 (i.e., rotation axis) on the rear end of the support unit 203 as described above. That is, the position of the center of incidence plane 2135 substantially coincides with the position of the axis C21 on an x-y plane. In addition, the terminal 2005 is arranged on the rear end of the protrusion 2113 at a position separated from the axis C21 in the radial direction as illustrated in FIGS. 39 to 42. Note that the rear end (i.e., the incidence plane 2135) of the light source supply system 213a corresponds to an example of a "third end."

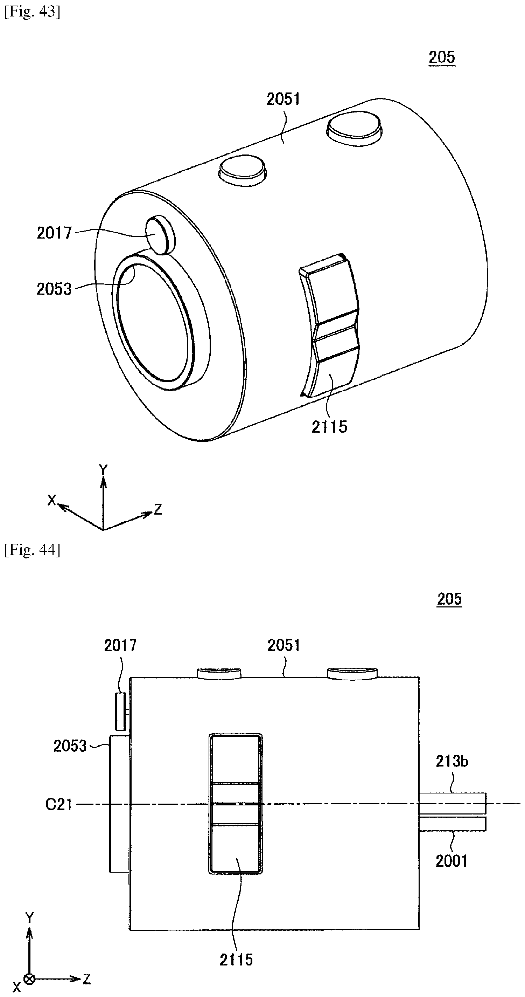

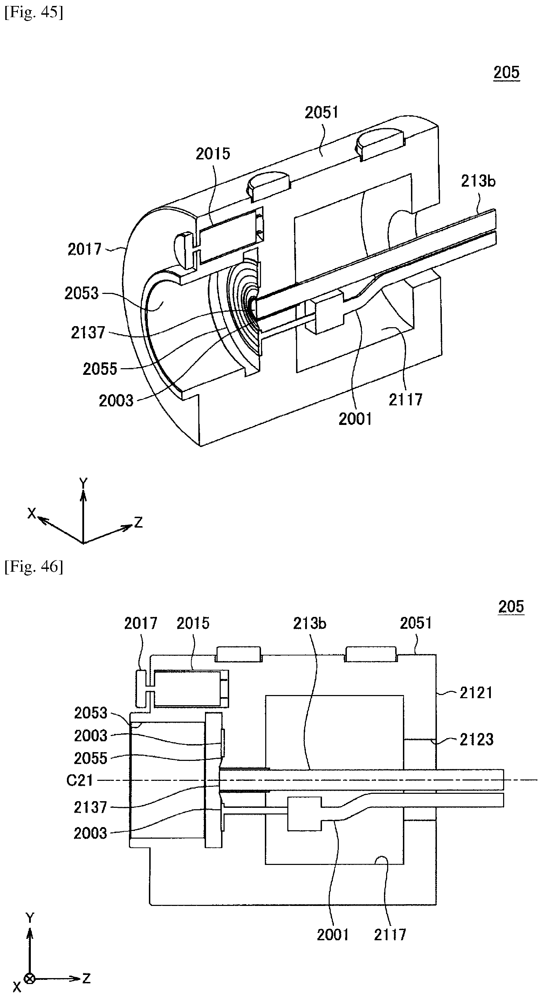

Next, in a structure of the base unit 205, particularly a structure of the front end thereof will be described with reference to FIGS. 43 to 46. FIGS. 43 and 44 are explanatory diagrams for describing an example of a structure of the base unit of the camera head according to the modified example, illustrating an appearance of the base unit. Specifically, FIG. 43 illustrates a perspective view obtained by obliquely viewing the base unit of the camera head according to the modified example viewed from the front side. In addition, FIG. 44 illustrates a side view of the base unit of the camera head according to the modified example. In addition, FIGS. 45 and 46 are explanatory diagrams for describing an example of an internal structure of the support unit of the camera head according to the modified example, illustrating a cross-section obtained by cutting the base unit 205 illustrated in FIGS. 43 and 44 at the y-z plane including the axis C21 illustrated in FIG. 44. Note that the axis C21 illustrated in FIGS. 44 and 46 corresponds to the axis C21 illustrated in FIGS. 37 and 38. In addition, since constituent elements in FIGS. 43 to 46 given with similar reference numerals to those in FIGS. 37 and 38 represent similar constituent elements in FIGS. 37 and 38, detailed description thereof will be omitted.

A recess 2053 having an opening formed in a substantially cylindrical shape extending along the axis C21 is formed on the front end of the base unit 205 such that the central axis of the opening substantially coincides with the axis C21 as illustrated in FIGS. 43, 45, and 46. Note that the recess 2053 is formed to have a width in the radial direction of the opening (i.e., a width in the radial direction around the axis C21) shorter than the width of the housing 2051 in the radial direction. In addition, the pinion gear 2017 is supported to be at a different position from the recess 2053 on the front end of the base unit 205. That is, the pinion gear 2017 is positioned outside the recess 2053 from the axis C21.

In addition, the recess 2053 is formed such that the inner diameter thereof is slightly wider than the outer diameter of the protrusion 2113 provided on the support unit 203 side. With this configuration, when the protrusion 2113 fits to the recess 2053, the support unit 203 is rotatably supported by the base unit 205 about the axis C21. In addition, in the case in which the protrusion 2113 fits to the recess 2053, the pinion gear 2017 fits to the gear 2019 provided on the support unit 203 side. With this configuration, when the pinion gear 2017 rotates by driving of the motor 2015 in the state in which the support unit 203 is supported by the base unit 205, the support unit 203 rotates with respect to the base unit 205 about the axis C21.

In addition, a trough surface 2055 formed to extend in the x-y direction is provided in the recess 2053 without connecting to the opening of the recess 2053 and the hollow portion of the housing 2051 (i.e., the opening 2117) as illustrated in FIGS. 45 and 46. Note that the depth of the opening of the recess 2053 (a width in the z direction) is adjusted such that the rear end of the protrusion 2113 comes in contact with the trough surface 2055 in the case in which the protrusion 2113 fits to the recess 2053.

The front end of the light source supply system 213b (which will also be referred to as an "emission plane 2137" below) and a part of the terminal 2003 are exposed on the trough surface 2055. Note that, the light source supply system 213b is arranged such that the central axis of the front end of the light source supply system 213b substantially coincides with the axis C21 (i.e., the rotation axis) on the front end of the base unit 205 as described above. That is, the position of the center of the emission plane 2137 substantially coincides with the position of the axis C21 on an x-y plane. With this configuration, in the case in which the protrusion 2113 fits to the recess 2053, the emission plane 2137 exposed on the trough surface 2055 faces the incidence plane 2135 exposed on the rear end of the protrusion 2113. In addition, each of the central axis of the light source supply system 213b on the emission plane 2137 and the central axis of the light source supply system 213a on the incidence plane 2135 substantially coincides with the axis C21. For this reason, even in the case in which the support unit 203 rotates with respect to the base unit 205 about the axis C21, the state in which the emission plane 2137 faces the incidence plane 2135 is maintained. Note that the front end of the light source supply system 213b (i.e., the emission plane 2137) corresponds to an example of a "fourth end."

In addition, the terminal 2003 is arranged at a position on the trough surface 2055 separated from the axis C21 in the radial direction as illustrated in FIGS. 45 and 46. In addition, a portion of the terminal 2003 exposed on the trough surface 2055 is formed in a ring shape extending in a circumferential direction around the axis C21 as illustrated in FIG. 45. At this time, the distance in which the axis C21 is separated from the terminal 2003 in the radial direction is substantially equal to the distance in which the axis C21 is separated from the terminal 2005 in the radial direction on the rear end of the protrusion 2113. With this configuration, in the case in which the protrusion 2113 fits to the recess 2053, the terminal 2005 exposed on the rear end of the protrusion 2113 comes in contact with at least a part (e.g., a part in the circumferential direction) of the terminal 2003 exposed on the trough surface 2055. That is, via the terminal 2003 and the terminal 2005 coming in contact with each other, the signal line 2001 supported on the base unit 205 side is electrically connected to the signal line 2007 (further, the signal lines 2009 and 2011) supported on the support unit 203 side. In addition, with the above-described configuration, even in the case in which the support unit 203 rotates with respect to the base unit 205 about the axis C21, the state in which at least a part of the terminal 2003 exposed on the trough surface 2055 comes in contact with the terminal 2005 exposed on the rear end of the protrusion 2113 is maintained. That is, the electrical connection relation between the terminal 2003 and the terminal 2005 can be maintained.

Note that, although the case in which the terminal 2003 has a ring shape has been described in the above example, configurations of the terminals 2003 and 2005 are not necessarily limited to the above-described example as long as at least one of the terminals 2003 or 2005 is formed in a ring shape. That is, the terminals 2003 and 2005 may be configured such that the terminal 2005 side is formed in a ring shape and the terminal 2003 comes in contact with a part of the terminal 2005 in the circumferential direction. In addition, both the terminals 2003 and 2005 may be formed in a ring shape.

With this configuration, even when the support unit 203 rotates with respect to the base unit 205 about the axis C21 in the camera head 201 according to the modified example, the light source supply system 213b and optionally the signal line 2001 on the base unit 205 side do not rotate in conjunction therewith. That is, even when the support unit 203 rotates with respect to the base unit 205, the bundle of the light source supply system 213b and the signal line 2001 is not tangled. Thus, a substantial physical restriction on the rotation of the support unit 203 with respect to the base unit 205 about the axis C21 does not occur in the camera head 201 according to the modified example.

The other example of the structure of the camera head has been described with reference to FIGS. 37 to 46 as a modified example of the medical imaging apparatus according to the present disclosure.

### <<6. Application examples>>

Next, application examples of the medical imaging apparatus will be described.

### <6.1. Application Example 1: Example of control in conjunction with arm>

First, as Application example 1, an example in which control of the medical imaging apparatus is in conjunction with control of an arm unit in a case in which the medical imaging apparatus is attached to a supporting arm apparatus will be described. Note that, in the following description, the medical imaging apparatus is assumed to be attached to an arm unit 420 of a supporting arm apparatus 400.

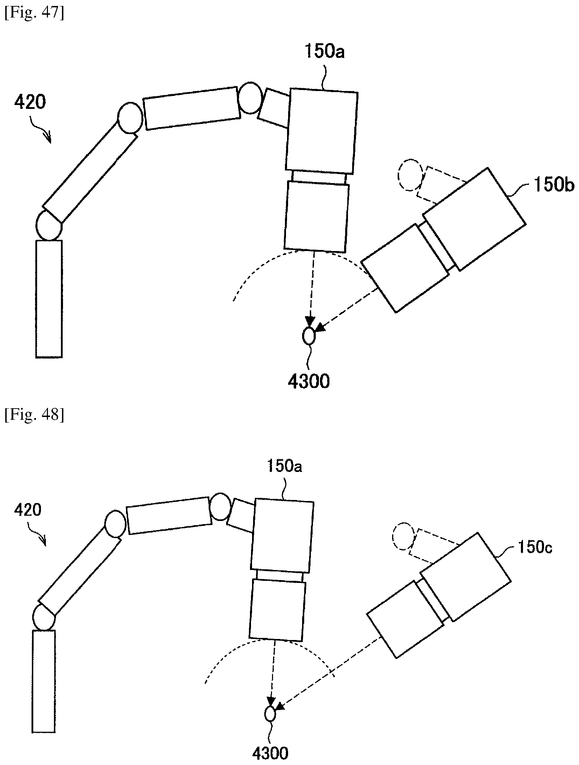

For example, FIG. 47 is an explanatory diagram for describing an aspect of Application Example 1 of the medical imaging apparatus. Specifically, FIG. 47 schematically illustrates an example of control of the arm unit 420 in a case in which the medical imaging apparatus 150 in which a fixed-focus microscope 151 is attached to the camera head 101 is held by the arm unit 420. In FIG. 47, reference numerals 150a and 150b schematically represent positions of the medical imaging apparatus 150 in each direction in a case in which a subject 4300 is captured by the medical imaging apparatus 150 in the different directions.

In the example illustrated in FIG. 47, motions of the arm unit 420 supporting the medical imaging apparatus 150 are restricted so that the subject 4300 is positioned within an imaging range of the medical imaging apparatus 150 in a case in which the medical imaging apparatus 150 is moved with respect to the subject 4300. In addition, at this time, motions of the arm unit 420 are restricted so that a distance between the microscope 151 and the subject 4300 is maintained to a distance in which the microscope 151 can focus on the subject 4300. With this control, while the microscope 151 maintains the focused state on the subject 4300, the medical imaging apparatus 150 can be moved with respect to the subject 4300 (e.g., make a pivot motion).

In this case, rotation of the support unit 103 (i.e., rotation of the image sensor 109 and the light source supply system 113) with respect to the base unit 105 of the camera head 101 may be controlled in accordance with the motions of the arm unit 420. Accordingly, attitudes of the image sensor 109 supported by the support unit 103 can also be controlled such that hand-eye coordination is maintained, for example, even in a case in which a relative position of the medical imaging apparatus 150 to the subject 4300 changes in accordance with a motion of the arm unit 420.

In addition, FIG. 48 is an explanatory diagram for describing another aspect of Application Example 1 of the medical imaging apparatus. FIG. 48 schematically illustrates an example of control of the arm unit 420 in a case in which the medical imaging apparatus 150 in which a microscope 151 capable of focus control is attached to the camera head 101 is held by the arm unit 420. In FIG. 48, reference numerals 150a and 150c schematically represent positions of the medical imaging apparatus 150 in each direction in a case in which a subject 4300 is captured by the medical imaging apparatus 150 in the different directions.

Also in the example illustrated in FIG. 48, in a case in which the medical imaging apparatus 150 is moved with respect to the subject 4300, motions of the arm unit 420 supporting the medical imaging apparatus 150 are restricted such that the subject 4300 is positioned within an imaging range of the medical imaging apparatus 150. Meanwhile, a focal distance of the microscope 151 can be controlled by AF control or the like in the example illustrated in FIG. 48. Thus, even when a distance between the microscope 151 and the subject 4300 is changed, the state in which the microscope 151 focuses on the subject 4300 can be maintained by controlling a focal distance of the microscope 151, unlike in the example illustrated in FIG. 47. That is, in a case in which a distance between the microscope 151 and the subject 4300 is changed in accordance with a motion of the arm unit 420 in the example illustrated in FIG. 48, the state in which the microscope 151 focuses on the subject 4300 is maintained by controlling a focal distance of the microscope 151.

Note that, also in that case, rotation of the support unit 103 with respect to the base unit 105 of the camera head 101 (i.e., rotation of the image sensor 109 and the light source supply system 113) may be controlled in accordance with a motion of the arm unit 420, as in the example illustrated in FIG. 47.

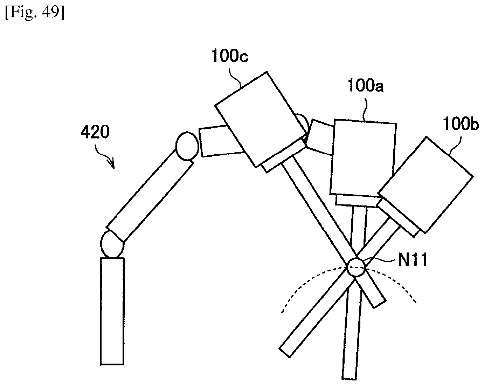

In addition, FIG. 49 is an explanatory diagram for describing another aspect of Application Example 1 of the medical imaging apparatus. FIG. 49 schematically illustrates an example of control of the arm unit 420 in a case in which the medical imaging apparatus 100 with the rigid endoscope 131 attached to the camera head 101 is held by the arm unit 420. In FIG. 49, reference numerals 100a, 100b, and 100c schematically represent positions and attitudes of the medical imaging apparatus 100 corresponding to different directions in a case in which an inside of a body cavity is captured in each of the directions with the rigid endoscope 131 inserted into the body cavity via a trocar. In addition, in FIG. 49, reference numeral N11 schematically represents a position held by the trocar (which will also be referred to as a "trocar point" below).

In the example illustrated in FIG. 49, even in a case in which the medical imaging apparatus 100 is moved to change a position and an attitude of the rigid endoscope 131, motions of the arm unit 420 are restricted so that a position of the trocar point N11 (i.e., a position of an insertion hole into which the lens barrel 135 of the rigid endoscope 131 is inserted) is maintained.

Also in that case, rotation of the support unit 103 with respect to the base unit 105 of the camera head 101 (i.e., rotation of the image sensor 109 and the light source supply system 113) may be controlled in accordance with a motion of the arm unit 420, as in the example illustrated in FIG. 47.

The example in which control of the medical imaging apparatus is in conjunction with control of the arm unit in the case in which the medical imaging apparatus is attached to the supporting arm apparatus has been described above as Application Example 1 with reference to FIGS. 47 to 49.

### <6.2. Application Example 2: Control example of arm that supports oblique-viewing endoscope>

Next, an example of a technique that can maintain hand-eye coordination in a case in which an oblique-viewing endoscope is used as the rigid endoscope 131 to be attached to the camera head 101 will be described as Application Example 2.

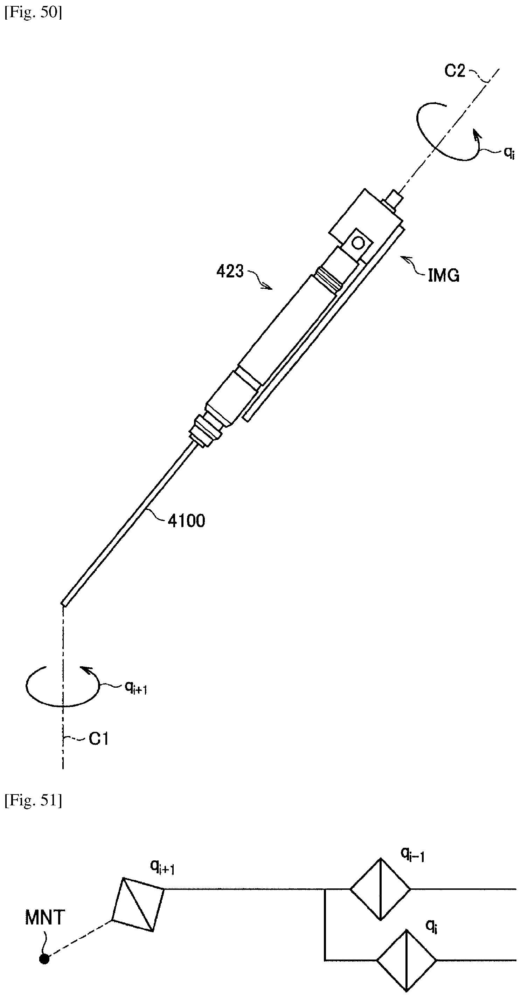

First, a method of use and an operation of the oblique-viewing endoscope will be described. FIG. 50 is an explanatory diagram for describing Application Example 2 of the medical imaging apparatus for describing an operation of the oblique-viewing endoscope. Referring to FIG. 50, an oblique-viewing endoscope optical axis C1 is oblique with respect to a rigid endoscope axis C2. In addition, referring to FIG. 50, an endoscope apparatus 423 has a camera head CH.

Here, a scopist rotates the camera head CH and adjusts a monitor screen to maintain hand-eye coordination of an operator in accordance with a rotation operation of the oblique-viewing endoscope during surgery. Then, when the scopist rotates the camera head CH, dynamic characteristics of the arm around rigid endoscope axis C2 change. A display screen on the monitor rotates about the oblique-viewing endoscope optical axis C1. In FIG. 50, a rotation angle about the rigid endoscope axis C2 is denoted by qᵢ , and a rotation angle about the oblique-viewing endoscope optical axis C1 is denoted by qᵢ+1.

The medical imaging apparatus according to Application Example 2 performs control by modeling units of the oblique-viewing endoscope as a plurality of conjunctive links. For example, FIG. 51 is an explanatory diagram for describing Application Example 2 of the medical imaging apparatus illustrating an example in which characteristics of an operation around the rigid endoscope axis C2 and an operation around the oblique-viewing endoscope optical axis C1 illustrated in FIG. 50 are modeled. Referring to FIG. 51, rotation angles of each of the links are shown. Note that when the plurality of conjunctive links are modeled, the plurality of conjunctive links may include actual links and virtual links. For example, in the examples illustrated in FIGS. 50 and 51, the rigid endoscope axis C2 corresponds to an example of an axis of the actual links (actual rotary links), and the oblique-viewing endoscope optical axis C1 corresponds to an example of an axis of the virtual links (virtual rotary links).

In addition, the medical imaging apparatus according to Application Example 2 performs unified whole-body cooperative control through expansion using a relative motion space and the conjunctive links. An actual rotation axis and a virtual rotation axis are considered in a joint space. The actual rotation axis and the virtual rotation axis are not dependent on an arm configuration. In addition, a relative motion space is considered for a motion objective, in addition to a Descartes space. By changing a motion objective in the Descartes space, various operations can be performed.

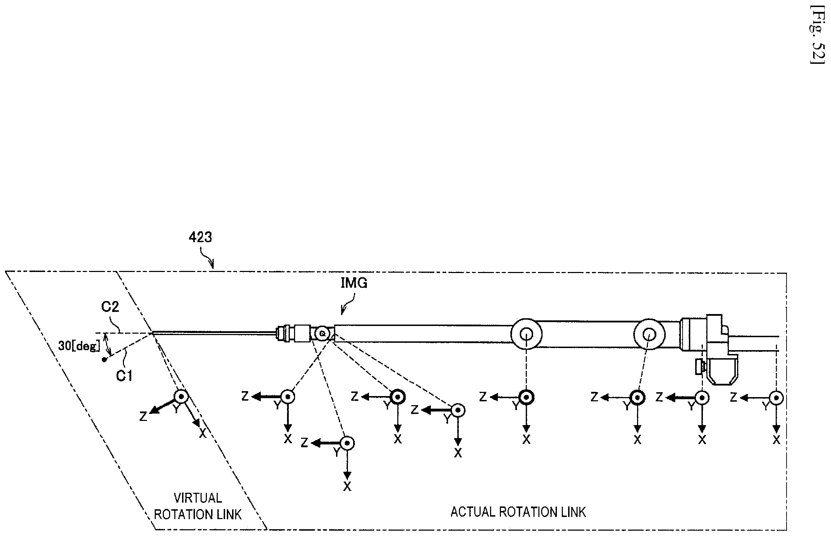

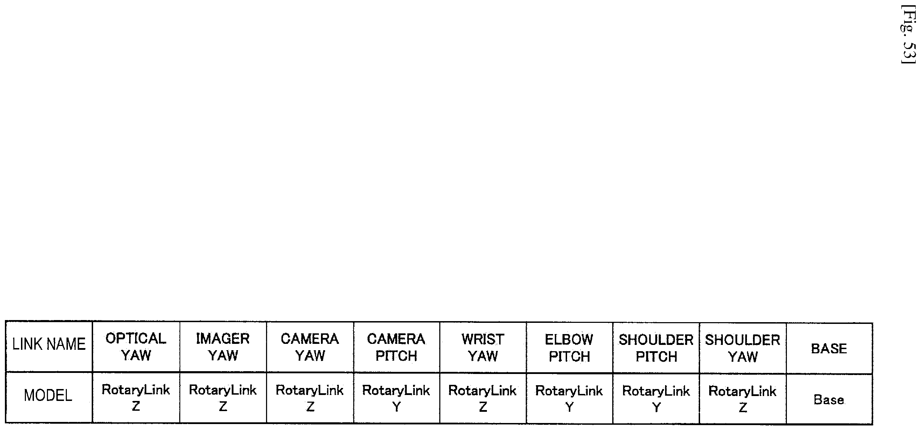

FIGS. 52 and 53, for example, are explanatory diagrams for describing Application Example 2 of the medical imaging apparatus illustrating an example of each of link configurations in a case in which expansion of whole-body cooperative control is applied to a six-axis arm and units of the oblique-viewing endoscope.

Next, a setting of virtual links will be described. The setting of virtual links is made by, for example, a control unit that controls operations of the supporting arm apparatus 400. Thus, the control unit will also be referred to as a "control unit 300" for the sake of convenience in the following description.

The control unit 300 can function as a virtual link setting unit that sets virtual rotary links as examples of virtual links. For example, the control unit 300 sets virtual links by setting at least one of distances or orientation of the virtual links.

The control unit 300 models virtual rotary links on the basis of a coordinate system defined with reference to distal ends of actual rotary links of the arm, arbitrary points on the oblique-viewing endoscope optical axis C1, and a line connecting the points, and uses whole-body cooperative control. Accordingly, without depending on a hardware configuration of the arm, motion objectives such as a fixed viewpoint in a direction of an arbitrary point on the distal end of the virtual rotary links can be realized while maintaining a fixed attitude in the coordinate system of the virtual rotary links and a position of a trocar point that is a scope insertion point during surgery.

The control unit 300 can set the virtual rotary links on the basis of specifications of a connected scope and an arbitrary point in a space.

The specifications of the scope can include at least one of an oblique viewing angle, dimensions, or a focal distance of the scope. Alternatively, the specifications of the scope may include a position of an axis of the scope (information regarding the axis of the scope can be used to set the actual rotary links). Accordingly, a motion objective can be switched in accordance with the specifications of the scope.

Note that the case in which the scope is an oblique-viewing endoscope has been mainly assumed above. However, an oblique viewing angle of the scope is arbitrarily changeable on the basis of the specifications of the scope as described above. Therefore, the scope may be a straight-viewing scope or a side-viewing scope.

In general, in use cases of a zoom operation of changing an amount of an oblique-viewing endoscope to be inserted into a body cavity or a scope rotation operation of changing a visual-field direction of the oblique-viewing endoscope, in a case in which the operation is performed only with reference to the actual rotary links of the arm, without considering an optical axis direction of the oblique-viewing endoscope, it is difficult to catch an observation target at the center of the camera.

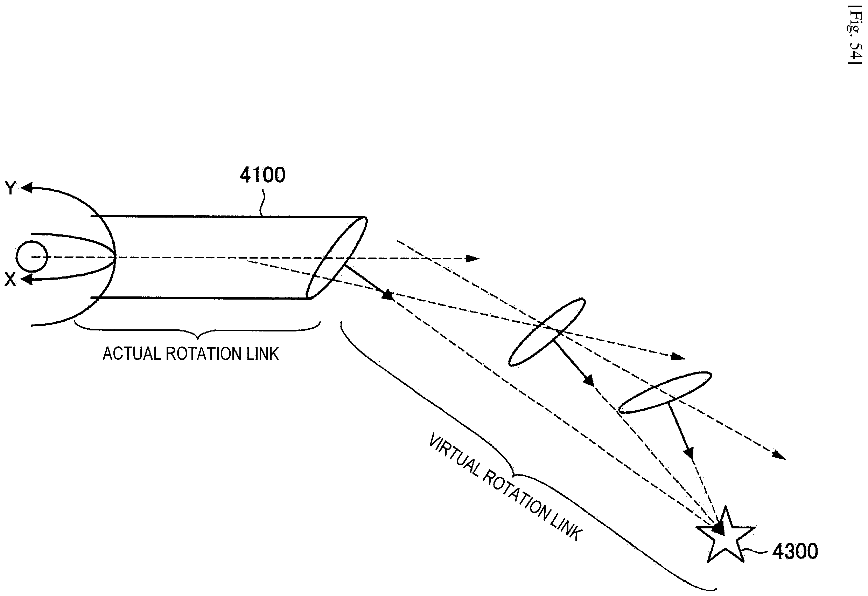

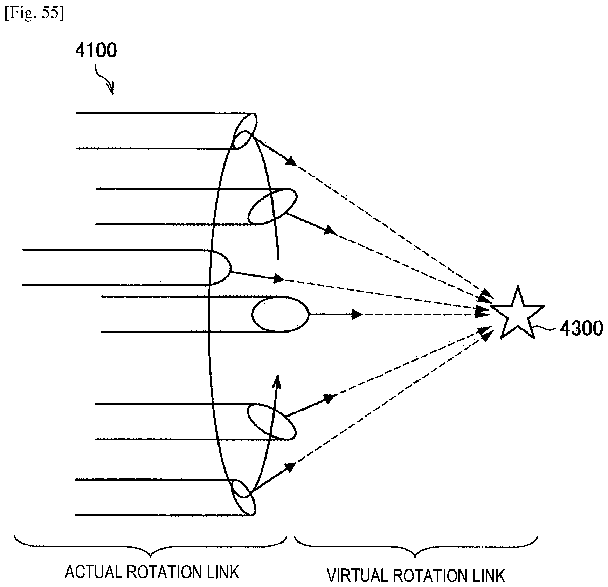

Meanwhile, by modeling virtual rotary links with an observation target set at a distal end, a gazing operation at the virtual rotary link distal end may be given as a motion objective while a connection relation between the actual rotary links of the arm and the virtual rotary links connected to the tip (which corresponds to an oblique-viewing angle in the case of the oblique-viewing endoscope) is maintained. That is, the control unit 300 may dynamically update the virtual rotary links on the basis of the zoom operation or rotation operation of the oblique-viewing angle-variable oblique-viewing endoscope. This example will be described with reference to FIGS. 54 and 55.

FIG. 54 is an explanatory diagram for describing Application Example 2 of the medical imaging apparatus, which is a diagram for describing updating of virtual rotary links taking a zoom operation of an oblique-viewing angle-variable oblique-viewing endoscope into consideration. Referring to FIG. 54, an oblique-viewing angle-variable oblique-viewing endoscope 4100 and an observation target 4300 are illustrated. In a case in which a zoom operation is performed, for example, the control unit 300 changes a distances and orientations of the virtual rotary links (shortens distances of the virtual rotary links and orientations of the virtual rotary links is significantly inclined with respect to the axis of the scope in a case of an enlargement operation as illustrated in FIG. 54), and thereby the observation target 4300 can be caught at the center of the camera and a motion objective can be realized as illustrated in FIG. 54.

FIG. 55 is an explanatory diagram for describing Application Example 2 of the medical imaging apparatus, which is a diagram for describing updating of virtual rotary links taking a rotation operation of the oblique-viewing angle-variable oblique-viewing endoscope into consideration. Referring to FIG. 55, the oblique-viewing angle-variable oblique-viewing endoscope 4100 and the observation target 4300 are illustrated. In a case in which a rotation operation is performed, for example, the control unit 300 sets distances of the virtual rotary links to be uniform as illustrated in FIG. 55, and thereby the observation target 4300 can be caught at the center of the camera and a motion objective can be realized as illustrated in FIG. 55.

In addition, the above-described technology for the setting of the virtual links can also be applied on the premise that use of the medical imaging apparatus according to the present embodiment. Specifically, the setting of virtual links (virtual rotary links) may be performed regarding that the portion of the support unit 103 that rotates with respect to the base unit 105 of the above-described camera head 101 due to drive of the drive unit 107 as an actual rotary link.

Next, an example of a technique that can maintain hand-eye coordination in a case in which an oblique-viewing endoscope is used as the rigid endoscope 131 to be attached to the camera head 101 will be described as Application Example 2 with reference to FIGS. 50 to 55.

### <<7. Conclusion>>

The medical imaging apparatus according to the present disclosure has an imaging unit that captures an image of an affected area, a light source supply system that guides light from a light source to be projected onto the affected area, a support unit that supports the imaging unit and the light source supply system, and a drive unit as described above. In addition, the drive unit causes the support unit to rotate such that the imaging unit and the light source supply system integrally rotate about an axis of the imaging unit extending in an imaging direction.

With the above-described configuration, for example, even in a case in which an imaging optical system (e.g., a medical optical apparatus such as a rigid endoscope) is separately attached in the front stage of the imaging unit, the imaging unit and the imaging optical system can be integrally rotated while the state in which the imaging optical system is positioned in the front stage of the imaging unit is maintained. That is, even in a case in which the support unit is rotated, the state in which the imaging optical system is positioned in the front stage of the imaging unit is maintained. The same applies to a case in which a separate light source supply system (i.e., a light source supply system provided in the medical optical apparatus) is attached to the latter stage of the light source supply system supported by the support unit.

In addition, with the above-described configuration, a light guide that guides light from a light source can be provided to extend from the rear side of the medical imaging apparatus toward the light source. Accordingly, for example, it is not necessary to separately provide a connection part for connecting the light guide to the medical optical apparatus such as a rigid endoscope, and thus the configuration becomes simple. For this reason, handling of the medical imaging apparatus according to the present embodiment by an operator becomes easier and further operability is further improved than in a case in which a connection part for connecting the light guide to the medical optical apparatus is provided.

In addition, the medical imaging apparatus can control such that an image captured by the imaging unit is rotated for correction in accordance with rotation of the support unit. Accordingly, the medical imaging apparatus can maintain hand-eye coordination of the operator in a preferable mode.

In addition, a medical optical apparatus such as a rigid endoscope or a microscope can be selectively attached to the support unit (i.e., the camera head). In addition, at this time, work of connecting a separate light guide to the medical optical apparatus to be attached or the like is unnecessary depending on the medical optical apparatus. That is, the operator may attach the medical optical apparatus to the support unit in accordance with an application or a procedure, and troublesome work of preparing apparatuses at each time of surgery in a tailor-made manner becomes unnecessary. In addition, since the medical imaging apparatus can be used in various procedures by selectively switching a medical optical apparatus to be attached to the support unit, it is not necessary to retain individual apparatuses for each procedure, and thus effects such as improvement in operation rates of apparatuses, a decrease in storage places, and lower costs can also be expected. In addition, since it is not necessary for the operator to learn operation methods of a plurality of apparatuses, a burden on the operator can be reduced.

Note that, although the case in which the medical optical apparatus such as an endoscope (for example a rigid endoscope) or a microscope is detachable from the camera head has been described in the above-described examples, a configuration of the medical imaging apparatus is not necessarily limited. That is, the camera head and the medical optical apparatus may be integrated as long as the image sensor and the light source supply system supported by the support unit are integrally rotated when the support unit rotates with respect to the base unit.

Moreover, although the aforementioned examples discuss the endoscope and microscope being used for surgery or medical applications, the disclosure is not so limited. For example, the endoscope may be an industrial endoscope for looking in pipes and the microscope may be used to investigate micro-cracks or surface damage on a pipe.

It should be understood by those skilled in the art that various modifications, combinations, sub-combinations and alterations may occur depending on design requirements and other factors insofar as they are within the scope of the appended claims.

The present invention is defined by the appended claims.

### Reference Signs List

100 medical imaging apparatus
101 camera head
103 support unit
105 base unit
107 drive unit
109 image sensor
111 imaging optical system
113 light source supply system
115 operation unit
121 adaptor
131 rigid endoscope
133 base unit
135 lens barrel
137 imaging optical system
139 light source supply system
151 microscope
153 lens barrel
155 imaging optical system
157 light source supply system

## Claims

1. A support unit (103) for a medical camera head (101), the support unit being configured to rotate about an axis (C11) and including:
an adapter (121) configured to detachably mount a plurality of different medical scopes thereon;
a light source supply system (139; 157) configured to supply light to the medical scope when mounted, the light source supply system being configured to pass through the axis; and
an image sensor (109) configured to capture an image from the medical scope, and wherein the axis is in the imaging direction of the image sensor, wherein
the support unit is configured to rotate about the axis relative to a mounted one of the plurality of different medical scopes without the mounted one of the plurality of different medical scopes becoming unmounted.

2. A support unit according to claim 1, wherein the image sensor is connected to a signal line (1001) and the signal line is configured to pass through the axis.

3. A support unit according to claim 1 or 2, wherein the light source supply system passes through a point of rotation.

4. A support unit according to any preceding claim, configured to be a cylindrical shape.

5. A support unit according to any preceding claim, wherein the adapter includes a lock mechanism (1113) configured to engage and lock a surgical microscope (151) or an endoscope (131) in position.

6. A support unit according to any preceding claim, further including a support unit terminal (1007) configured to engage with a respective terminal (1121) on the surgical microscope or endoscope, the support unit terminal being configured to receive information from the respective terminal.

7. A support unit according to claim 6, wherein the information relates to the microscope or endoscope.

8. A support unit according to claim 7, wherein the information identifies the scope or an optical parameter of the scope.

9. A support unit according to either claim 2 or 3, including an attitude detection unit configured to sense the orientation of the image sensor.

10. A support unit according to any preceding claim, wherein the light source supply system is split into two parts.

11. A support unit according to any preceding claim, comprising a power supply line connected to the image sensor, the power supply line being configured to pass through the axis with the light source supply system.

12. A support unit according to any preceding claim, comprising signal lines configured to pass through the centre of the support unit, the signal lines having ring shaped terminals extending radially from the centre of the support unit.

13. A surgical camera head (101) including a support unit according to any one of claims 1 to 12 and a drive unit (107) configured to rotate the support unit about the axis.

14. A surgical camera head including a support unit according to claim 9 and a drive unit configured to rotate the support unit about the axis.

15. A method of operating a support unit according to any of claims 1-12, the method including:
detachably mounting the plurality of different medical scopes on the adapter;
supplying light to the medical scope when mounted, the light source supply system being configured to pass through the axis; and
capturing an image from the medical scope, using the image sensor (109) wherein the axis is in the imaging direction of the image sensor, wherein
the support unit is configured to rotate about the axis relative to a mounted one of the plurality of different medical scopes without the mounted one of the plurality of different medical scopes becoming unmounted.

## Patentansprüche

1. Trägereinheit (103) für einen medizinischen Kamerakopf (101), wobei die Trägereinheit konfiguriert ist, um sich um eine Achse (C11) zu drehen und einschließt:
einen Adapter (121), der konfiguriert ist, um eine Vielzahl verschiedener medizinischer Geräte lösbar daran zu montieren;
ein Lichtquellenversorgungssystem (139; 157), das konfiguriert ist, um das medizinische Gerät, wenn montiert, mit Licht zu versorgen, wobei das Lichtquellenversorgungssystem konfiguriert ist, um durch die Achse zu verlaufen; und
einen Bildsensor (109), der konfiguriert ist, um ein Bild von dem medizinischen Gerät aufzunehmen, und wobei die Achse in der Abbildungsrichtung des Bildsensors liegt, wobei
die Trägereinheit konfiguriert ist, um sie sich relativ zu einem montierten der Vielzahl verschiedener medizinischer Geräte um die Achse zu drehen, ohne dass das montierte der Vielzahl verschiedener medizinischer Geräte abmontiert wird.

2. Trägereinheit nach Anspruch 1, wobei der Bildsensor mit einer Signalleitung (1001) verbunden ist und die Signalleitung konfiguriert ist, um durch die Achse zu verlaufen.

3. Trägereinheit nach Anspruch 1 oder 2, wobei das Lichtquellenversorgungssystem durch einen Drehpunkt verläuft.

4. Trägereinheit nach einem der vorstehenden Ansprüche, die konfiguriert ist, um eine zylindrische Form zu sein.

5. Trägereinheit nach einem der vorstehenden Ansprüche, wobei der Adapter einen Verriegelungsmechanismus (1113) einschließt, der konfiguriert ist, um ein Operationsmikroskop (151) oder ein Endoskop (131) in Eingriff zu nehmen und in Position zu verriegeln.

6. Trägereinheit nach einem der vorstehenden Ansprüche, die ferner einen Trägereinheitsanschluss (1007) einschließt, der konfiguriert ist, um einen entsprechenden Anschluss (1121) an dem Operationsmikroskop oder Endoskop in Eingriff zu nehmen, wobei der Trägereinheitsanschluss konfiguriert ist, um Informationen von dem entsprechenden Anschluss zu empfangen.

7. Trägereinheit nach Anspruch 6, wobei sich die Informationen auf das Mikroskop oder Endoskop beziehen.

8. Trägereinheit nach Anspruch 7, wobei die Informationen das Gerät oder einen optischen Parameter des Geräts identifizieren.

9. Trägereinheit nach Anspruch 2 oder 3, die eine Lageerkennungseinheit einschließt, die konfiguriert ist, um die Ausrichtung des Bildsensors zu erfassen.

10. Trägereinheit nach einem der vorstehenden Ansprüche, wobei das Lichtquellenversorgungssystem in zwei Teile aufgeteilt ist.

11. Trägereinheit nach einem der vorstehenden Ansprüche, umfassend eine Stromversorgungsleitung, die mit dem Bildsensor verbunden ist, wobei die Stromversorgungsleitung konfiguriert ist, um durch die Achse mit dem Lichtquellenversorgungssystem zu verlaufen.

12. Trägereinheit nach einem der vorstehenden Ansprüche, umfassend Signalleitungen, die konfiguriert sind, um durch die Mitte der Trägereinheit zu verlaufen, wobei die Signalleitungen ringförmige Anschlüsse aufweisen, die sich von der Mitte der Trägereinheit radial erstrecken.

13. Chirurgischer Kamerakopf (101), der eine Trägereinheit nach einem der Ansprüche 1 bis 12 und eine Antriebseinheit (107), die konfiguriert ist, um die Trägereinheit um die Achse zu drehen, einschließt.

14. Chirurgischer Kamerakopf, der eine Trägereinheit nach Anspruch 9 und eine Antriebseinheit, die konfiguriert ist, um die Trägereinheit um die Achse zu drehen, einschließt.

15. Verfahren zum Betreiben einer Trägereinheit nach einem der Ansprüche 1 bis 12, wobei das Verfahren das lösbare Montieren der Vielzahl verschiedener medizinischer Geräte an dem Adapter einschließt;
Versorgen des medizinischen Geräts mit Licht, wenn montiert, wobei das Lichtquellenversorgungssystem konfiguriert ist, um durch die Achse zu verlaufen; und
Aufnehmen eines Bildes von dem medizinischen Gerät unter Verwendung des Bildsensors (109), wobei die Achse in der Abbildungsrichtung des Bildsensors liegt, wobei
die Trägereinheit konfiguriert ist, um sie sich relativ zu einem montierten der Vielzahl verschiedener medizinischer Geräte um die Achse zu drehen, ohne dass das montierte der Vielzahl verschiedener medizinischer Geräte abmontiert wird.

## Revendications

1. Unité de support (103) pour une tête de caméra médicale (101), l'unité de support étant conçue pour tourner autour d'un axe (C11) et comportant :
un adaptateur (121) conçu pour monter de manière amovible une pluralité de différents instruments d'exploration médicaux ;
un système d'alimentation de source lumineuse (139 ; 157) configuré pour fournir de la lumière à l'instrument d'exploration médical lorsqu'il est monté, le système d'alimentation de source lumineuse étant conçu pour traverser l'axe ; et
un capteur d'image (109) configuré pour capturer une image à partir de l'instrument d'exploration médical, et dans laquelle l'axe est dans la direction d'imagerie du capteur d'image, dans laquelle
l'unité de support est conçue pour tourner autour de l'axe par rapport à un instrument d'exploration médical monté de la pluralité d'instruments d'exploration médicaux différents sans que l'instrument d'exploration médical monté de la pluralité d'instruments d'exploration médicaux différents ne se démonte.

2. Unité de support selon la revendication 1, dans laquelle le capteur d'image est connecté à une ligne de signal (1001) et la ligne de signal est conçue pour traverser l'axe.

3. Unité de support selon la revendication 1 ou 2, dans laquelle le système d'alimentation de source lumineuse passe par un point de rotation.

4. Unité de support selon l'une quelconque revendication précédente, conçue pour être de forme cylindrique.

5. Unité de support selon l'une quelconque revendication précédente, dans laquelle l'adaptateur comporte un mécanisme de verrouillage (1113) conçu pour mettre en prise et verrouiller un microscope chirurgical (151) ou un endoscope (131) en position.

6. Unité de support selon l'une quelconque revendication précédente, comportant en outre un terminal d'unité de support (1007) conçu pour entrer en prise avec un terminal respectif (1121) sur le microscope chirurgical ou l'endoscope, le terminal d'unité de support étant configuré pour recevoir des informations du terminal respectif.

7. Unité de support selon la revendication 6, dans laquelle les informations concernent le microscope ou l'endoscope.

8. Unité de support selon la revendication 7, dans laquelle les informations identifient l'instrument d'exploration ou un paramètre optique de l'instrument d'exploration.

9. Unité de support selon la revendication 2 ou 3, comportant une unité de détection d'attitude configurée pour détecter l'orientation du capteur d'image.

10. Unité de support selon l'une quelconque revendication précédente, dans laquelle le système d'alimentation de source lumineuse est divisé en deux parties.

11. Unité de support selon l'une quelconque revendication précédente, comprenant une ligne d'alimentation connectée au capteur d'image, la ligne d'alimentation étant conçue pour traverser l'axe avec le système d'alimentation de source lumineuse.

12. Unité de support selon l'une quelconque revendication précédente, comprenant des lignes de signal conçues pour traverser le centre de l'unité de support, les lignes de signal présentant des terminaux en forme d'anneau s'étendant radialement à partir du centre de l'unité de support.

13. Tête de caméra chirurgicale (101) comportant une unité de support selon l'une quelconque des revendications 1 à 12 et une unité d'entraînement (107) conçue pour faire tourner l'unité de support autour de l'axe.

14. Tête de caméra chirurgicale comportant une unité de support selon la revendication 9 et une unité d'entraînement conçue pour faire tourner l'unité de support autour de l'axe.

15. Procédé d'exploitation d'une unité de support selon l'une quelconque des revendications 1 à 12, le procédé comportant le montage amovible de la pluralité de différents instruments d'exploration médicaux sur l'adaptateur ;
la fourniture de lumière à l'instrument d'exploration médical lorsqu'il est monté, le système d'alimentation de source lumineuse étant conçu pour traverser l'axe ; et
la capture d'une image de l'instrument d'exploration médical à l'aide du capteur d'image (109), dans lequel l'axe se trouve dans la direction d'imagerie du capteur d'image, dans lequel
l'unité de support est conçue pour tourner autour de l'axe par rapport à un instrument d'exploration médical monté de la pluralité d'instruments d'exploration médicaux différents sans que l'instrument d'exploration médical monté de la pluralité d'instruments d'exploration médicaux différents ne se démonte.
